# EUROPEAN PATENT SPECIFICATION

(11) **EP 1 808 490 B1**
(45) Date of publication and mention of the grant of the patent: **14.11.2012**
(21) Application number: 06124074.3
(22) Date of filing: 31.07.2000
(51) Int. Cl.: C07K 14/195, C12N 1/21, C07K 14/22, A61K 39/095

(54) **Genetically engineered bleb vaccine**
Genetisch manipulierter Bleb Impfstoff
Vaccin à bleb mis au point par génie génétique

(30) Priority: 03.08.1999 GB 9918319
(43) Date of publication of application: 18.07.2007
(62) Divisional of application: 00956369.3
(73) Proprietor: GlaxoSmithKline Biologicals SA, 1330 Rixensart (BE)
(72) Inventor: Berthet, Francois-Xavier Jacques, 1330 Rixensart (BE); Dalemans, Wilfred L. J., 1330 Rixensart (BE); Denoel, Philippe, 1330 Rixensart (BE); Dequesne, Guy, 1330 Rixensart (BE); Feron, Christiane, 1330 Rixensart (BE); Lobet, Yves, 1330 Rixensart (BE); Poolman, Jan, 1330 Rixensart (BE); Thiry, Georges, 1330 Rixensart (BE); Thonnard, Joelle, 1330 Rixensart (BE); Voet, Pierre, 1330 Rixensart (BE)
(74) Representative: Lubienski, Michael John

(56) References cited:
- WO-A-98/33923
- WO-A-99/10497
- WO-A1-97/19688
- US-A- 5 098 997
- CLAASSEN I ET AL: "Production, characterization and control of a Neisseria meningitidis hexavalent class 1 outer membrane protein containing vesicle vaccine" VACCINE,GB,BUTTERWORTH SCIENTIFIC. GUILDFORD, vol. 14, no. 10, 1 July 1996 (1996-07-01), pages 1001-1008, XP004057632 ISSN: 0264-410X
- VAN DER LEY P ET AL: "Construction of Neisseria meningitidis strains carrying multiple chromosomal copies of the porA gene for use in the production of a multivalent outer membrane vesicle vaccine" VACCINE,BUTTERWORTH SCIENTIFIC. GUILDFORD,GB, vol. 13, no. 4, 1995, pages 401-407, XP004057740 ISSN: 0264-410X
- CARTWRIGHT K ET AL: "Immunogenicity and reactogenicity in UK infants of a novel meningococcal vesicle vaccine containing multiple class 1 (PorA) outer membrane proteins." VACCIN, vol. 17, no. 20-21, 4 June 1999 (1999-06-04), pages 2612-2619, XP002154106
- HELMINEN M E ET AL: "A major outer membrane protein of moraxella catarrhalis is a target for antibodies that enhance pulmonary clearance of the pathogen in an animal model" INFECTION AND IMMUNITY,US,AMERICAN SOCIETY FOR MICROBIOLOGY. WASHINGTON, vol. 61, no. 5, 1 May 1993 (1993-05-01), pages 2003-2010, XP002048787 ISSN: 0019-9567

## Description

### FIELD OF THE INVENTION

The present invention relates to the field of Gram-negative bacterial vaccine compositions, their manufacture, and the use of such compositions in medicine. More particularly it relates to the field of novel outer-membrane vesicle (or bleb) vaccines, and advantageous methods of rendering these vaccines more effective and safer.

### BACKGROUND OF THE INVENTION

Gram-negative bacteria are separated from the external medium by two successive layers of membrane structures. These structures, referred to as the cytoplasmic membrane and the outer membrane (OM), differ both structurally and functionally. The outer membrane plays an important role in the interaction of pathogenic bacteria with their respective hosts. Consequently, the surface exposed bacterial molecules represent important targets for the host immune response, making outer-membrane components attractive candidates in providing vaccine, diagnostic and therapeutics reagents.

Whole cell bacterial vaccines (killed or attenuated) have the advantage of supplying multiple antigens in their natural micro-environment. Drawbacks around this approach are the side effects induced by bacterial components such as endotoxin and peptidoglycan fragments. On the other hand, acellular subunit vaccines containing purified components from the outer membrane may supply only limited protection and may not present the antigens properly to the immune system of the host.

Proteins, phospholipids and lipopolysaccharides are the three major constituents found in the outer-membrane of all Gram-negative bacteria. These molecules are distributed asymmetrically: membrane phospholipids (mostly in the inner leaflet), lipooligosaccharides (exclusively in the outer leaflet) and proteins (inner and outer leaflet lipoproteins, integral or polytopic membrane proteins). For many bacterial pathogens which impact on human health, lipopolysaccharide and outer-membrane proteins have been shown to be immunogenic and amenable to confer protection against the corresponding disease by way of immunization.

The OM of Gram-negative bacteria is dynamic and, depending on the environmental conditions, can undergo drastic morphological transformations. Among these manifestations, the formation of outer-membrane vesicles or "blebs" has been studied and documented in many Gram-negative bacteria (Zhou, L et al. 1998. FEMS Microbiol. Lett. 163: 223-228). Among these, a non-exhaustive list of bacterial pathogens reported to produce blebs include: *Bordetella pertussis, Borrelia burgdorferi, Brucella melitensis, Brucella ovis, Chlamydia psittaci, Chlamydia trachomatis, Esherichia coli, Haemophilus influenzae, Legionella pneumophila, Neisseria gonorrhoeae, Neisseria meningitidis, Pseudomonas aeruginosa* and *Yersinia enterocolitica.* Although the biochemical mechanism responsible for the production of OM blebs is not fully understood, these outer membrane vesicles have been extensively studied as they represent a powerful methodology in order to isolate outer-membrane protein preparations in their native conformation. In that context, the use of outer-membrane preparations is of particular interest to develop vaccines against *Neisseria, Moraxella catarrhalis, Haemophilus influenzae, Pseudomonas aeruginosa* and *Chlamydia.* Moreover, outer membrane blebs combine multiple proteinaceaous and non-proteinaceous antigens that are likely to confer extended protection against intra-species variants.

In comparison with the other, more widely used, types of bacterial vaccine (whole cell bacterial and purified subunit vaccines), the inventors will show that outer membrane bleb vaccines (if modified in certain ways) may represent the ideal compromise.

The wide-spread use of bacterial subunit vaccines has been due to the intensive study of bacterial surface proteins that have been found to be useful in vaccine applications [for instance *B. pertussis* pertactin]. These proteins are loosely associated with the bacterial outer membrane and can be purified from culture supernatant or easily extracted from the bacterial cells. However it has also been shown that structural, integral outer membrane proteins are also protective antigens. Examples are PorA for *N. meningitidis* serogroup B; D15 for *H. influenzae*; OMP CD for *M. catarrhalis*; OMP F for *P. Aeruginosa.* Such proteins however have rather specific structural features, particularly multiple amphipathic β-sheets, which complicates their straightforward use as purified (recombinant) subunit vaccines.

In addition, it has become clear that multiple component vaccines are needed (in terms of bacterial surface proteins and integral membrane proteins) to supply a reasonable level of protection. For instance, in the case of *B. pertussis* subunit vaccines multicomponent vaccines are superior to mono or bicomponent products.

In order to incorporate integral outer-membrane proteins into such a subunit product, native (or near-native) conformational folding of the proteins must be present in the product in order to have a useful immunological effect. The use of excreted outer membrane vesicles or blebs may be an elegant solution to the problem of including protective integral membrane proteins into a subunit vaccine whilst still ensuring that they fold properly.

*N. meningitidis* serogroup B (menB) excretes outer membrane blebs in sufficient quantities to allow their manufacture on an industrial scale. Such multicomponent outer-membrane protein vaccines from naturally-occurring menB strains have been found to be efficacious in protecting teenagers from menB disease and have become registered in Latin America. An alternative method of preparing outer-membrane vesicles is via the process of detergent extraction of the bacterial cells (EP 11243).

Examples of bacterial species from which bleb vaccines can be made are the following.

### Neisseria meningitidis:

*Neisseria meningitidis* (meningococcus) is a Gram-negative bacterium frequently isolated from the human upper respiratory tract. It occasionally causes invasive bacterial diseases such as bacteremia and meningitis. The incidence of meningococcal disease shows geographical seasonal and annual differences (Schwartz, B., Moore, P.S., Broome, C.V.; Clin. Microbiol. Rev. 2 (Supplement), S18-S24, 1989). Most disease in temperate countries is due to strains of serogroup B and varies in incidence from 1-10/100,000/year total population sometimes reaching higher values (Kaczmarski, E.B. (1997), Commun. Dis. Rep. Rev. 7: R55-9, 1995; Scholten, R.J.P.M., Bijlmer, H.A., Poolman, J.T. et al. Clin. Infect. Dis. 16: 237-246, 1993; Cruz, C., Pavez, G., Aguilar, E., et al. Epidemiol. Infect. 105: 119-126, 1990). Age-specific incidences in the two high risk-groups, infants and teenagers, reach higher levels.

Epidemics dominated by serogroup A meningococci occur, mostly in central Africa, sometimes reaching levels up to 1000/100,000/year (Schwartz, B., Moore, P.S., Broome, C.V. Clin. Microbiol. Rev. 2 (Supplement), S18-524, 1989). Nearly all cases of meningococcal disease as a whole are caused by serogroup A, B, C, W-135 and Y meningococci. A tetravalent A, C, W-135, Y capsular polysaccharide vaccine is available (Armand, J., Arminjon, F., Mynard, M.C., Lafaix, C., J. Biol. Stand. 10: 335-339, 1982).

The polysaccharide vaccines are currently being improved by way of chemically conjugating them to carrier proteins (Lieberman, J.M., Chiu, S.S., Wong, V.K., et al. JAMA 275 : 1499-1503, 1996). A serogroup B vaccine is not available, since the B capsular polysaccharide is non-immunogenic, most likely because it shares structural similarity to host components (Wyle, F.A., Artenstein, M.S., Brandt, M.L. et al. J. Infect. Dis. 126: 514-522, 1972; Finne, J.M., Leinonen, M., Mäkelä, P.M. Lancet ii.: 355-357, 983).

For many years efforts have been focused on developing meningococcal outer membrane based vaccines (de Moraes, J.C., Perkins, B., Camargo, M.C. et al. Lancet 340: 1074-1078, 1992; Bjune, G., Hoiby, E.A. Gronnesby, J.K. et al. 338: 1093-1096, 1991). Such vaccines have demonstrated efficacies from 57% - 85% in older children (>4 years) and adolescents. Most of these efficacy trials were performed with OMV (outer membrane vesicles, derived by LPS depletion from blebs) vaccines derived from wild-type *N. meningitidis* B strains.

Many bacterial outer membrane components are present in these vaccines, such as PorA, PorB, Rmp, Opc, Opa, FrpB and the contribution of these components to the observed protection still needs further definition. Other bacterial outer membrane components have been defined (using animal or human antibodies) as potentially being relevant to the induction of protective immunity, such as TbpB, NspA (Martin, D., Cadieux, N., Hamel, J., Brodeux, B.R, J. Exp. Med. 185: 1173-1183, 1997; Lissolo, L., Maitre-Wilmotte, C., Dumas, p. et al., Inf. Immun. 63: 884-890, 1995). The mechanism of protective immunity will involve antibody mediated bactericidal activity and opsonophagocytosis.

Genetically-engineered bleb preparations from *Neisseira meningitidis* have also been investigated. Starting with *Neisseria meningitidis* strain H44/76, a set of strains carrying multiple chromosomal copies of the *porA* gene were constructed for use in the production of a multivalent outer membrane vesicle vaccine (Van der Ley, P., Van der Biezen, J. and Poolman, J. T., Vaccine 13: 401-407). It has also proved possible to inactivate the early stage of lipid A synthesis of mucosal gram negative bacteria without compromising cell viability. In particular, the *lpxA* gene in *N. meningitidis* was mutated and resulting *lpxA* knockout mutants were found to be completely lipopolysaccharide (LPS)-deficient (WO 99/10497).

The frequency of *Neisseria meningitidis* infections has risen dramatically in the past few decades. This has been attributed to the emergence of multiple antibiotic resistant strains, and increased exposure due to an increase in social activities (for instance swimming pools or theatres). It is no longer uncommon to isolate *Neisseria meningitidis* strains that are resistant to some or all of the standard antibiotics. This phenomenon has created an unmet medical need and demand for new anti-microbial agents, vaccines, drug screening methods, and diagnostic tests for this organism.

### Moraxella catarrhalis

*Moraxella catarrhalis* (also named *Branhamella catarrhalis*) is a Gram-negative bacterium frequently isolated from the human upper respiratory tract. It is responsible for several pathologies, the main ones being otitis media in infants and children, and pneumonia the elderly. It is also responsible for sinusitis, nosocomial infections and, less frequently, for invasive diseases.

Bactericidal antibodies have been identified in most adults tested (Chapman, AJ et al. (1985) J. Infect.Dis. 151:878). Strains of *M. catarrhalis* present variations in their capacity to resist serum bactericidal activity: in general, isolates from diseased individuals are more resistant than those who are simply colonized (Hol, C et al. (1993) Lancet 341:1281, Jordan, KL et al. (1990) Am.J.Med. 88 (suppl. 5A):28S). Serum resistance could therfore be considered as a virulence factor of the bacteria. An opsonizing activity has been observed in the sera of children recovering from otitis media.

The antigens targetted by these different immune responses in humans have not been identified, with the exception of OMP B1, a 84 kDa protein, the expression of which is regulated by iron, and that is recognized by the sera of patients with pneumonia (Sethi, S, et al. (1995) Infect.Immun. 63:1516), and of UspA1 and UspA2 (Chen D. et al.(1999), Infect.Immun. 67:1310).

A few other membrane proteins present on the surface of *M. catarrhalis* have been characterized using biochemical methods for their potential implication in the induction of a protective immunity (for review, see Murphy, TF (1996) Microbiol.Rev. 60:267). In a mouse pneumonia model, the presence of antibodies raised against some of them (UspA, CopB) favors a faster clearance of the pulmonary infection. Another polypeptide (OMP CD) is highly conserved among *M. catarrhalis* strains, and presents homologies with a porin of *Pseudomonas aeruginosa,* which has been demonstrated to be efficacious against this bacterium in animal models.

*M. catarrhalis* produces outer membrane vesicles (Blebs). These Blebs have been isolated or extracted by using different methods (Murphy T.F., Loeb M.R. 1989. Microb. Pathog. 6: 159-174; Unhanand M., Maciver, I., Ramillo O., Arencibia-Mireles O., Argyle J.C., McCracken G.H. Jr., Hansen E.J. 1992. J. Infect. Dis. 165:644-650). The protective capacity of such Bleb preparations has been tested in a murine model for pulmonary clearance of *M. catarrhalis.* It has been shown that active immunization with Bleb vaccine or passive transfer of anti-Blebs antibody induces significant protection in this model (Maciver I., Unhanand M., McCracken G.H. Jr., Hansen, E.J. 1993. J. Infect. Dis. 168: 469-472).

### Haemophilus influenzae

*Haemophilus influenzae* is a non-motile Gram-negative bacterium. Man is its only natural host. *H. influenzae* isolates are usually classified according to their polysaccharide capsule. Six different capsular types designated 'a' through 'f' have been identified. Isolates that fail to agglutinate with antisera raised against one of these six serotypes are classified as nontypeable, and do not express a capsule.

*H. influenzae* type b (Hib) is clearly different from the other types in that it is a major cause of bacterial meningitis and systemic diseases. Nontypeable strains of *H*. *influenzae* (NTHi) are only occasionally isolated from the blood of patients with systemic disease. NTHi is a common cause of pneumonia, exacerbation of chronic bronchitis, sinusitis and otitis media. NTHi strains demonstrate a large variability as identified by clonal analysis, whilst Hib strains as a whole are more homogeneous.

Various proteins of *H. influenzae* have been shown to be involved in pathogenesis or have been shown to confer protection upon vaccination in animal models.

Adherence of NTHi to human nasopharygeal epithelial cells has been reported (Read RC. et al. 1991. J. Infect. Dis. 163:549). Apart from fimbriae and pili (Brinton CC. et al. 1989. Pediatr. Infect. Dis. J. 8:S54; Kar S. et al. 1990. Infect. Immun. 58:903; Gildorf JR. et al. 1992. Infect. Immun. 60:374; St. Geme JW et al. 1991. Infect. Immun. 59:3366; St. Geme JW et al. 1993. Infect. Immun. 61: 2233), many adhesins have been identified in NTHi. Among them, two surface exposed high-molecular-weight proteins designated HMW1 and HMW2 have been shown to mediate adhesion of NTHi to epithelial cells (St. Geme JW. et al. 1993. Proc. Natl. Acad. Sci. USA 90:2875). Another family of high-molecular-weight proteins has been identified in NTHi strains that lack proteins belonging to HMW1/HMW2 family. The NTHi 115-kDa Hia protein (Barenkamp SJ., St Geme S.W. 1996. Mol. Microbiol. In press) is highly similar to the Hsf adhesin expressed by *H. influenzae* type b strains (St. Geme JW. et al. 1996. J. Bact. 178:6281). Another protein, the Hap protein shows similarity to IgA1 serine proteases and has been shown to be involved in both adhesion and cell entry (St. Geme JW. et al. 1994. Mol. Microbiol. 14:217).

Five major outer membrane proteins (OMP) have been identified and numerically numbered. Original studies using *H. influenzae* type b strains showed that antibodies specific for P1 and P2 OMPs protected infant rats from subsequent challenge (Loeb MR. et al. 1987. Infect. Immun. 55:2612; Musson RS. Jr. et al. 1983. J. Clin. Invest. 72:677). P2 was found to be able to induce bactericidal and opsonic antibodies, which are directed against the variable regions present within surface exposed loop structures of this integral OMP (Haase EM. et al. 1994 Infect. Immun. 62:3712; Troelstra A. et al. 1994 Infect. Immun. 62:779). The lipoprotein P4 also may induce bactericidal antibodies (Green BA. et al. 1991. Infect.Immun.59:3191).

OMP P6 is a conserved peptidoglycan associated lipoprotein making up 1-5 % of the outer membrane (Nelson MB. et al. 1991. Infect. Immun. 59:2658). Later a lipoprotein of about the same molecular weight was recognized called PCP (P6 cross-reactive protein) (Deich RM. et al. 1990. Infect. Immun. 58:3388). A mixture of the conserved lipoproteins P4, P6 and PCP did not reveal protection as measured in a chinchilla otitis-media model (Green BA. et al. 1993. Infect.immun. 61:1950). P6 alone appears to induce protection in the chinchilla model (Demaria TF. et al. 1996. Infect. Immun. 64:5187).

A fimbrin protein (Miyamoto N., Bakaletz, LO. 1996. Microb. Pathog. 21:343) has also been described with homology to OMP P5, which itself has sequence homology to the integral *Escherichia coli* OmpA (Miyamoto N., Bakaletz, LO. 1996. Microb. Pathog. 21:343; Munson RS. Jr. et al. 1993. Infect. Immun. 61:1017). NTHi seem to adhere to mucus by way of fimbriae.

In line with the observations made with gonococci and meningococci, NTHi expresses a dual human transferrin receptor composed of TbpA and TbpB when grown under iron limitation. Anti-TbpB antibody protected infant rats (Loosmore SM. et al. 1996. Mol. Microbiol. 19:575). Hemoglobin / haptoglobin receptor also have been described for NTHi (Maciver I. et al. 1996. Infect. Immun. 64:3703). A receptor for Haem:Hemopexin has also been identified (Cope LD. et al. 1994. Mol.Microbiol. 13:868). A lactoferrin receptor is also present amongst NTHi, but is not yet characterized (Schryvers AB. et al. 1989. J. Med. Microbiol. 29:121). A protein similar to neisserial FrpB-protein has not been described amongst NTHi.

An 80kDa OMP, the D15 surface antigen, provides protection against NTHi in a mouse challenge model. (Flack FS. et al. 1995. Gene 156:97). A 42kDa outer membrane lipoprotein, LPD is conserved amongst *Haemophilus influenzae* and induces bactericidal antibodies (Akkoyunlu M. et al. 1996. Infect. Immun. 64:4586). A minor 98kDa OMP (Kimura A. et al. 1985. Infect. Immun. 47:253), was found to be a protective antigen, this OMP may very well be one of the Fe-limitation inducible OMPs or high molecular weight adhesins that have been characterized thereafter. *H*. *Influenzae* produces IgA1-protease activity (Mulks MH., Shoberg RJ. 1994. Meth. Enzymol. 235:543). IgA1-proteases of NTHi have a high degree of antigenic variability (Lomholt H., van Alphen L., Kilian, M. 1993. Infect. Immun. 61:4575).

Another OMP of NTHi, OMP26, a 26-kDa protein has been shown to enhance pulmonary clearance in a rat model (Kyd, J.M., Cripps, A.W. 1998. Infect. Immun. 66:2272). The NTHi HtrA protein has also been shown to be a protective antigen. Indeed, this protein protected Chinchilla against otitis media and protected infant rats against *H. influenzae* type b bacteremia (Loosmore S.M. et al. 1998. Infect. Immun. 66:899).

Outer membrane vesicles (or blebs) have been isolated from *H. influenzae* (Loeb M.R., Zachary A.L., Smith D.H. 1981. J. Bacteriol. 145:569-604; Stull T.L., Mack K., Haas J.E., Smit J., Smith A.L. 1985. Anal. Biochem. 150: 471-480). The vesicles have been associated with the induction of blood-brain barrier permeability (Wiwpelwey B., Hansen E.J., Scheld W.M. 1989 Infect. Immun. 57: 2559-2560), the induction of meningeal inflammation (Mustafa M.M., Ramilo O., Syrogiannopoulos G.A., Olsen K.D., McCraken G.H. Jr., Hansen, E.J. 1989. J. Infect. Dis. 159: 917-922) and to DNA uptake (Concino M.F., Goodgal S.H. 1982 J. Bacteriol. 152: 441-450). These vesicles are able to bind and be absorbed by the nasal mucosal epithelium (Harada T., Shimuzu T., Nishimoto K., Sakakura Y. 1989. Acta Otorhinolarygol. 246: 218-221) showing that adhesins and/or colonization factors could be present in Blebs. Immune response to proteins present in outer membrane vesicles has been observed in patients with various *H. influenzae* diseases (Sakakura Y., Harada T., Hamaguchi Y., Jin C.S. 1988. Acta Otorhinolarygol. Suppl. (Stockh.) 454: 222-226; Harada T., Sakakura Y., Miyoshi Y. 1986. Rhinology 24: 61-66).

### Pseudomonas aeruginosa:

The genus *Pseudomonas* consists of Gram-negative, polarly flagellated, straight and slightly curved rods that grow aerobically and do not forms spores. Because of their limited metabolic requirements, *Pseudomonas spp.* are ubiquitous and are widely distributed in the soil, the air, sewage water and in plants. Numerous species of *Pseudomonas* such as *P. aeruginosa, P. pseudomallei, P. mallei, P. maltophilia and P. cepacia* have also been shown to be pathogenic for humans. Among this list, *P. aeruginosa* is considered as an important human pathogen since it is associated with opportunistic infection of immuno-compromised host and is responsible for high morbidity in hospitalized patients. Nosocomial infection with P. *aeruginosa* afflicts primarily patients submitted for prolonged treatment and receiving immuno-suppressive agents, corticosteroids, antimetabolites antibiotics or radiation.

The *Pseudomonas,* and particularly *P. aeruginosa,* produces a variety of toxins (such as hemolysins, fibrinolysins, esterases, coagulases, phospholipases, endo- and exo-toxins) that contribute to the pathogenicity of these bacteria. Moreover, these organisms have high intrinsic resistance to antibiotics due to the presence of multiple drug efflux pumps. This latter characteristic often complicates the outcome of the disease.

Due to the uncontrolled use of antibacterial chemotherapeutics the frequency of nosocomial infection caused by *P. aeruginosa* has increased considerably over the last 30 years. In the US, for example, the economic burden of *P. aeruginosa* nosocomial infection is estimated to 4.5 billion US$ annually. Therefore, the development of a vaccine for active or passive immunization against *P. aeruginosa* is actively needed (for review see Stanislavsky et al. 1997. FEMS Microbiol. Lett. 21: 243-277).

Various cell-associated and secreted antigens of *P. aeruginosa* have been the subject of vaccine development. Among *Pseudomonas* antigens, the mucoid substance, which is an extracellular slime consisting predominantly of alginate, was found to be heterogenous in terms of size and immunogenicity. High molecular mass alginate components (30-300 kDa) appear to contain conserved epitopes while lower molecular mass alginate components (10-30 kDa) possess conserved epitopes in addition to unique epitopes. Among surface-associated proteins, PcrV, which is part of the type III secretion-translocation apparatus, has also been shown to be an interesting target for vaccination (Sawa et at. 1999. Nature Medicine 5:392-398).

Surface-exposed antigens including O-antigens (O-specific polysaccharide of LPS) or H-antigens (flagellar antigens) have been used for serotyping due to their highly immunogenic nature. Chemical structures of repeating units of O-specific polysaccharides have been elucidated and these data allowed the identification of 31 chemotypes of *P. aeruginosa.* Conserved epitopes among all serotypes *of P. aeruginosa* are located in the core oligosaccharide and the lipid A region of LPS and immunogens containing these epitopes induce cross-protective immunity in mice against different *P. aeruginosa* immunotypes. The outer core of LPS was implicated to be a ligand for binding of *P. aeruginosa* to airway and ocular epithelial cells of animals. However, heterogeneity exists in this outer core region among different serotypes. Epitopes in the inner core are highly conserved and have been demonstrated to be surface-accessible, and not masked by O-specific polysaccharide.

To examine the protective properties of OM proteins, a vaccine containing *P*. *aeruginosa* OM proteins of molecular masses ranging from 20 to 100 kDa has been used in pre-clinical and clinical trials. This vaccine was efficacious in animal models against *P. aeruginosa* challenge and induced high levels of specific antibodies in human volunteers. Plasma from human volunteers containing anti-*P*. *aeruginosa* antibodies provided passive protection and helped the recovery of 87% of patients with severe forms of *P. aeruginosa* infection. More recently, a hybrid protein containing parts of the outer membrane proteins OprF (amino acids 190-342) and OprI (amino acids 21-83) *from Pseudomonas aeruginosa* fused to the glutathione-S-transferase was shown to protect mice against a 975-fold 50% lethal dose of *P*. *aeruginosa* (Knapp et al. 1999. Vaccine. 17:1663-1669).

The present inventors have realised a number of drawbacks associated with the above wild-type bleb vaccines (either naturally occurring or chemically made).

Examples of such problems are the following:
- the presence of immunodominant but variable proteins on the bleb (PorA; TbpB, Opa [*N. meningitidis* B]; P2, P5 [non-typeable *H. influenzae*]) *-* such blebs being effective only against a restricted selection of bacterial species. Type-specificity of the bactericidal antibody response may preclude the use of such vaccines in infancy.
- the presence of unprotective (non relevant) antigens (Rmp, H8, ...) on the bleb - antigens that are decoys for the immune system
- the lack of presence of important molecules which are produced conditionally (for instance iron-regulated outer membrane proteins, IROMP's, in vivo regulated expression mechanisms) - such conditions are hard to control in bleb production in order to optimise the amount of antigen on the surface
- the low level of expression of protective, (particularly conserved) antigens (NspA, P6)
- the toxicity of the LPS remaining on the surface of the bleb
- the potential induction of an autoimmune response because of host-identical structures (for example the capsular polysaccharide in *Neisseria meningitidis* serogroup B, the lacto-N-neotetraose in *Neisseria* LPS, saccharide structure within ntHi LPS, saccharide structures within Pili).

Such problems may prevent the use of bleb vaccines as human vaccine reagents. This is particularly so for paediatric use (<4 years) where reactogenicity against bleb vaccines is particularly important, and where bleb vaccines (for instance the previously mentioned marketed MenB bleb vaccine) have been shown to be ineffective at immuno-protecting. Accordingly, the present invention provides methods of alleviating the above problems using genetically engineered bacterial strains, which result in improved bleb vaccines. Such methods will be especially useful in the generation of new vaccines against bacterial pathogens such as *Neisseiria meningitidis, Moraxella catarrhalis, Haemophilus influenzae, Pseudomonas aeruginosa,* and others.

The bleb vaccines of the disclosure are designed to focus the immune response on a few protective (preferably conserved) antigens or epitopes - formulated in a multiple component vaccine. Where such antigens are integral OMPs, the outer membrane vesicles of bleb vaccines will ensure their proper folding. This invention provides methods to optimize the LPS composition of OMV (bleb) vaccines . In addition the invention provides for the reduction in toxicity of lipid A by modification of the lipid portion or by changing the phosphoryl composition whilst retaining its adjuvant activity or by masking it. Each of these new methods of improvement individually improve the bleb vaccine, however a combination of one or more of these methods work in conjunction so as to produce an optimised engineered bleb vaccine which is immuno-protective and non-toxic - particularly suitable for paediatric use.

### SUMMARY OF THE INVENTION

The present invention provides a genetically-engineered bleb preparation from a Neisserial, *Moraxella catarrhalis* or *Haemophilus influenzae* Gram-negative bacterial strain characterized in that said preparation is obtainable by employing the following process:
d) a process of modifying lipid A portion of bacterial LPS within the bleb preparation, comprising the steps of identifying a msbB gene involved in rendering the lipid A portion of LPS toxic, engineering a bacterial strain so as to reduce or switch off expression of said gene, and making blebs from said strain.

Further aspects of the disclosure include, preferential processes for obtaining the above bleb preparation, including optimal positioning of strong promoters for the upregulation of expression of antigens within blebs, preferential antigens for upregulation and downreguation for various bacterial strains in order to obtain bleb preparations particularly suitable for vaccine use. Preferential formulations comprising the blebs of the invention are also provided which are particularly suitable for global vaccines against certain disease states. Vectors for producing the blebs of the invention, and modified bacterial strains from which the blebs of the invention are produced are still further aspects of the invention.

The present invention provides for the first time a bleb vaccine which is immuno-protective and non-toxic when used with children under 4 years of age.

### BRIEF DESCRIPTION OF THE DRAWINGS

**Figure 1****:** Reactivity of the 735 mAb on different colonies.
**Figure 2****:** Reactivities of specific monoclonal antibodies by whole cell Elisa.
**Figure 3****:** Schematic representation of the pCMK vectors used to deliver genes, operons and/or expression cassettes in the genome *of Neisseria meningitidis.*
**Figure 4****:** Analysis of PorA expression in total protein extracts of recombinant *N*.
*meningitidis* serogroupB (H44/76 derivatives). Total proteins were recovered from cps- (lanes 3 and 4), *cps- porA: : pCMK+* (lanes 2 and 5) and *cps- porA::nspA* (lanes 1 and 6) recombinant *N. meningitidis* serogroupB strains and were analyzed under SDS-PAGE conditions in a 12% polyacrylamide gel. Gels were stained with Coomassie blue (lanes 1 to 3) or transferred to a nitrocellulose membrane and immuno-stained with an anti-PorA monoclonal antibody.

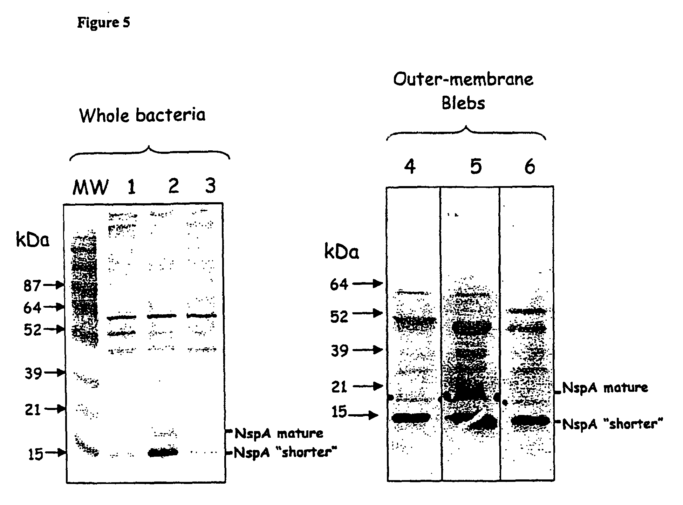

**Figure 5****:** Analysis of NspA expression in protein extracts of recombinant N. *meningitidis* serogroupB strains (H44/76 derivatives). Proteins were extracted from whole bacteria (lanes 1 to 3) or outer-membrane blebs preparations (lanes 4 to 6) separated by SDS-PAGE on a 12% acrylamide gel and analyzed by immuno-blotting using an anti-NspA polyclonal serum. Samples corresponding to *cps-* (lanes 1 and 6), *cps- pora::pCMK*+ (lanes 3 and 4) and *cps- porA::nspA* (lanes 2 and 5) were analyzed. Two forms of NspA were detected: a mature form (18kDa) co-migrating with the recombinant purified NspA, and a shorter form (15kDa).

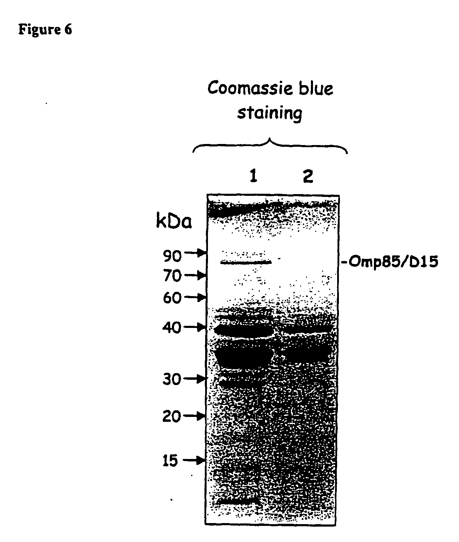

**Figure 6****:** Analysis of D15/omp85 expression in protein extracts of recombinant *N*. *meningitidis* serogroupB strains (H44/76 derivatives). Proteins were extracted from outer-membrane blebs preparations and were separated by SDS-PAGE on a 12% acrylamide gel and analyzed by immuno-blotting using an anti-omp85 polyclonal serum. Samples corresponding to *cps-* (lane 2), and *cps-, PorA+, pCMK+Omp85*/*D15* (lane 1) recombinant *N. meningitidis* serogroupB strains were analyzed.

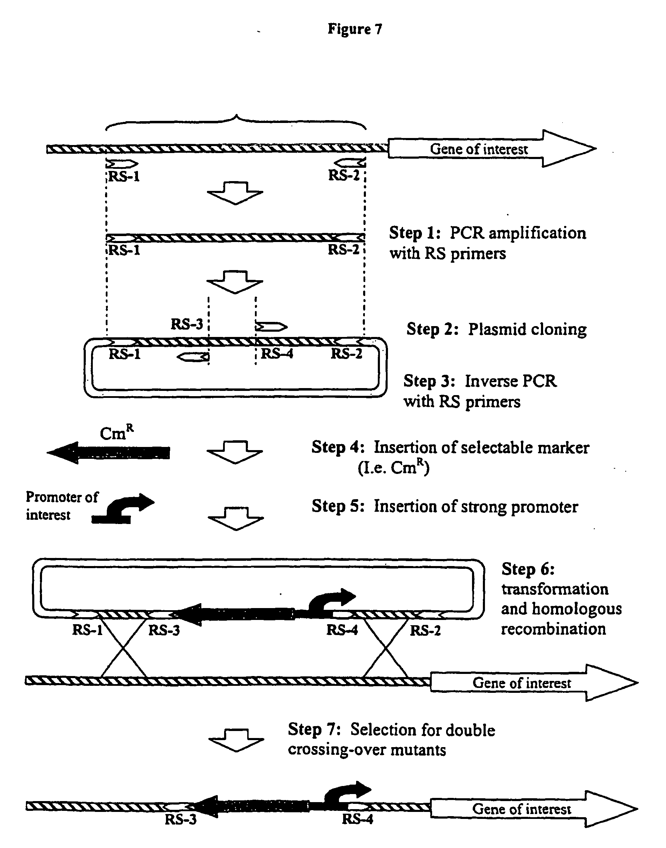

**Figure 7****:** General strategy for modulating gene expression by promoter delivery (RS stands for restriction site).

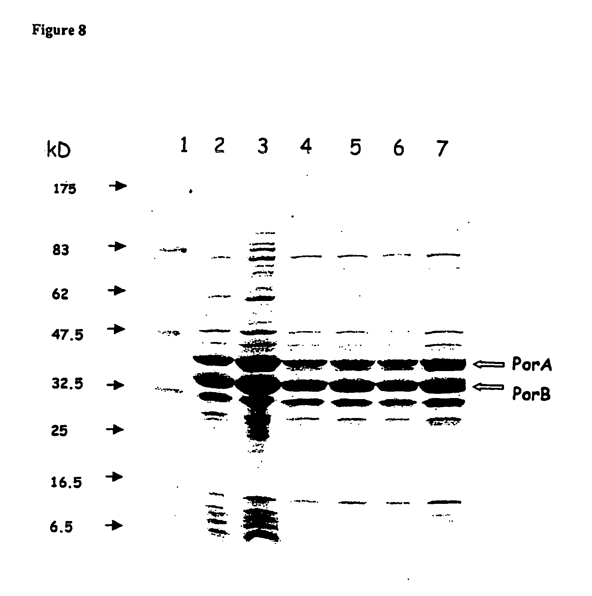

**Figure 8****:** Analysis of outer-membrane blebs produced by recombinant *N*. *meningitidis* serogroupB *cps-* strains (H44/76 derivatives). Proteins were extracted from outer-membrane bleb preparations and were separated by SDS-PAGE under reducing conditions on a 4-20% gradient polyacrylamide gel. The gel was stained with Coomassie brilliant blue R250. Lanes 2, 4, 6 corresponded to 5µg of total proteins whereas lanes 3, 5 and 7 were loaded with 10µg proteins.

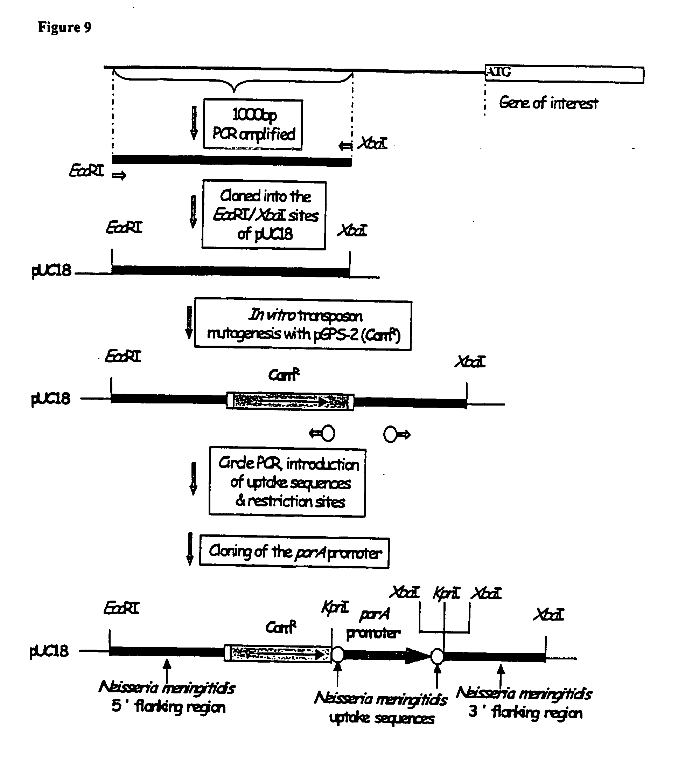

**Figure 9****:** Construction of a promoter replacement plasmid used to up-regulate the expression/production of Omp85/D15 in *Neisseria meningitidis* H44/76.

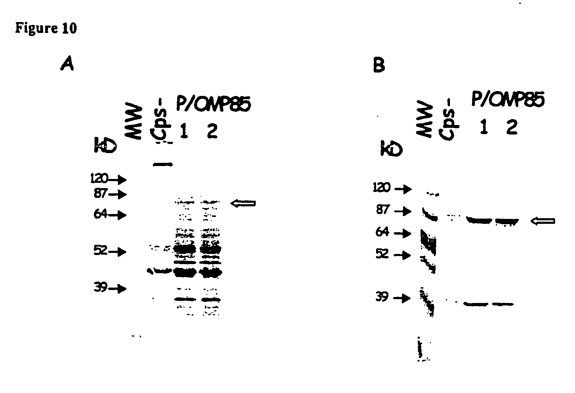

**Figure 10****:** Analysis of OMP85 expression in total protein extracts of recombinant *Nm*B (H44/76 derivatives). Gels were stained with Coomassie blue (A) or transferred to nitrocellulose membrane and immuno-stained with rabbit anti-OMP85 (*N.gono*) monoclonal antibody (B).

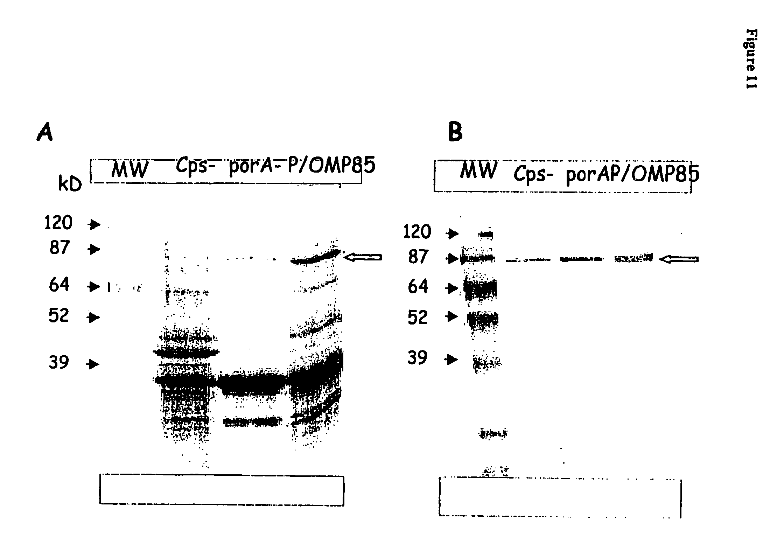

**Figure 11****:** Analysis of OMP85 expression in OMV preparations from recombinant *Nm*B (H44/76 derivatives). Gels were stained with Coomassie blue (A) or transferred to nitrocellulose membrane and immuno-stained with rabbit anti-OMP85 polyclonal antibody (B).

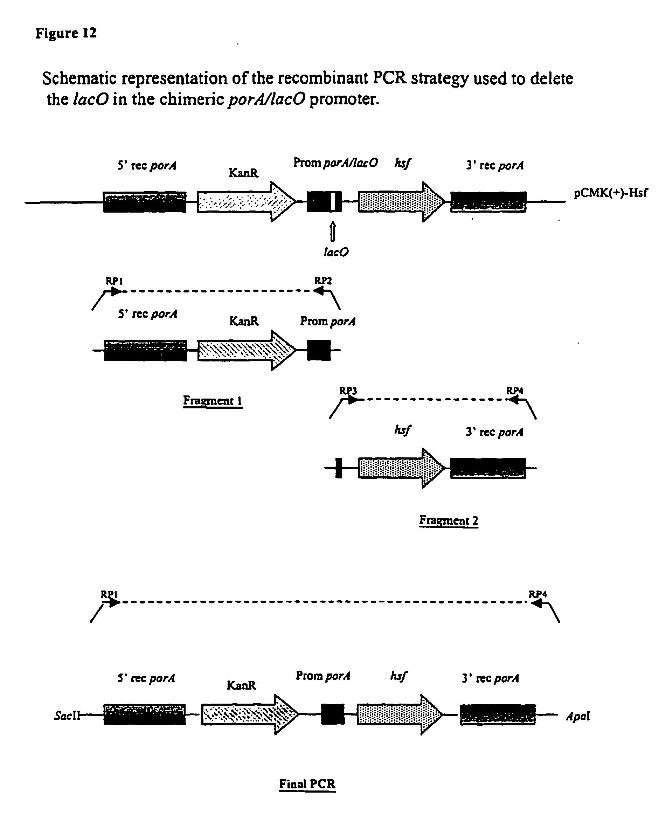

**Figure 12****:** Schematic representation of the recombinant PCR strategy used to delete the *lacO* in the chimeric *porA*/*lacO* promoter.

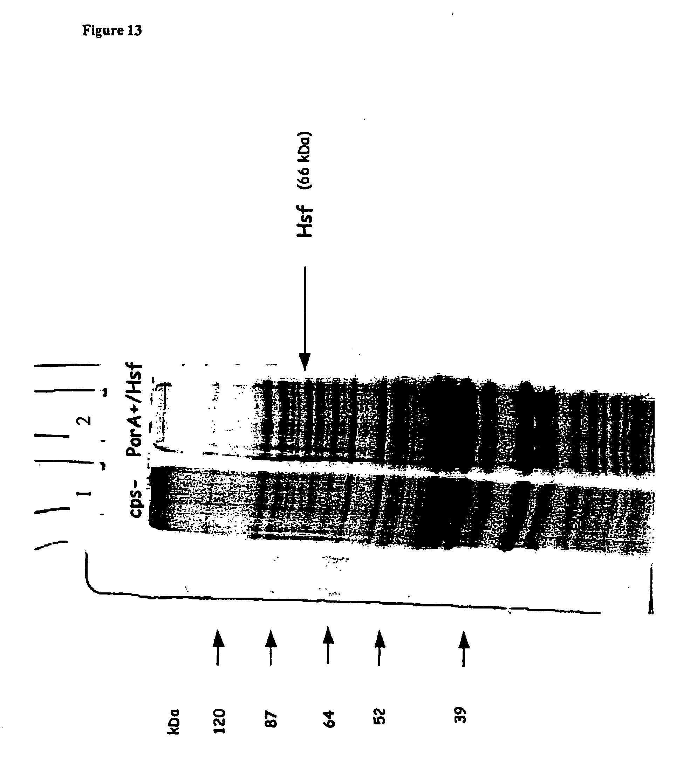

**Figure 13****:** Analysis of Hsf expression in total protein extracts of recombinant *N*. *meningitidis* serogroup B (H44/76 derivatives). Total proteins were recovered from Cps-PorA+(lanes 1), and Cps-PorA+/Hsf (lanes 2) recombinant *N. meningitidis* serogroup B strains and were analyzed under SDS-PAGE conditions in a 12% polyacrylamide gel. Gels were stained with Coomassie blue.

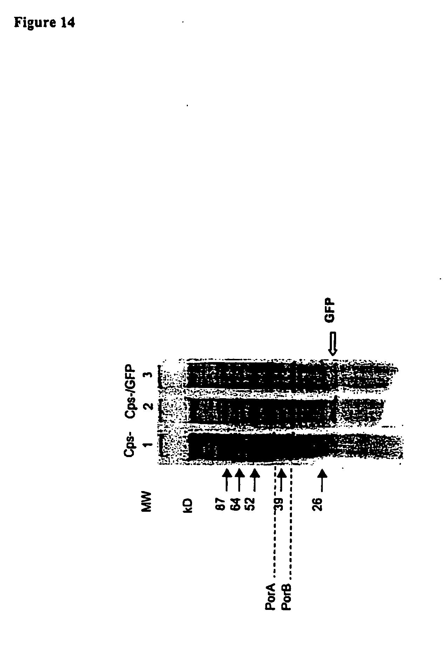

**Figure 14****:** Analysis of GFP expression in total protein extracts of recombinant *N*. *meningitidis* (H44/76 derivative). Total protein were recovered from Cps-, PorA+ (lanel), Cps-, PorA- GFP+ (lane2 & 3) recombinant strains. Proteins were separated by PAGE-SDS in a 12% polyacrylamide gel and then stained with Coomassie blue.

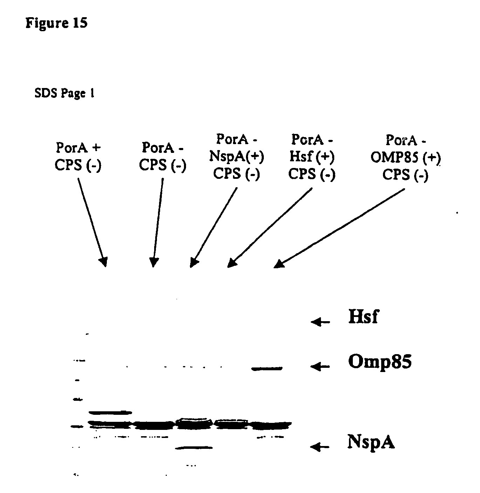

**Figure 15****:** Illustration of the pattern of major proteins on the surface of various recominant bleb preparations as analysed by SDS-PAGE (Coomassie staining).

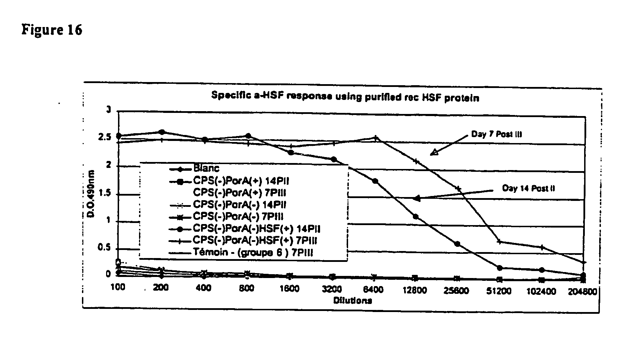

**Figure 16****:** Specific anti-Hsf response for various bleb and recombinant bleb preparations using purified recombinant Hsf protein.

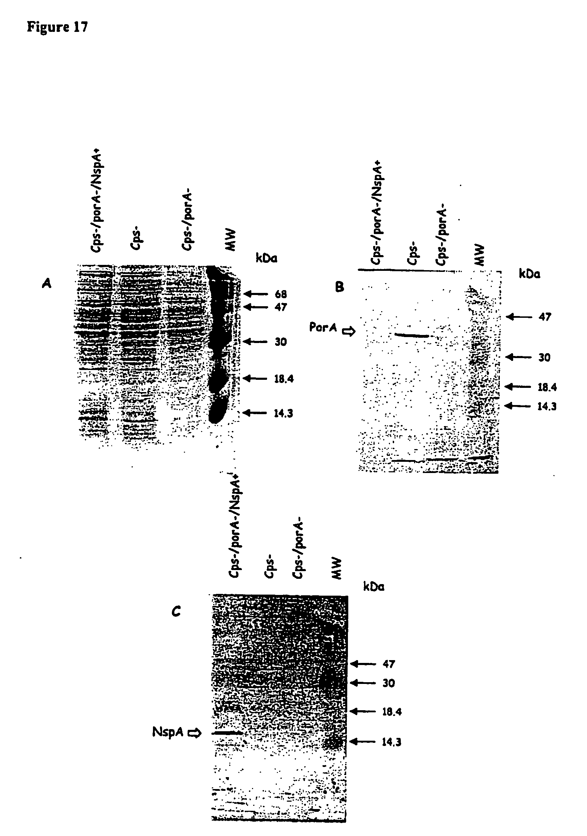

**Figure 17****:** Analysis of NspA expression in total protein extracts of recombinant *Nm*B (serogroup B derivatives). Gels were stained with Coomassie blue (A) or transferred to nitrocellulose membrane and immuno-stained with mouse anti-PorA monoclonal antibody (B) or mouse anti-NspA polyclonal antibody (C).

### DESCRIPTION OF THE INVENTION

The present invention relates to a general set of tools and methods capable of being used for manufacturing improved, genetically engineered blebs from Gram-negative bacterial strains. The invention includes methods used to make recombinant blebs less toxic and safer for their use in a human and/or animal vaccine. Moreover, the present invention also describes specific methods necessary for constructing, producing, obtaining and using recombinant, engineered blebs from various Gram-negative bacteria, for vaccine, therapeutic and/or diagnostic purposes. By the methods of the invention, the biochemical composition of bacterial blebs can be manipulated by acting upon/altering the expression of bacterial genes encoding products present in or associated with bacterial outer-membrane blebs (outer membrane proteins or OMPs). The production of blebs using a method of genetic modification to increase, decrease or render conditional the expression of one or more genes encoding outer-membrane components are also included in the scope of this invention.

For clarity, the term "expression cassette" will refer herein to all the genetic elements necessary to express a gene or an operon and to produce and target the corresponding protein(s) of interest to outer-membrane blebs, derived from a given bacterial host. A non-exhaustive list of these features includes control elements (transcriptional and/or translational), protein coding regions and targeting signals, with appropriate spacing between them. Reference to the insertion of promoter sequences means, for the purposes of this invention, the insertion of a sequence with at least a promoter function, and preferably one or more other genetic regulatory elements comprised within an expression cassette. Moreover, the term "integrative cassette" will refer herein to all the genetic elements required to integrate a DNA segment in given bacterial host. A non-exhaustive list of these features includes a delivery vehicle (or vector), with recombinogenic regions, and selectable and counter selectable markers.

Again for the purpose of clarity, the terms 'engineering a bacterial strain to produce less of said antigen' refers to any means to reduce the expression of an antigen of interest, relative to that of the non-modified (i.e., naturally occurring) bleb such that expression is at least 10% lower than that of the non-modified bleb. Preferably it is at least 50% lower. "Stronger promoter sequence" refers to a regulatory control element that increases transcription for a gene encoding antigen of interest. "Upregulating expression" refers to any means to enhance the expression of an antigen of interest, relative to that of the non-modified (i.e., naturally occurring) bleb. It is understood that the amount of 'upregulation' will vary depending on the particular antigen of interest but will not exceed an amount that will disrupt the membrane integrity of the bleb. Upregulation of an antigen refers to expression that is at least 10% higher than that of the non-modified bleb. Preferably it is at least 50% higher. More preferably it is at least 100% (2 fold) higher.

Aspects of the disclosure relate to individual methods for making improved engineered blebs, to a combination of such methods, and to the bleb compositions made as a result of these methods. Another aspect of the invention relates to the genetic tools used in order to genetically modify a chosen bacterial strain in order to extract improved engineered blebs from said strain.

The engineering steps of the processes of the invention can be carried out in a variety of ways known to the skilled person. For instance, sequences (e.g. promoters or open reading frames) can be inserted, and promoters/genes can be disrupted by the technique of transposon insertion. For instance, for upregulating a gene's expression, a strong promoter could be inserted via a transposon up to 2 kb upstream of the gene's initiation codon (more preferably 200-600 bp upstream, most preferably approximately 400 bp upstream). Point mutation or deletion may also be used (particularly for down-regulating expression of a gene).

Such methods, however, may be quite unstable or uncertain, and therefore it is preferred that the engineering step [particularly for processes a), b), c), d), e), h) and i) as described below] is performed via a homologous recombination event. Preferably, the event takes place between a sequence (a recombinogenic region) of at least 30 nucleotides on the bacterial chromosome, and a sequence (a second recombinogenic region) of at least 30 nucleotides on a vector transformed within the strain. Preferably the regions are 40-1000 nucleotides, more preferably 100-800 nucleotides, most preferably 500 nucleotides). These recombinogenic regions should be sufficiently similar that they are capable of hybridising to one another under highly stringent conditions (as defined later).

Recombination events may take place using a single recombinogenic region on chromosome and vector, or via a double cross-over event (with 2 regions on chromosome and vector). In order to perform a single recombination event, the vector should be a circular DNA molecule. In order to perform a double recombination event, the vector could be a circular or linear DNA molecule (see Figure 7). It is preferable that a double recombination event is employed and that the vector used is linear, as the modified bacterium so produced will be more stable in terms of reversion events. Preferably the two recombinogenic regions on the chromosome (and on the vector) are of similar (most preferably the same) length so as to promote double cross-overs. The double cross-over functions such that the two recombinogenic regions on the chromosome (separated by nucleotide sequence 'X') and the two recombinogenic regions on the vector (separated by nucleotide sequence 'Y') recombine to leave a chromosome unaltered except that X and Y have interchanged. The position of the recombinogenic regions can both be positioned upstream or down stream of, or may flank, an open reading frame of interest. These regions can consist of coding, non-coding, or a mixture of coding and non-coding sequence. The identity of X and Y will depend on the effect desired. X may be all or part of an open reading frame, and Y no nucleotides at all, which would result in sequence X being deleted from the chromosome. Alternatively Y may be a strong promoter region for insertion upstream of an open reading frame, and therefore X may be no nucleotides at all.

Suitable vectors will vary in composition depending what type of recombination event is to be performed, and what the ultimate purpose of the recombination event is. Integrative vectors used to deliver region Y can be conditionally replicative or suicide plasmids, bacteriophages, transposons or linear DNA fragments obtained by restriction hydrolysis or PCR amplification. Selection of the recombination event is selected by means of selectable genetic marker such as genes conferring resistance to antibiotics (for instance kanamycin, erythromycin, chloramphenicol, or gentamycin), genes conferring resistance to heavy metals and/or toxic compounds or genes complementing auxotrophic mutations (for instance *pur, leu, inert, aro).*

### Process a) and f) - Down regulation/Removal of Variable and non-protective immunodominant antigens

Many surface antigens are variable among bacterial strains and as a consequence are protective only against a limited set of closely related strains. An aspect of this disclosure covers the reduction in expression, or, preferably, the deletion of the gene(s) encoding variable surface protein(s) which results in a bacterial strain producing blebs which, when administered in a vaccine, have a stronger potential for cross-reactivity against various strains due to a higher influence exerted by conserved proteins (retained on the outer membranes) on the vaccinee's immune system. Examples of such variable antigens include: for *Neisseria -* pili (PilC) which undergoes antigenic variations, PorA, Opa, TbpB, FrpB; for *H*. *influenzae -* P2, P5, pilin, IgA1-protease; and for *Moraxella -* CopB, OMP106.

Other types of gene that could be down-regulated or switched off are genes which, *in vivo,* can easily be switched on (expressed) or off by the bacterium. As outer membrane proteins encoded by such genes are not always present on the bacteria, the presence of such proteins in the bleb preparations can also be detrimental to the effectiveness of the vaccine for the reasons stated above. A preferred example of the disclosure to down-regulate or delete is *Neisseria* Opc protein. Anti-Opc immunity induced by an Opc containing bleb vaccine would only have limited protective capacity as the infecting organism could easily become Opc⁻. *H. influenzae* HgpA and HgpB are other examples of such proteins.

In process a) of the disclosure, these variable or non-protective genes are down-regulated in expression, or terminally switched off. This has the above-mentioned surprising advantage of concentrating the immune system on better antigens that are present in low amounts on the outer surface of blebs.

The strain can be engineered in this way by a number of strategies including transposon insertion to disrupt the coding region or promoter region of the gene, or point mutations or deletions to achieve a similar result. Homologous recombination may also be used to delete a gene from a chromosome (where sequence X comprises part (preferably all) of the coding sequence of the gene of interest). It may additionally be used to change its strong promoter for a weaker (or no) promoter (where nucleotide sequence X comprises part (preferably all) of the promoter region of the gene, and nucleotide sequence Y comprises either a weaker promoter region [resulting in a decreased expression of the gene(s)/operon(s) of interest], or no promoter region). In this case it is preferable for the recombination event to occur within the region of the chromosome 1000 bp upstream of the gene of interest.

Alternatively, Y may confer a conditional transcriptional activity, resulting in a conditional expression of the gene(s)/operon(s) of interest (down-regulation). This is useful in the expression of molecules that are toxic to or not well supported by the bacterial host.

Most of the above-exemplified proteins are integral OMPs and their variability may be limited only to one or few of their surface exposed loops. Another aspect of this disclosure process g)] covers the deletion of DNA regions coding for these surface exposed loops which leads to the expression of an integral OMP containing conserved surface exposed loops. Surface exposed loops of *H. influenzae* P2 and P5 are preferred examples of proteins that could be transformed into cross-reactive antigens by using such a method. Again, homologous recombination is a preferred method of performing this engineering process.

### Process b) - Promoter delivery and modulation:

A further aspect of the disclosure relates to modifying the composition of blebs by altering *in situ* the regulatory region controlling the expression of gene(s) and/or operon(s) of interest. This alteration may include partial or total replacement of the endogenous promoter controlling the expression of a gene of interest, with one conferring a distinct transcriptional activity. This distinct transcriptional activity may be conferred by variants (point mutations, deletions and/or insertions) of the endogenous control regions, by naturally occurring or modified heterologous promoters or by a combination of both. Such alterations will preferably confer a transcriptional activity stronger than the endogenous one (introduction of a strong promoter), resulting in an enhanced expression of the gene(s)/operon(s) of interest (up-regulation). Such a method is particularly useful for enhancing the production of immunologically relevant Bleb components such as outer-membrane proteins and lipoproteins (preferably conserved OMPs, usually present in blebs at low concentrations).

Typical strong promoters that may be integrated in *Neisseria* are *porA* [SEQ ID NO: 24], *porB* [SEQ ID NO:26], *lgtF,* Opa, *p110, lst,* and *hpuAB.* PorA and PorB are preferred as constitutive, strong promoters. It has been established (Example 9) that the PorB promoter activity is contained in a fragment corresponding to nucleotides -1 to -250 upstream of the initation codon of *porB.* In *Moraxella,* it is preferred to use the ompH, ompG, ompE, OmpB1, ompB2, ompA, OMPCD and Omp106 promoters, and in *H. influenzae,* it is preferred to integrate the P2, P4, P1, P5 and P6 promoters.

Using the preferred double cross-over homologous recombination technology to introduce the promoter in the 1000 bp upstream region, promoters can be placed anywhere from 30-970 bp upstream of the initiation codon of the gene to be up-regulated. Although conventionally it is thought the promoter region should be relatively close to the open reading frame in order to obtain optimal expression of the gene, the present inventors have surprisingly found that placement of the promoter further away from the initiation codon results in large increases in expression levels. Thus it is preferred if the promoter is inserted 200-600 bp from the initiation codon of the gene, more preferably 300-500 bp, and most preferably approximately 400 bp from the initiation ATG.

### Process c) - Bleb components produced conditionally

The expression of some genes coding for certain bleb components is carefully regulated. The production of the components is conditionally modulated and depends upon various metabolic and/or environmental signals. Such signals include, for example, iron-limitation, modulation of the redox potential, pH and temperature variations, nutritional changes. Some examples of bleb components known to be produced conditionally include iron-regulated outer-membrane proteins from *Neisseiria* and *Moraxella* (for instance TbpB, LbpB), and substrate-inducible outer-membrane porins. The present disclosure covers the use of the genetic methods described previously (process a) or b)) to render constitutive the expression of such molecules. In this way, the influence of environmental signal upon the expression of gene(s) of interest can be overcome by modifying/replacing the gene's corresponding control region so that it becomes constitutively active (for instance by deleting part [preferably all] or the repressive control sequence - e.g. the operator region), or inserting a constitutive strong promoter. For iron regulated genes the *fur* operator may be removed. Alternatively, process i) of the disclosure may be used to deliver an additional copy of the gene/operon of interest in the chromosome which is placed artificially under the control of a constitutive promoter.

### Processes d), and e) - Detoxification of LPS

The toxicity of bleb vaccines presents one of the largest problems in the use of blebs in vaccines. A further aspect of the invention relates to methods of genetically detoxifying the LPS present in Blebs. Lipid A is the primary component of LPS responsible for cell activation. Many mutations in genes involved in this pathway lead to essential phenotypes. However, mutations in the genes responsible for the terminal modifications steps lead to temperature-sensitive (*htrB*) or permissive (*msbB*) phenotypes. Mutations resulting in a decreased (or no) expression of these genes result in altered toxic activity of lipid A. Indeed, the non-lauroylated (htrB mutant) or non-myristoylated (msbB mutant) lipid A are less toxic than the wild-type lipid A. Mutations in the lipid A 4'-kinase encoding gene (*lpxK*) also decreases the toxic activity of lipid A.

Process d) thus involves either the deletion of part (or preferably all) of msbB above open reading frame or promoter. Alternatively, the promoter could be replaced with a weaker promoter. Preferably the homologous recombination techniques described above are used to carry out the process.

The sequences of the htrB and msbB genes from *Neisseria meningitidis* B, *Moraxella catarrhalis,* and *Haemophilus influenzae* are additionally provided for this purpose.

LPS toxic activity could also be altered by introducing mutations in genes/loci involved in polymyxin B resistance (such resistance has been correlated with addition of aminoarabinose on the 4' phosphate of lipid A). These genes/loci could be *pmrE* that encodes a UDP-glucose dehydrogenase, or a region of antimicrobial peptide-resistance genes common to many enterobacteriaciae which could be involved in aminoarabinose synthesis and transfer. The gene *pmrF* that is present in this region encodes a dolicol-phosphate manosyl transferase (Gunn J.S., Kheng, B.L., Krueger J., Kim K.,Guo L., Hackett M., Miller S.I. 1998. Mol. Microbiol. 27: 1171-1182).

Mutations in the PhoP-PhoQ regulatory system, which is a phospho-relay two component regulatory system (f. i. PhoP constitutive phenotype, PhoP^{c}), or low Mg⁺⁺ environmental or culture conditions (that activate the PhoP-PhoQ regulatory system) lead to the addition of aminoarabinose on the 4'-phosphate and 2-hydroxymyristate replacing myristate (hydroxylation of myristate). This modified lipid A displays reduced ability to stimulate E-selectin expression by human endothelial cells and TNF-α secretion from human monocytes.

Process e) of the disclosure involves the upregulation of these genes using a strategy as described above (strong promoters being incorporated, preferably using homologous recombination techniques to carry out the process).

Alternatively, rather than performing any such mutation, a polymyxin B resistant strain could be used as a vaccine production strain (in conjunction with one or more of the other processes of the invention), as blebs from such strains also have reduced LPS toxicity (for instance as shown for meningococcus - van der Ley, P, Hamstra, HJ, Kramer, M, Steeghs, L, Petrov, A and Poolman, JT. 1994. In: Proceedings of the ninth international pathogenic Neisseria conference. The Guildhall, Winchester, England).

As a further alternative (and further aspect of the disclosure) the inventors provide a method of detoxifying a Gram-negative bacterial strain comprising the step of culturing the strain in a growth medium containing 0.1mg-100g of aminoarabinose per litre medium.

As a further still alternative of the disclosure, synthetic peptides that mimic the binding activity of polymyxin B (described below) may be added to the Bleb preparation in order to reduce LPS toxic activity (Rustici, A, Velucchi, M, Faggioni, R, Sironi, M, Ghezzi, P, Quataert, S, Green, B and Porro M 1993. Science 259: 361-365; Velucchi, M, Rustici, A, Meazza, C, Villa, P, Ghezzi, P and Porro, M. 1997. J. Endotox. Res. 4:).

### Process f) - Anchoring homologous or heterologous proteins to outer-membrane blebs whilst reducing the toxicity of LPS

A further aspect of this disclosure covers the use of genetic sequences encoding polymyxin B peptides (or analogues thereof) as a means to target fusion proteins to the outer-membrane. Polymyxin B is a cyclic peptide composed of non tRNA-encoded amino acids (produced by Gram-positive actinomycetal organisms) that binds very strongly to the Lipid A part of LPS present in the outer-membrane. This binding decreases the intrinsic toxicity of LPS (endotoxin activity). Peptides mimicking the structure of Polymyxin B and composed of canonical (tRNA encoded) amino acids have been developed and also bind lipid A with a strong affinity. These peptides have been used for detoxifying LPS. One of these peptides known as SAEP-2 (Nterminus-Lys-Thr-Lys-Cys-Lys-Phe-Leu-Lys-Lys-Cys-Cterminus) was shown to be very promising in that respect (Molecular Mapping and detoxifying of the Lipid A binding site by synthetic peptides (1993). Rustici, A., Velucchi, M., Faggioni, R., Sironi, M., Ghezzi, P., Quataert, S., Green, B. and M. Porro. Science 259, 361-365).

The present process f) of the disclosure provides an improvement of this use. It has been found that the use of DNA sequences coding for the SEAP-2 peptide (or derivatives thereof), fused genetically to a gene of interest (encoding for instance a T cell antigen or a protective antigen that is usually secreted such as a toxin, or a cytosolic or periplasmic protein) is a means for targeting the corresponding recombinant protein to the outer-membrane of a preferred bacterial host (whilst at the same time reducing the toxicity of the LPS).

This system is suitable for labile proteins which would not be directly exposed to the outside of the bleb. The bleb would therefore act as a delivery vehicle which would expose the protein to the immune system once the blebs had been engulfed by T-cells. Alternatively, the genetic fusion should also comprise a signal peptide or transmembrane domain such that the recombinant protein may cross the outer membrane for exposure to the host's immune system.

This targeting strategy might be of particular interest in the case of genes encoding proteins that are not normally targeted to the outer-membrane. This methodology also allows the isolation of recombinant blebs enriched in the protein of interest. Preferably, such a peptide targeting signal allows the enrichment of outer membrane blebs in one or several proteins of interest, which are naturally not found in that given subcellular localization. A non exhaustive list of bacteria that can be used as a recipient host for such a production of recombinant blebs includes *Neisseria meningitidis, Neisseiria gonorrhoeae Moraxella catarrhalis, Haemophilus influenzae, Pseudomonas aeruginosa, Chlamydia trachomatis,* and *Chlamydia pneumonia.*

Although it is preferred that the gene for the construct is engineered into the chromosome of the bacterium [using process i) of the disclosure], an alternative preferred embodiment of the disclosure is for SAEP-2-tagged recombinant proteins to be made independently, and attached at a later stage to a bleb preparation.

A further embodiment of the disclosure is the use of such constructs in a method of protein purification. The system could be used as part of an expression system for producing recombinant proteins in general. The SAEP-2 peptide tag can be used for affinity purification of the protein to which it is attached using a column containing immobilised lipid A molecules.

### Process h) - Cross-reactive polysaccharides

The isolation of bacterial outer-membrane blebs from encapsulated Gram-negative bacteria often results in the co-purification of capsular polysaccharide. In some cases, this "contaminant" material may prove useful since polysaccharide may enhance the immune response conferred by other bleb components. In other cases however, the presence of contaminating polysaccharide material in bacterial bleb preparations may prove detrimental to the use of the blebs in a vaccine. For instance, it has been shown at least in the case of *N. meningitidis* that the serogroup B capsular polysaccharide does not confer protective immunity and is susceptible to induce an adverse auto-immune response in humans. Consequently, process h) of the disclosure is the engineering of the bacterial strain for bleb production such that it is free of capsular polysaccharide. The blebs will then be suitable for use in humans. A particularly preferred example of such a bleb preparation is one from *N. meningitidis* serogroup B devoid of capsular polysaccharide.

This may be achieved by using modified bleb production strains in which the genes necessary for capsular biosynthesis and/or export have been impaired. Inactivation of the gene coding for capsular polysaccharide biosynthesis or export can be achieved by mutating (point mutation, deletion or insertion) either the control region, the coding region or both (preferably using the homologous recombination techniques described above). Moreover, inactivation of capsular biosynthesis genes may also be achieved by antisense over-expression or transposon mutagenesis. A preferred method of the disclosure is the deletion of some or all of the *Neisseria meningitidis cps* genes required for polysaccharide biosynthesis and export. For this purpose, the replacement plasmid pMF121 (described in Frosh et al.1990, Mol. Microbiol. 4:1215-1218) can be used to deliver a mutation deleting the *cpsCAD* (+ *galE*) gene cluster. Alternatively the *siaD* gene could be deleted, or down-regulated in expression (the meningococcal *siaD* gene encodes alpha-2,3-sialyltransferase, an enzyme required for capsular polysaccharide and LOS synthesis). Such mutations may also remove host-similar structures on the saccharide portion of the LPS of the bacteria.

### Process i) - Delivery of one or more further copies of a Gene and/or operon in a host chromosome, or delivery of a heterologous gene and/or operon in a host chromosome.

An efficient strategy to modulate the composition of a Bleb preparation is to deliver one or more copies of a DNA segment containing an expression cassette into the genome of a Gram-negative bacterium. A non exhaustive list of preferred bacterial species that could be used as a recipient for such a cassette includes *Neisseria meningitidis, Neisseiria gonorrhoeae, Moraxella catarrhalis, Haemophilus influenzae, Pseudomonas aeruginosa, Chlamydia trachomatis, Chlamydia pneumoniae.* The gene(s) contained in the expression cassette may be homologous (or endogenous) (i.e. exist naturally in the genome of the manipulated bacterium) or heterologous (i.e. do not exist naturally in the genome of the manipulated bacterium). The reintroduced expression cassette may consist of unmodified, "natural" promoter/gene/operon sequences or engineered expression cassettes in which the promoter region and/or the coding region or both have been altered. A non-exhaustive list of preferred promoters that could be used for expression includes the promoters *porA, porB, IbpB, tbpB, p110, lst, hpuAB* from *N. meningitidis* or *N. gonorroheae,* the promoters p2, p5, p4, ompF, pl, ompH, p6, hin47 from *H. influenzae,* the promoters ompH, ompG, ompCD, ompE, ompB1 ompB2, ompA of *M. catarrhalis,* the promoter λpL, *lac, tac, araB* of *Escherichia coli* or promoters recognized specifically by bacteriophage RNA polymerase such as the *E. coli* bacteriophage T7. A non-exhaustive list of preferred genes that could be expressed in such a system includes *Neisseria* NspA, Omp85, PilQ, TbpA/B complex, Hsf, PldA, HasR; *Chlamydia* MOMP, HMWP; *Moraxella* OMP106, HasR, PilQ, OMP85, PldA; *Bordetella pertussis* FHA, PRN, PT.

In a preferred embodiment of the disclosure the expression cassette is delivered and integrated in the bacterial chromosome by means of homologous and/or site specific recombination. Integrative vectors used to deliver such genes and/or operons can be conditionally replicative or suicide plasmids, bacteriophages, transposons or linear DNA fragments obtained by restriction hydrolysis or PCR amplification. Integration is preferably targeted to chromosomal regions dispensable for growth *in vitro.* A non exhaustive list of preferred loci that can be used to target DNA integration includes the *porA, porB, opa, opc, rmp, omp26, lecA, cps, lgtB* genes of *Neisseiria meningitidis* and *Neisseria gonorrhoeae,* the *P1, P5, hmw1*/*2, IgA-protease, fimE* genes of NTHi; the *lecA1, lecA2, omp106, uspA1, uspA2* genes of *Moraxella catarrhalis.* Alternatively, the expression cassette used to modulate the expression of bleb component(s) can be delivered into a bacterium of choice by means of episomal vectors such as circular/linear replicative plasmids, cosmids, phasmids, lysogenic bacteriophages or bacterial artificial chromosomes. Selection of the recombination event can be selected by means of selectable genetic marker such as genes conferring resistance to antibiotics (for instance kanamycin, erythromycin, chloramphenicol, or gentamycin), genes conferring resistance to heavy metals and/or toxic compounds or genes complementing auxotrophic mutations (for instance *pur, leu, met, aro*).

### Heterologous Genes - Expression of foreign proteins in outer-membrane blebs

Outer-membrane bacterial blebs represent a very attractive system to produce, isolate and deliver recombinant proteins for vaccine, therapeutic and/or diagnostic uses. A further aspect of this disclosure is in respect of the expression, production and targeting of foreign, heterologous proteins to the outer-membrane, and the use of the bacteria to produce recombinant blebs.

A preferred method of achieving this is via a process comprising the steps of: introducing a heterologous gene, optionally controlled by a strong promoter sequence, into the chromosome of a Gram-negative strain by homologous recombination. Blebs may be made from the resulting modified strain.

A non-exhaustive list of bacteria that can be used as a recipient host for production of recombinant blebs includes *Neisseria meningitidis, Neisseiria gonorrhoeae Moraxella catarrhalis, Haemophilus influenzae, Pseudomonas aeruginosa, Chlamydia trachomatis, Chlamydia pneumoniae.* The gene expressed in such a system can be of viral, bacterial, fungal, parasitic or higher eukaryotic origin.

A preferred application of the disclosure includes a process for the expression of *Moraxella, Haemophilus* and/or *Pseudomonas* outer-membrane proteins (integral, polytopic and/or lipoproteins) in *Neisseria meningitidis* recombinant blebs. The preferable integration loci are stated above, and genes that are preferably introduced are those that provide protection against the bacterium from which they were isolated. Preferred protective genes for each bacterium are described below.

Further preferred applications of the disclosure are: blebs produced from a modified *Haemophilus influenzae* strain where the heterologous gene is a protective OMP from *Moraxella catarrhalis;* and blebs produced from a modified *Moraxella catarrhalis* strain where the heterologous gene is a protective OMP from *Haemophilus influenzae* (preferred loci for gene insertion are given above, and preferred protective antigens are described below).

A particularly preferred application of this aspect of the disclosure is in the field of the prophylaxis or treatment of sexually-transmitted diseaseses (STDs). It is often difficult for practitioners to determine whether the principal cause of a STD is due to gonococcus or *Chlamydia trachomatis* infection. These two organisms are the main causes of salpingitis - a disease which can lead to sterility in the host. It would therefore be useful if a STD could be vaccinated against or treated with a combined vaccine effective against disease caused by both organisms. The Major Outer Membrane Protein (MOMP) of *C*. *trachomatis* has been shown to be the target of protective antibodies. However, the structural integrity of this integral membrane protein is important for inducing such antibodies. In addition, the epitopes recognised by these antibodies are variable and define more than 10 serovars. The previously described aspect of this disclosure allows the proper folding of one or more membrane proteins within a bleb outer membrane preparation. The engineering of a gonococcal strain expressing multiple *C*. *trachomatis* MOMP serovars in the outer membrane, and the production of blebs therefrom, produces a single solution to the multiple problems of correctly folded membrane proteins, the presentation of sufficient MOMP serovars to protect against a wide spectrum of serovars, and the simultaneous prophylaxis/treatment of gonococcal infection (and consequently the non-requirement of practitioners to initially decide which organism is causing particular clinical symptoms - both organisms can be vaccinated against simultaneously thus allowing the treatment of the STD at a very early stage). Preferred loci for gene insertion in the gonoccocal chromosome are give above. Other preferred, protective *C*. *trachomatis* genes that could be incorporated are HMWP, PmpG and those OMPs disclosed in WO 99/28475.

### Targeting of heterologous proteins to outer-membrane blebs:

The expression of some heterologous proteins in bacterial blebs may require the addition of outer-membrane targeting signal(s). The preferred method of the disclosure to solve this problem is by creating a genetic fusion between a heterologous gene and a gene coding for a resident OMP as a specific approach to target recombinant proteins to blebs. Most preferably, the heterologous gene is fused to the signal peptides sequences of such an OMP.

### Neisserial bleb preparations

One or more of the following genes (encoding protective antigens) are preferred for upregulation via processes b) and/or i) of the disclosure when carried out on a Neisserial strain, including gonococcus, and meningococcus (particularly *N*. *meningitidis* B): NspA (WO 96/29412), Hsf-like (WO 99/31132), Hap (PCT/EP99/02766), PorA, PorB, OMP85 (WO 00/23595), PilQ (PCT/EP99/03603), PldA (PCT/EP99/06718), FrpB (WO 96/31618), TbpA (US 5,912,336), TbpB, FrpA/FrpC (WO 92/01460), LbpA/LbpB (PCT/EP98/05117), FhaB (WO 98/02547), HasR (PCT/EP99/05989), lipo02 (PCT/EP99/08315), Tbp2 (WO 99/57280), MltA (WO 99/57280), and ctrA (PCT/EP00/00135). They are also preferred as genes which may be heterologously introduced into other Gram-negative bacteria.

One or more of the following genes are preferred for downregulation via process a) of the disclosure: PorA, PorB, PilC, TbpA, TbpB, LbpA, LbpB, Opa, and Opc.

The following genes downregulated via process d): msbB and optionally htrB

One or more of the following genes are preferred for upregulation via process e) of the disclosure: pmrA, pmrB, pmrE, and pmrF.

Preferred repressive control sequences for process c) of the_disclosure are: the *fur* operator region (particularly for either or both of the TbpB or LbpB genes); and the DtxR operator region.

One or more of the following genes are preferred for downregulation via process h) of the disclosure: galE, siaA, siaB, siaC, siaD, ctrA, ctrB, ctrC, and ctrD.

### Pseudomonas aeruginosa bleb preparations

One or more of the following genes (encoding protective antigens) are preferred for upregulation via processes b) and/or i) of the disclosure: PcrV, OprF, OprI. They are also preferred as genes which may be heterologously introduced into other Gram-negative bacteria.

### Moraxella catarrhalis bleb preparations

One or more of the following genes (encoding protective antigens) are preferred for upregulation via processes b) and/or i) of the disclosure: OMP106 (WO 97/41731 & WO 96/34960), HasR (PCT/EP99/03824), PilQ (PCT/EP99/03823), OMP85 (PCT/EP00/01468), lipo06 (GB 9917977.2), lipo10 (GB 9918208.1), lipo11 (GB 9918302.2), lipo18 (GB 9918038.2), P6 (PCT/EP99/03038), ompCD, CopB (Helminen ME, et al (1993) Infect. Immun. 61:2003-2010), D15 (PCT/EP99/03822), OmplA1 (PCT/EP99/06781), Hly3 (PCT/EP99/03257), LbpA and LbpB (WO 98/55606), TbpA and TbpB (WO 97/13785 & WO 97/32980), OmpE, UspA1 and UspA2 (WO 93/03761), and Omp21. They are also preferred as genes which may be heterologously introduced into other Gram-negative bacteria.

One or more of the following genes are preferred for downregulation via process a) of the disclosure: CopB, OMP106, OmpB1, TbpA, TbpB, LbpA, and LbpB.

The following gene is domnregulated via process d): msbB- and optionally htr

One or more of the following genes are preferred for upregulation via process e) of the disclosure: pmrA, pmrB, pmrE, and pmrF.

### Haemophilus influenzae bleb preparations

One or more of the following genes (encoding protective antigens) are preferred for upregulation via processes b) and/or i) of the disclosure: D15 (WO 94/12641), P6 (EP 281673), TbpA, TbpB, P2, P5 (WO 94/26304), OMP26 (WO 97/01638), HMW1, HMW2, HMW3, HMW4, Hia, Hsf, Hap, Hin47, and Hif (all genes in this operon should be upregulated in order to upregulate pilin). They are also preferred as genes which may be heterologously introduced into other Gram-negative bacteria.

One or more of the following genes are preferred for downregulation via process a) of the disclosure: P2, P5, Hif, IgA1-protease, HgpA, HgpB, HMW1, HMW2, Hxu, TbpA, and TbpB.

The following genes is downregulated via process d): msbB and optionally htrB

One or more of the following genes are preferred for upregulation via process e) of the disclosure: pmrA, pmrB, pmrE, and pmrF.

### Vaccine Formulations

A preferred embodiment of the invention is the formulation of the bleb preparations of the invention in a vaccine which may also comprise a pharmaceutically acceptable excipient.

The manufacture of bleb preparations from any of the aforementioned modified strains may be achieved by any of the methods well known to a skilled person. Preferably the methods disclosed in EP 301992, US 5,597,572, EP 11243 or US 4,271,147 are used. Most preferably, the method described in Example 8 is used.

Vaccine preparation is generally described in Vaccine Design ("The subunit and adjuvant approach" (eds Powell M.F. & Newman M.J.) (1995) Plenum Press New York).

The bleb preparations of the present invention may be adjuvanted in the vaccine formulation of the invention. Suitable adjuvants include an aluminium salt such as aluminum hydroxide gel (alum) or aluminium phosphate, but may also be a salt of calcium (particularly calcium carbonate), iron or zinc, or may be an insoluble suspension of acylated tyrosine, or acylated sugars, cationically or anionically derivatised polysaccharides, or polyphosphazenes.

Suitable Th1 adjuvant systems that may be used include, Monophosphoryl lipid A, particularly 3-de-O-acylated monophosphoryl lipid A, and a combination of monophosphoryl lipid A, preferably 3-de-O-acylated monophosphoryl lipid A (3D-MPL) together with an aluminium salt. An enhanced system involves the combination of a monophosphoryl lipid A and a saponin derivative particularly the combination of QS21 and 3D-MPL as disclosed in WO 94/00153, or a less reactogenic composition where the QS21 is quenched with cholesterol as disclosed in WO96/33739. A particularly potent adjuvant formulation involving QS21 3D-MPL and tocopherol in an oil in water emulsion is described in W095/17210 and is a preferred formulation.

The vaccine may comprise a saponin, more preferably QS21. It may also comprise an oil in water emulsion and tocopherol. Unmethylated CpG containing oligo nucleotides (WO 96/02555) are also preferential inducers of a TH1 response and are suitable for use in the present invention.

The vaccine preparation of the present invention may be used to protect or treat a mammal susceptible to infection, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts. Thus one aspect of the present invention is a method of immunizing a human host against a disease caused by infection of a gram-negative bacteria, which method comprises administering to the host an immunoprotective dose of the bleb preparation of the present invention.

The amount of antigen in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-100µg of protein antigen, preferably 5-50µg, and most typically in the range 5 - 25pg.

An optimal amount for a particular vaccine can be ascertained by standard studies involving observation of appropriate immune responses in subjects. Following an initial vaccination, subjects may receive one or several booster immunisations adequately spaced.

### Ghost or Killed Whole cell vaccines

The inventors envisage that the above improvements to bleb preparations and vaccines can be easily extended to ghost or killed whole cell preparations and vaccines (with identical advantages). The modified Gram-negative strains of the invention from which the bleb preparations are made can also be used to made ghost and killed whole cell preparations. Methods of making ghost preparations (empty cells with intact envelopes) from Gram-negative strains are well known in the art (see for example WO 92/01791). Methods of killing whole cells to make inactivated cell preparations for use in vaccines are also well known. The terms 'bleb preparations' and 'bleb vaccines' as well as the processes described throughout this document are therefore applicable to the terms 'ghost preparation' and 'ghost vaccine', and 'killed whole cell preparation' and 'killed whole cell vaccine', respectively, for the purposes of this disclosure.

### Combinations of methods a) - i)

It may be appreciated that one or more of the above processes may be used to produce a modified strain from which to make improved bleb preparations of the invention. Preferably one such process is used, more preferably two or more (2, 3, 4, 5, 6, 7, 8 or 9) of the processes are used in order to manufacture the bleb vaccine. As each additional method is used in the manufacture of the bleb vaccine, each improvement works in conjunction with the other methods used in order to make an optimised engineered bleb preparation.

A preferred meningococcal (particularly *N. meningitidis* B) bleb preparation of the disclosure comprises the use of processes a), b), d) and/or e), and h). Such bleb preparations are safe (no structures similar to host structures), non-toxic, and structured such that the host immune response will be focused on high levels of protective (and preferably conserved) antigens. All the above elements work together in order to provide an optimised bleb vaccine.

Similarly for M. catarrhalis and non-typeable H. influenzae, preferred bleb preparations comprise the use of processes a), b), and d) and/or e).

A further aspect of the invention is thus an immuno-protective and non-toxic Gram-negative bleb; vaccine of the invention suitable for paediatric use.

By paediatric use it is meant use in infants less than 4 years old.

By immunoprotective it is meant that at least 40% (and preferably 50, 60, 70, 80, 90 and 100%) of infants seroconvert (4-fold increase in bactericidal activity [the dilution of antisera at which 50% of bacteria die - see for example PCT/EP98/05117]) against a set of heterologous strains to be selected from the major clonal groups known. For meningococcus B these stains should have a different PorA type from the bleb production strain, and should preferably be 2, 3, 4 or, most preferably, all 5 of strains H44/76, M97/252078, BZ10, NGP165 and CU385. For non-typeable *H*. *influenzae,* the strains should preferably be 2, 3, 4 or, most preferably, all 5 of strains 3224A, 3219C, 3241 A, 640645, and A840177. For *M*. *catarrhalis,* the strains should preferably be 2, 3, 4 or, most preferably, all 5 of strains ATCC 43617, 14, 358, 216 and 2926.

By non-toxic it is meant that there is a significant (2-4 fold, preferably 10 fold) decrease of endotoxin activity as measured by the well-known LAL and pyrogenicity assays.

### Vaccine Combinations

A further aspect of the invention are vaccine combinations comprising the bleb preparations of the invention with other antigens which are advantageously used against certain disease states. It has been found that blebs are particularly suitable for formulating with other antigens, as they advantageously have an adjuvant effect on the antigens they are mixed with.

In one preferred combination, the meningoccocus B bleb preparations of the invention are formulated with 1, 2, 3 or preferably all 4 of the following meningococcal capsular polysaccharides which may be plain or conjugated to a protein carrier: A, C, Y or W. Such a vaccine may be advantageously used as a global meningococcus vaccine. Rather than use the meningoccocus B bleb preparations of the invention, it is also envisaged that the formulation of the disclosure could alternatively contain wild-type meningococcus B bleb preparations from 2 or more (preferably several) strains belonging to several subtype/serotypes (for instance chosen from P1.15, P1.7,16, P1.4, and P1.2).

In a further preferred embodiment, the meningoccocus B bleb preparations of the invention [or the aforementioned mix of 2 or more wild-type meningococcus B bleb preparations], preferably formulated with 1, 2, 3 or all 4 of the plain or conjugated meningococcal capsular polysaccharides A, C, Y or W, are formulated with a conjugated *H. influenzae* b capsular polysaccharide, and one or more plain or conjugated pneumococcal capsular polysaccharides. Optionally, the vaccine may also comprises one or more protein antigens that can protect a host against *Streptococcus pneumoniae* infection. Such a vaccine may be advantageously used as a global meningitis vaccine.

The pneumococcal capsular polysaccharide antigens are preferably selected from serotypes 1, 2, 3, 4, 5, 6B, 7F, 8, 9N, 9V, 10A, 11A, 12F, 14, 15B, 17F, 18C, 19A, 19F, 20, 22F, 23F and 33F (most preferably from serotypes 1, 3, 4, 5, 6B, 7F, 9V, 14, 18C, 19F and 23F).

Preferred pneumococcal proteins antigens are those pneumococcal proteins which are exposed on the outer surface of the pneumococcus (capable of being recognised by a host's immune system during at least part of the life cycle of the pneumococcus), or are proteins which are secreted or released by the pneumococcus. Most preferably, the protein is a toxin, adhesin, 2-component signal tranducer, or lipoprotein of *Streptococcus pneumoniae,* or fragments thereof. Particularly preferred proteins include, but are not limited to: pneumolysin (preferably detoxified by chemical treatment or mutation) [Mitchell et al. Nucleic Acids Res. 1990 Jul 11; 18(13): 4010 "Comparison of pneumolysin genes and proteins from Streptococcus pneumoniae types 1 and 2.", Mitchell et al. Biochim Biophys Acta 1989 Jan 23; 1007(1): 67-72 "Expression of the pneumolysin gene in Escherichia coli: rapid purification and biological properties.", WO 96/05859 (A. Cyanamid), WO 90/06951 (Paton et al), WO 99/03884 (NAVA)]; PspA and transmembrane deletion variants thereof (US 5804193 - Briles et al.); PspC and transmembrane deletion variants thereof (WO 97/09994 - Briles et al); PsaA and transmembrane deletion variants thereof (Berry & Paton, Infect Immun 1996 Dec;64(12):5255-62 "Sequence heterogeneity of PsaA, a 37-kilodalton putative adhesin essential for virulence of Streptococcus pneumoniae*");* pneumococcal choline binding proteins and transmembrane deletion variants thereof; CbpA and transmembrane deletion variants thereof (WO 97/41151; WO 99/51266); Glyceraldehyde-3-phosphate - dehydrogenase (Infect. Immun. 1996 64:3544); HSP70 (WO 96/40928); PcpA (Sanchez-Beato et al. FEMS Microbiol Lett 1998, 164:207-14); M like protein, SB patent application No. EP 0837130; and adhesin 18627, SB Patent application No. EP 0834568. Further preferred pneumococcal protein antigens are those disclosed in WO 98/18931, particularly those selected in WO 98/18930 and PCT/US99/30390.

In a further preferred embodiment, the *Moraxella catarrhalis* bleb preparations of the invention are formulated with one or more plain or conjugated pneumococcal capsular polysaccharides, and one or more antigens that can protect a host against non-typeable *H. influenzae* infection. Optionally, the vaccine may also comprise one or more protein antigens that can protect a host against *Streptococcus pneumoniae* infection. The vaccine may also optionally comprise one or more antigens that can protect a host against RSV and/or one or more antigens that can protect a host against influenza virus. Such a vaccine may be advantageously used as a global otitis media vaccine.

Preferred non-typeable *H. influenzae* protein antigens include Fimbrin protein (US 5766608) and fusions comprising peptides therefrom (eg LB1 Fusion) (US 5843464 - Ohio State Research Foundation), OMP26, P6, protein D, TbpA, TbpB, Hia, Hmw1, Hmw2, Hap, and D15.

Preferred influenza virus antigens include whole, live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof.

Preferred RSV (Respiratory Syncytial Virus) antigens include the F glycoprotein, the G glycoprotein, the HN protein, or derivatives thereof.

In a still further preferred embodiment, the non-typeable *H. influenzae* bleb preparations of the invention are formulated with one or more plain or conjugated pneumococcal capsular polysaccharides, and one or more antigens that can protect a host against *M. catarrhalis* infection. Optionally, the vaccine may also comprise one or more protein antigens that can protect a host against *Streptococcus pneumonia* infection. The vaccine may also optionally comprise one or more antigens that can protect a host against RSV and/or one or more antigens that can protect a host against influenza virus. Such a vaccine may be advantageously used as a global otitis media vaccine.

### Nucleotide sequences of the invention

A further aspect of the disclosure relates to the provision of new nucleotide sequences which may be used in the processes of the invention. Specific upstream regions from various genes from various strains are provided which can be used in, for instance, processes a), b), d) and h). In addition, coding regions are provided for performing process d).

### General method for the analysis of the non-coding flanking region of a bacterial gene, and its exploitation for modulated expression of the gene in blebs

The non-coding flanking regions of a specific gene contain regulatory elements important in the expression of the gene. This regulation takes place both at the transcriptional and translational level. The sequence of these regions, either upstream or downstream of the open reading frame of the gene, can be obtained by DNA sequencing. This sequence information allows the determination of potential regulatory motifs such as the different promoter elements, terminator sequences, inducible sequence elements, repressors, elements responsible for phase variation, the Shine-Dalgarno sequence, regions with potential secondary structure involved in regulation, as well as other types of regulatory motifs or sequences.

This sequence information allows the modulation of the natural expression of the gene in question. The upregulation of the gene expression may be accomplished by altering the promoter, the Shine-Dalgarno sequence, potential repressor or operator elements, or any other elements involved. Likewise, downregulation of expression can be achieved by similar types of modifications. Alternatively, by changing phase variation sequences, the expression of the gene can be put under phase variation control, or may be uncoupled from this regulation. In another approach, the expression of the gene can be put under the control of one or more inducible elements allowing regulated expression. Examples of such regulation includes, but is not limited to, induction by temperature shift, addition of inductor substrates like selected carbohydrates or their derivatives, trace elements, vitamins, co-factors, metal ions, etc.

Such modifications as described above can be introduced by several different means. The modification of sequences involved in gene expression can be done *in vivo* by random mutagenesis followed by selection for the desired phenotype. Another approach consists in isolating the region of interest and modifying it by random mutagenesis, or site-directed replacement, insertion or deletion mutagenesis. The modified region can then be reintroduced into the bacterial genome by homologous recombination, and the effect on gene expression can be assessed. In another approach, the sequence knowledge of the region of interest can be used to replace or delete all or part of the natural regulatory sequences. In this case, the regulatory region targeted is isolated and modified so as to contain the regulatory elements from another gene, a combination of regulatory elements from different genes, a synthetic regulatory region, or any other regulatory region, or to delete selected parts of the wild-type regulatory sequences. These modified sequences can then be reintroduced into the bacterium via homologous recombination into the genome.

In process b) of the disclosure, for example, the expression of a gene can be modulated by exchanging its promoter with a stronger promoter (through isolating the upstream sequence of the gene, in vitro modification of this sequence, and reintroduction into the genome by homologous recombination). Upregulated expression can be obtained in both the bacterium as well as in the outer membrane vesicles shed (or made) from the bacterium.

In other preferred examples, the described approaches can be used to generate recombinant bacterial strains with improved characteristics for vaccine applications, as described above. These can be, but are not limited to, attenuated strains, strains with increased expression of selected antigens, strains with knock-outs (or decreased expression) of genes interfering with the immune response, and strains with modulated expression of immunodominant proteins.

SEQ ID NO:2-23, 25, 27-38 are all Neisserial upstream sequences (upstream of the initiation codon of various preferred genes) comprising approximately 1000 bp each. SEQ ID NO: 39-62 are all *M. catarrhalis* upstream sequences (upstream of the initiation codon of various preferred genes) comprising approximately 1000 bp each. SEQ ID NO: 63-75 are all *H. influenzae* upstream sequences (upstream of the initiation codon of various preferred genes) comprising approximately 1000 bp each. All of these can be used in genetic methods (particularly homologous recombination) for up-regulating, or down-regulating the open reading frames to which they are associated (as described before). SEQ ID NO: 76-81 are the coding regions for the HtrB and MsbB genes from Neisseria, *M. catarrhalis,* and *Haemophilus influenzae.* These can be used in genetic methods (particularly homologous recombination) for down-regulating (in particular deleting) part (preferably all) of these genes [process d)].

Another aspect of the disclosure is thus an isolated polynucleotide sequence which hybridises under highly stringent conditions to at least a 30 nucleotide portion of the nucleotides in SEQ ID NO: 2-23, 25, 27-81 or a complementary strand thereof. Preferably the isolated sequence should be long enough to perform homologous recombination with the chromosomal sequence if it is part of a suitable vector-namely at least 30 nucleotides (preferably at least 40, 50, 60, 70, 80, 90, 100, 200, 300, 400, or 500 nucleotides). More preferably the isolated polynucleotide should comprise at least 30 nucleotides (preferably at least 40, 50, 60, 70, 80, 90, 100, 200, 300, 400, or 500 nucleotides) of SEQ ID NO: 2-23, 25, 27-81 or a complementary strand thereof.

As used herein, highly stringent hybridization conditions include, for example, 6X SSC, 5X Denhardt, 0.5% SDS, and 100 µg/mL fragmented and denatured salmon sperm DNA hybridized overnight at 65 °C and washed in 2X SSC, 0.1% SDS one time at room temperature for about 10 minutes followed by one time at 65 °C for about 15 minutes followed by at least one wash in 0.2X SCC, 0.1% SDS at room temperature for at least 3-5 minutes.

A further aspect is the use of the isolated polynucleotide sequences of the disclosure in performing a genetic engineering event (such as transposon insertion, or site specific mutation or deletion, but preferably a homologous recombination event) within 1000 bp upstream of a Gram-negative bacterial chromosomal gene in order to either increase or decrease expression of the gene. Preferably the strain in which the recombination event is to take place is the same as the strain from which the upstream sequences of the invention were obtained. However, the meningococcus A, B, C, Y and W and gonococcus genomes are sufficiently similar that upstream sequence from any of these strains may be suitable for designing vectors for performing such events in the other strains. This is may also be the case for *Haemophilus influenzae* and non-typeable *Haemophilus influenzae.*

### EXAMPLES

The examples below are carried out using standard techniques, which are well known and routine to those of skill in the art, except where otherwise described in detail. The examples are illustrative, but do not limit the invention.

### Example 1: Construction of a Neisseiria meningitidis serogroup B strain lacking capsular polysaccharides.

The plasmid pMF121 (Frosch et al., 1990) has been used to construct a *Neisseria meningitidis B* strain lacking the capsular polysaccharide. This plasmid contains the flanking regions of the gene locus coding for the biosynthesis pathway of the group B polysaccharide (B PS), and the erythromycin resistance gene. Deletion of the B PS resulted in loss of expression of the group B capsular polysaccharide as well as a deletion in the active copy of *gaLE* leading to the synthesis of galactose deficient LPS.

### Strain transformation:

*Neisseria meningitidis* B H44/76 strain (B:15:P1.7, 16;Los 3,7,9) was selected for transformation. After an overnight CO₂ incubation on MH plate (without erythromycin), cells were collected in liquid MH containing 10 mM MgCl₂ (2 ml were used per MH plate) and diluted up to an OD of 0.1 (550 nm). To this 2 ml solution, 4 µl of the plasmid pMF121 stock solution (0.5 µg/ml) were added for a 6 hours incubation period at 37°C (with shaking). A control group was done with the same amount of *Neisseria meningitidis* B bacteria, but without addition of plasmid. After the incubation period, 100 µl of culture, as such, at 1/10, 1/100 and 1/1000 dilutions, were put in MH plates containing 5, 10, 20, 40 or 80 µg erythromycin /ml before incubation for 48 hours at 37°C.

### Colony blotting:

After plate incubation, 20 colonies were grown and selected from the 10 and 20 µg erythromycin/ml MH plates, while there was no colony growth in the control group without plasmid transformation. The H44/76 wild type strain was unable to grow in the selected erythromycin plates (10 to 80 µg erythromycin/ml). The day after, all the visible colonies were placed on new MH plates without erythromycin in order to let them grow. Afterwards, they were transferred onto nitrocellulose sheets (colony blotting) for presence of B polysaccharide. Briefly, colonies were blotted onto a nitrocellulose sheet and rinsed directly in PBS-0.05 % Tween 20 before cell inactivation for 1 hour at 56°C in PBS-0.05% Tween 20 (diluant buffer). Afterwards, the membrane was overlaid for one hour in the diluant buffer at room temperature (RT). Then, sheets were washed again for three times 5 minutes in the diluant buffer before incubation with the anti-B PS 735 Mab (Boerhinger) diluted at 1/3000 in the diluant buffer for 2 hours at RT. After a new washing step (3 times 5 minutes), the monoclonal antibody was detected with a biotinylated anti-mouse Ig from Amersham (RPN 1001) diluted 500 times in the diluant buffer (one hour at RT) before the next washing step (as described above). Afterwards, sheets were incubated for one hour at RT with a solution of streptavidin-peroxidase complex diluted 1/1000 in the diluant buffer. After the last three washing steps using the same method, nitrocellulose sheets were incubated for 15 min in the dark using the revelation solution (30 mg of 4-chloro-1-naphtol solution in 10 ml methanol plus 40 ml PBS and 30 mcl of H₂O₂ 37% from Merck). The reaction was stopped with a distillated water-washing step.

### Whole cell Elisas:

Whole cell Elisas were also done using the two transformed colonies ("D" and "R") and the wild type strain (H44/76) as coated bacteria (20 µg protein/ml), and a set of different monoclonal antibodies used to characterize *Neisseria meningitidis* strains. The following Mabs were tested: anti-B PS (735 from Dr Frosch), and the other Mabs from NIBSC: anti-B PS (Ref 95/750) anti-P1.7 (A-PorA, Ref 4025), anti-P1.16 (A-PorA, Ref 95/720), anti-Los 3,7,9 (A-LPS, Ref 4047), anti-Los 8 (A-LPS, Ref 4048), and anti-P1.2 (A-PorA Ref 95/696).

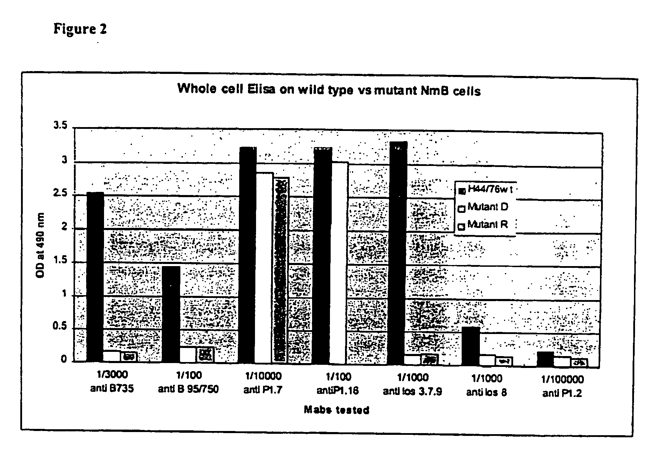

Microtiter plates (Maxisorp, Nunc) were coated with 100 µl of the recombinant meningococcal B cells solution overnight (ON) at 37°C at around 20 µg/ml in PBS. Afterwards, plates are washed three times with 300 µl of 150 mM NaCl - 0.05 % Tween 20, and were overlaid with 100 µl of PBS-0.3 % Casein and incubated for 30 min at room temperature with shaking. Plates were washed again using the same procedure before incubation with antibodies. Monoclonal antibodies (100 µl) were used at different dilutions (as shown in Figure 2) in PBS-0.3 % Casein 0.05 % Tween 20 and put onto the microplates before incubation at room temperature for 30 min with shaking, before the next identical washing step. 100 µl of the anti-mouse Ig (from rabbit, Dakopatts E0413) conjugated to biotin and diluted at 1/2000 in PBS-0.3 % Casein - 0.05 % Tween 20 were added to the wells to detect bound monoclonal antibodies. After the washing step (as before), plates were incubated with a streptavidin-peroxidase complex solution (100 µl of the Amersham RPN 1051) diluted at 1/4000 in the same working solution for 30 min at room temperature under shaking conditions. After this incubation and the last washing step, plates are incubated with 100 µl of the chromogen solution (4 mg orthophenylenediamine (OPD) in 10 ml 0.1 M citrate buffer pH4.5 with 5 µl H₂O₂) for 15 min in the dark. Plates are then read at 490/620 nm using a spectrophotometer.

### Results:

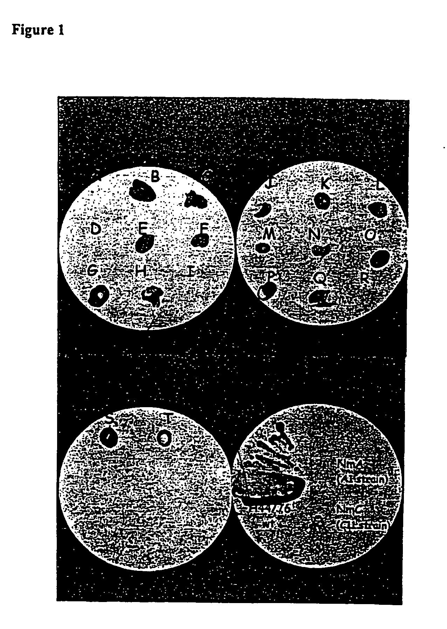

Figure 1 shows that from the 20 isolated colonies, which were able to growth on the selected medium with erythromycin, only two (the "D" and the "R") colonies were shown negative for presence of B polysaccharide. Among the others, 16 were clearly positive for B PS and still resistant to erythromycin. This indicated that they integrated the plasmid into their genome, but in the wrong orientation, and keeping intact the B PS and LPS gene (no double crossing-over). Positive and negative controls were also tested on the plates, and showed that the H44/76 wild type NmB strain was clearly positive for the B polysaccharide, while meningococcus A (A1) and meningococcus C (C11) strains were clearly negative with this anti-B PS 735 Mab. These results indicate that around 10 % of the selected colonies correctly integrated the plasmid in their genome by making a double crossing-over, while the other strains/colonies were obtained after a simple crossing-over, keeping the B PS and LPS genes intact and expressed.

Using whole cell Elisa, results (Figure 2 and the Table below) clearly indicate that the two "D" and "R" transformants (derived from D and R colonies) can not be recognized anymore by the anti-B PS Mabs (735 and 95/750), nor by the anti-Los 3,7,9 and anti-Los 8 Mabs. However, when using specific anti-PorA Mabs, there is a clear reaction with the anti-P1.7 and anti-P1.16 Mabs on the cells, as also observed in the wild-type strain. No reaction was observed with a non-specific anti-PorA Mab (anti-P1.2 mab). These results confirm that the PorA protein, and specifically P1.7 and P1.16 epitopes are still present after transformation, while B polysaccharide and Los 3.7,9 and Los 8 epitopes (LPS) were not.

**Table : Specificities of the monoclonal antibodies tested**

| **Mabs Tested** | **Directed against** | **Result** |
|---|---|---|
| Anti-B PS 735 | B polysaccharide | ++ on the wild type strain |
| | | (-) on the "D" and "R" mutants |
| Anti-B PS 95/750 from NIBSC | B PS | ++ on the wild type strain |
| | | (-) on the "D" and "R" mutants |
| Anti-P1.7 (NIBSC) | Loop 1 of Porin A | ++ on all wild type and mutants strains |
| Anti-P1.16 (NIBSC) | Loop 4 of Porin A | ++ on all wild type and mutants strains |
| Anti-Los 3,7,9 | LPS | ++ on the wild type strain |
| | | (-) on the "D" and "R" mutants |
| Anti-Los 8 (NIBSC) | LPS | +/- on the wild type strain |
| | | (-) on the "D" and "R" mutants |
| Anti-P1.2 (NIBSC) | Anti-Porin A Sero-subtype 1.2 | (-) on all wild type and mutants strains |

### Example 2: Construction of versatile gene delivery vectors (the pCMK series) targeting integration in the porA locus of Neisseiria meningitidis.

A plasmid allowing homologous recombination and stable integration of foreign DNA in the *porA* locus of *Neisseiria meningitidis* was constructed. This delivery vector (genes, operons and/or expression cassettes) is useful for constructing *Neisseiria meningitidis* strains producing recombinant, improved blebs. Typically, such a vector contains at least: (1) a plasmid backbone replicative in *E. coli* but not in *Neisseria meningitidis* (a suicide plasmid), (2) at least one, but preferably two regions of homology for targeting the integration in a chromosomal locus such as porA, (3) Efficient transcriptional (promoter, regulatory region and terminator) and translational (optimised ribosome binding site and initiation codon) signals functional in *Neisseria meningitidis,* (4) a multiple cloning site and (5) selectable gene(s) allowing the maintenance of the plasmid in *E. coli* and the selection of integrants in *Neisseria meningitidis.* Additional elements include, for example, uptake sequences to facilitate the entry of foreign DNA in *Neisseiria meningitidis,* and counter selectable markers such as *sacB, rpsL, gltS* to enhance the frequency of double cross-over events.

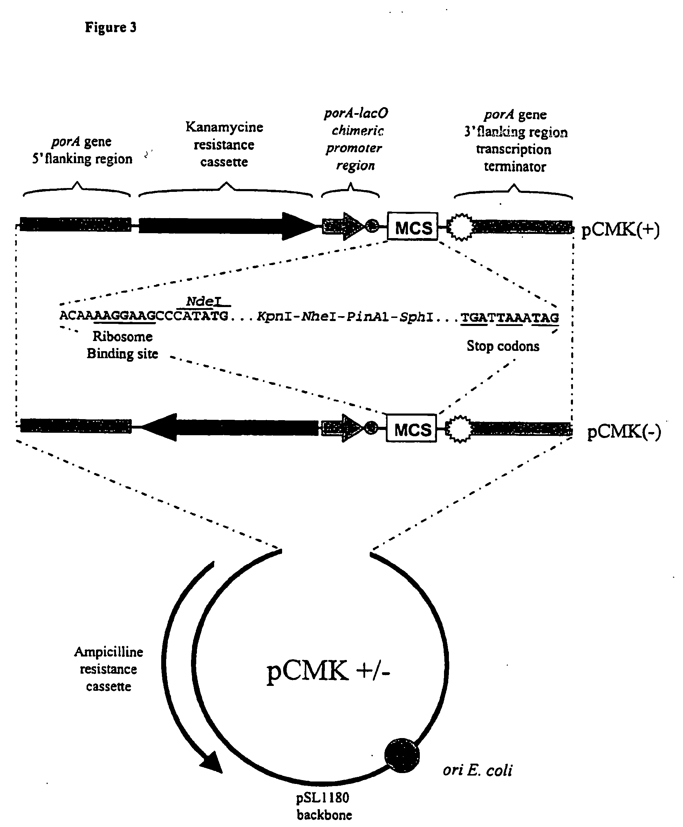

A schematic drawing of the vector constructed in this example and designated pCMK is represented in Figure 3. Its corresponding complete nucleotide sequence is shown in SEQ. ID NO:1. pCMK derives from a pSL1180 backbone (PharmaciaBiotech, Sweeden), a high copy number plasmid replicative in *E*. *coli,* harbouring the *bla* gene (and thereby conferring resistance to ampicillin). In addition to this, pCMK functionally contains two *porA* flanking regions (porA5' and porA3' containing a transcription terminator) necessary for homologous recombination, a selectable marker conferring resistance to kanamycin, two uptake sequences, a porA/lacO chimeric promoter repressed in the *E.coli* host expressing *lacl^{q}* but transcriptionally active in *Neisseria meningitidis,* and a multiple cloning site (5 sites present: *Nde*I, *Kpn*I, *Nhe*I, *PinA*1 and *Sph*I) necessary for the insertion of foreign DNA in pCMK.

pCMK was constructed as follows. The *porA*5' and *porA*3' recombinogenic regions, the *porA*/*lacO* promoter were PCR amplified using the oligonucleotides listed in the table below, cloned in pTOPO and sequenced. These DNA fragments were successively excised from pTOPO and recloned in pSL1180. The kanamycin resistance cassette was excised from pUC4K (PharmaciaBiotech, Sweeden) and was introduced between the porA5' flanking region and the *porA*/*lacO* promoter region.

**Table: Oligonucleotides used in this work**

| **Oligonucleotides** | **Sequence** | **Remark(s)** |
|---|---|---|
| PorA5' Fwd | | *Hind*III **cloning site** Uptake sequence (_) |
| PorA5'Rev | | *Nru* I **cloning site** |
| PorA3' Fwd | | *Bgl* II **cloning site** Stop codons (_) |
| PorA3'Rev | | *Eco*RI **cloning site** Uptake sequence (_) |
| PoLa Rev1 | | |
| PoLa Rev2 | | *Nde*I **cloning site** |
| PorAlacO Fwd | | *Pst*I **cloning site** |
| PorAlacO Rev | 5'-CTT AAG GCA TAT GGG CTT CCT TTT GTA A-3' | *Nde*I **cloning site** |
| PPA1 | 5'- GCG GCC GTT GCC GAT GTC AGC C-3' | |
| PPA2 | 5'-GGC ATA GCT GAT GCG TGG AAC TGC-3' | |
| N-full-01: | | *Nde*I **cloning site** |
| Nde-NspA-3: | | *Nde*I **cloning site** |
| PNS1 | | *Eco*RI **cloning site** |
| PNS1 | 5'- CGT CTA GAC GTA GCG GTA TCC GGC TGC -3' | XbaI **cloning site** |
| PromD15-51 X | | *Eco*RI and *Not*I **cloning sites** |
| PromD15-S2 | | *Xba*I **cloning site** |
| PNS4 | | *Swa*I and *PacI* cloning sites |
| PNS5 | | *PmeI* **cloning site** |
| D15-S4 | | *SwaI* and *PacI* **cloning sites** |
| D15-S5 | | *PmeI* **cloning site** |

### Example 3: Construction of a Neisseiria meningitidis serogroup B strain lacking both capsular polysaccharides and the major immunodominant antigen PorA.

Modulating the antigenic content of outer membrane blebs may be advantageous in improving their safety and efficacy in their use in vaccines, or diagnostic or therapeutic uses. Components such as the *Neisseiria meningitidis* serogroup B capsular polysaccharides should be removed to exclude the risk of inducing autoimmunity (see example 1). Similarly, it is beneficial to suppress the immunodominance of major outer-membrane antigens such as PorA, which induce strain-specific bactericidal antibodies but fail to confer cross-protection. To illustrate such an approach, we used the pCMK(+) vector to construct a *Neisseiria meningitidis* serogroup B strain lacking both capsular polysaccharides and the immunodominant PorA outer membrane protein antigen. For this purpose, a deletion of the *porA* gene was introduced in the H44/76 *cps-* strain, described in example 1 by means of homologous recombination.

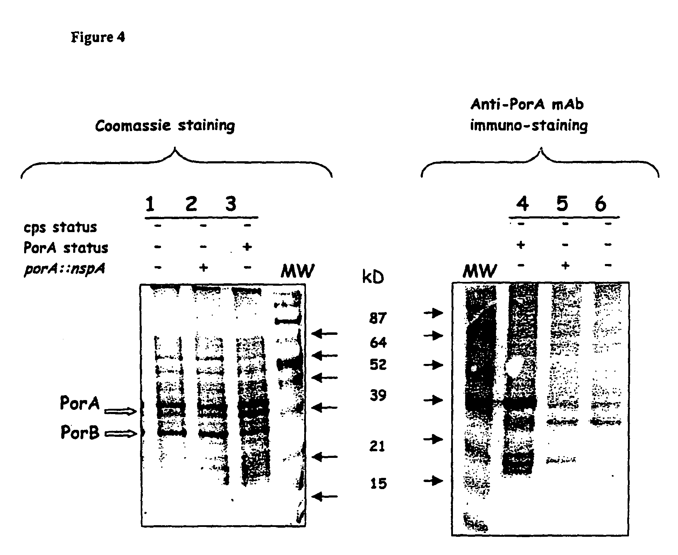

The H44/76 *cps-* strain was prepared competent and transformed with two 2µg of supercoiled pCMK(+) plasmid DNA as described previously. Aliquot fractions of the transformation mixture (100µl) were plated on Mueller-Hinton plates supplemented with Kanamycin (200µg/ml) and incubated at 37°C for 24 to 48 hours. Kanamycin-resistant colonies were selected, restreaked on MH-Kn and grown for an additional 24 hours at 37°C. At that stage half of the bacterial culture was used to prepare glycerol stocks (15 % vol./vol.) and was kept frozen at -70°C. Another fraction (estimated to be 10⁸ bacteria) was resuspended in 15 µl of distilled water, boiled ten minutes and used as a template for PCR screening. Two *porA* internal primers named , PPA1 and PPA2, were synthesized and used to perform PCR amplification on boiled bacterial lysates in the conditions described by the supplier (HiFi DNA polymerase, Boehringer Mannheim, GmbH). The thermal cycling used was the following: 25 times (94°C 1min., 52°C 1min., 72°C 3min.) and 1 time (72°C 10min., 4°C up to recovery). Since a double crossing-over between pCMK DNA and the chromosomal *porA* locus deletes the region required for #1 and #2 annealing, clones lacking a 1170bp PCR amplification fragment were selected as *porA* deletion mutants. These PCR results were further confirmed by analyzing in parallel, the presence of PorA in the corresponding bacterial protein extracts. For that purpose, another aliquot of bacteria (estimated to be 5.10⁸ bacteria) was re-suspended in 50 µl of PAGE-SDS buffer (SDS 5%, Glycerol 30%, Beta-mercaptoethanol 15%, Bromophenol blue 0.3mg/ml, Tris-HCl 250 mM pH6.8), boiled (100°C)frozen(-20°C) / boiled (100°C) three times and was separated by PAGE-SDS electrophoresis on a 12.5 % gel. Gels were then stained by Coomassie Brilliant blue R250 or transferred to a nitrocellulose membrane and probed with an anti-PorA monoclonal antibody as described in Maniatis *et al.* As represented in Figure 4, both Coomassie and immunoblot staining confirmed that *porA* PCR negative clones do not produce detectable levels of PorA. This result confirm that the pCMK vector is functional and can be used successfully to target DNA insertion in the *porA* gene, abolishing concomitantly the production of the PorA outer membrane protein antigen.

### Example 4: Up-regulation of the NspA outer membrane protein production in blebs derived from a recombinant Neisseiria meningitidis serogroup B strain lacking functional poor A and cps genes.

Enriching bleb vesicles with protective antigens is advantageous for improving the efficiency and the coverage of outer membrane protein-based vaccines. In that context, recombinant *Neisseria meningitidis* strains lacking functional *cps* and *porA* genes were engineered so that the expressions level of the outer-membrane protein NspA was up-regulated. For that purpose, the gene coding for NspA was PCR amplified using the N01-full-*Nde*I and *Nde*I-3'oligonucleotide primers (see table in example 2). The conditions used for PCR amplification were those described by the supplier (HiFi DNA polymerase, Boehringer Mannheim, GmbH). Thermal cycling done was the following: 25 times (94°C 1min., 52°C 1min., 72°C 3min.) and 1 time (72°C 10min., 4°C up to recovery). The corresponding amplicon was digested with NdeI and inserted in the *Nde*I restriction site of the pCMK(+) delivery vector. Insert orientation was checked and recombinant plasmids, designed pCMK(+)-NspA, were purified at a large scale using the QIAGEN maxiprep kit and 2 µg of this material was used to transform a *Neisseiria meningitidis* serogroup B strain lacking functional *cps* genes (strain described in example 1). Integration resulting from a double crossing-over between the pCMK(+)-NspA vector and the chromosomal *porA* locus were selected using a combination of PCR and Western blot screening procedures presented in example 3.

Bacteria (corresponding to about 5.10⁸ bacteria) were re-suspended in 50 µl of PAGE-SDS buffer, frozen(-20°C) / boiled (100°C) three times and then were separated by PAGE-SDS electrophoresis on a 12.5 % gel. Gels were then stained by Coomassie Brilliant blue R250 or transferred to a nitrocellulose membrane and probed with an anti-NspA polyclonal serum. Both Coomassie (data not shown) and immunoblot staining (see figure 4) confirmed that *porA* PCR negative clones do not produce detectable levels of PorA. The expression of NspA was examined in Whole-cell bacterial lysates (WCBL) or outer-membrane bleb preparations derived from NmB [*cps*-, *porA*-] or NmB [*cps*-, *porA*-, *Nspa*+]. Although no difference was observable by Coomassie staining, immunoblotting with the anti-NspA polyclonal serum detected a 3-5 fold increased in the expression of NspA (with respect to the endogenous NspA level), both in WCBL and outer-membrane bleb preparations (see figure 5). This result confirm that the pCMK(+)-NspA vector is functional and can be used successfully to up-regulate the expression of outer membrane proteins such as NspA, abolishing concomitantly the production of the PorA outer membrane protein antigen.

### Example 5: Up-regulation of the D15/Omp85 outer membrane protein antigen in blebs derived from a recombinant Neisseiria meningitidis serogroup B strain lacking functional cps genes but expressing PorA.

Certain geographically isolated human populations (such as Cuba) are infected by a limited number of *Neisseiria meningitidis* isolates belonging largely to one or few outer membrane protein serotypes. Since PorA is a major outer-membrane protein antigen inducing protective and strain-specific bactericidal antibodies, it is then possible to confer vaccine protection using a limited number of porA serotypes in a vaccine. In such a context, the presence of PorA in outer membrane vesicles may be advantageous, strengthening the vaccine efficacy of such recombinant improved blebs. Such PorA containing vaccines, however, can be improved still further by increasing the level of other cross-reactive OMPs such as omp85/D15.

In the following example, the pCMK(+) vector was used to up-regulate the expression of the Omp85/D15 outer membrane protein antigen in a strain lacking functional *cps* genes but expressing *porA*. For that purpose, the gene coding for Omp85/D15 was PCR amplified using the D15-*Nde*I and D15-*Not*I oligonucleotide primers. The conditions used for PCR amplification were those described by the supplier (HiFi DNA polymerase, Boehringer Mannheim, GmbH). Thermal cycling done was the following: 25 times (94°C 1min., 52°C 1min., 72°C 3min.) and 1 time (72°C 10min., 4°C up to recovery). The corresponding amplicon was inserted in the pTOPO cloning vector according to the manufacturer's specifications and confirmatory sequencing was performed. This Omp85/D15 DNA fragment was excised from pTOPO by restriction hydrolysis using *Nde*I/*Nsi*I and subsequently cloned in the corresponding restriction sites of the pCMK(+) delivery vector. Recombinant plasmids, designed pCMK(+)-D15 were purified on a large scale using the QIAGEN maxiprep kit and 2 µg of this material was used to transform a *Neisseiria meningitidis* serogroup B strain lacking functional *cps* genes (strain described in example 1). In order to preserve the expression of *porA*, integration resulting from a single crossing-over (either in Omp85/D15 or in porA) were selected by a combination of PCR and Western blot screening procedures. Kanamycin resistant clones testing positive by *porA*-specific PCR and western blot were stored at -70°C as glycerol stocks and used for further studies.

Bacteria (corresponding to about 5.10⁸ bacteria) were re-suspended in 50 µl of PAGE-SDS buffer, frozen(-20°C) / boiled (100°C) three times and then were separated by PAGE-SDS electrophoresis on a 12.5 % gel. Gels were then stained by Coomassie Brilliant blue R250 or transferred to a nitrocellulose membrane and probed with an anti-porA monoclonal antibody. As represented in Figure 6, both Coomassie and immunoblot staining confirmed that *porA* PCR positive clones produce PorA.

The expression of D 15 was examined using outer-membrane bleb preparations derived from NmB [cps-, porA-] or NmB [cps-, porA+, D15+]. Coomassie detected a significant increase in the expression of D15 (with respect to the endogenous D15 level), preparations (see Figure 6). This result confirmed that the pCMK(+)-D15 vector is functional and can be used successfully to up-regulate the expression of outer membrane proteins such as D15, without abolishing the production of the major PorA outer membrane protein antigen.

### Example 6: Construction of versatile promoter delivery vectors

Rational: The rational of this approach is represented in Figure 7 and can be summarized in 7 essential steps. Some of these steps are illustrated below with the construction of Vector for up-regulating the expression of NspA and D15/Omp85.

### Vector for up-regulating the expression of the NspA gene.

**Step 1**. A DNA region (997bp) located upstream from the NspA coding gene was discovered (SEQ. ID NO:2) in the private Incyte PathoSeq data base containing unfinished genomic DNA sequences of the *Neisseria meningitidis* strain ATCC 13090. Two oligonucleotide primers referred to as PNS1 and PNS2 (see table in example 2) were designed using this sequence and synthesized. These primers were used for PCR amplification using genomic DNA extracted from the H44/76 strain. **Step 2**. The corresponding amplicons were cleaned-up using the Wizard PCR kit (Promega, USA) and submitted to digestion with the *Eco*RI/*Xba*I restriction enzymes for 24 hours using the conditions described by the supplier (Boehringer Mannheim, Germany). The corresponding DNA fragments were gel purified and inserted in the corresponding sites of the pUC18 cloning vector. **Step 3**. Recombinant plasmids were prepared on a large scale and an aliquot fraction was used as a template for inverse PCR amplification. Inverse PCR was performed using the PNS4 and PNS5 oligonucleotides using the following thermal cycling conditions: 25 times (94°C 1min., 50°C 1min., 72°C 3min.) and 1 time (72°C 10min., 4°C up to recovery). Linearized pUC 18 vectors harbouring a deletion in the NspA upstream region insert were obtained.

### Vector for up-regulating the expression of the D15/omp85 gene.

**Step 1**. A DNA region (1000 bp) located upstream from the D15/omp85 coding gene was discovered (SEQ. ID NO:3) in the private Incyte PathoSeq database containing unfinished genomic DNA sequences of the *Neisseria meningitidis* strain ATCC 13090. Two oligonucleotide primers refererred to as PromD15-51X and PromD15-S2 (see table in example 2) were designed using this sequence and synthesized. These primers were used for PCR amplification using genomic DNA extracted from the H44/76 strain. **Step 2**. The corresponding amplicons were cleaned-up using the Wizard PCR kit (Promega, USA) and submitted to digestion with the *Eco*RI/*Xba*I restriction enzymes for 24 hours in the conditions described by the supplier (Boehringer Mannheim, Germany). The corresponding DNA fragments were gel purified and inserted in the corresponding sites of the pUC18 cloning vector. **Step 3**. Recombinant plasmids were prepared on a large scale and an aliquot fraction was used as a template for inverse PCR amplification. Inverse PCR was performed using the D15-S4 and D15-S5 oligonucleotides using the following thermal cycling conditions: 25 times (94°C 1min., 50°C 1min., 72°C 3min.) and 1 time (72°C 10min., 4°C up to recovery). Linearized pUC 18 vectors harbouring a deletion in the D15/omp85 upstream region insert were obtained.

### Example 7: Fermentation processes for producing recombinant blebs.

The examples listed below describe methods for producing recombinant blebs lacking either capsular polysaccharides or capsular polysaccharides and PorA. Such a procedure may be used for a wide range of *Neisseiria meningitidis* recombinant strains and may be adapted over an extended scale range.

Culture *media:Neisseiria meningitidis* serogroup B strains were propagated in solid (FNE 004 AA, FNE 010 AA) or liquid (FNE 008 AA) culture media. These new media for growing meningococcus are advantageiously free of animal products, and are considered a further aspect of the invention.

| **Components** | **FNE 004 AA** | **FNE 008 AA** | **FNE 010 AA** |
|---|---|---|---|
| Agar | 18 g/L | - | 18 g/L |
| NaCl | 6 g/L | 6 g/L | 6 g/L |
| Na-Glutamate | - | 1.52 g/L | - |
| NaH₂PO₄.2H₂O | 2.2 g/L | 2.2 g/L | 2.2 g/L |
| KCl | 0.09 g/L | 0.09 g/L | 0.09 g/L |
| NH₄Cl | 1.25 g/L | 1.25 g/L | 1.25 g/L |
| Glucose | 5g/L | 20 g/L | 5 g/L. |
| Yeast Extract UF | - | 2.5 g/L | - |
| Soy Pepton | 5 g/L | 30 g/L | 5 g/L |
| CaCl₂.2H₂O | 0.015 g/L | - | 0.015 g/L |
| MgSO₄.7H₂O | 0.6 g/L | 0.6 g/L | 0.6 g/L |
| Erythromycine : | 0.015 g/L | - | - |
| Kanamycine | - | - | 0.2 g/L |

Flask cultivation of *Neisseiria meningitidis* serogroup B cps- recombinant blebs: This was performed in two steps comprising preculture on solid medium followed by liquid cultivation. **Solid pre-culture** A vial of seed was removed from freezer (-80°C), thawed to room temperature and 0.1 mL was streaked into a Petri dish containing 15 mL of FNE004AA (see above).The Petri dish was incubated at 37°C for 18 ± 2 hours. The surface growth was resuspended in 8 mL of FNE008AA (see above) supplemented with 15 mg/L of erythromycin. **Flask culture.** 2 mL of resuspended solid pre-culture were added to a 2 litre flask containing 400 mL of FNE008AA supplemented with 15 mg/L of erythromycin. The flask was placed on a shaking table (200 rpm) and incubated at 37°C for 16 ± 2 hours. The cells were separated from the culture broth by centrifugation at 5000g at 4°C for 15 minutes.

Batch mode cultivation of *Neisseiria meningitidis* serogroup B *cps*- recombinant blebs: This was performed in three steps comprising preculture on solid medium, liquid cultivation and batch mode cultivation. **Solid pre-culture**._A vial of seed was removed from freezer (-80°C), thawed to room temperature and 0.1 mL was streaked into a Petri dish containing 15 mL of FNE004AA (see above). The Petri dish was incubated at 37°C for 18 ± 2 hours. The surface growth was resuspended in 8 mL of FNE008AA (see above) supplemented with 15 mg/L of erythromycin. **Liquid pre-culture**._2 mL of resuspended solid pre-culture were added to one 2 liters flask containing 400 mL of FNE008AA supplemented with 15 mg/L of erythromycin. The flask was placed on a shaking table (200 rpm) and incubated at 37°C for 16 ± 2 hours. The content of the flask was used to inoculate the 20 liters fermenter. **Batch mode culture in fermenter**. The inoculum (400 mL) was added to a pre-sterilized 20 liters (total volume) fermenter containing 10 L of FNE008AA supplemented with 15 mg/L of erythromycin. The pH was adjusted to and maintained at 7.0 by the automated addition of NaOH (25% w/v) and H₃PO₄ (25% v/v). The temperature was regulated at 37°C. The aeration rate was maintained at 20 L of air / min and the dissolved oxygen concentration was maintained at 20% of saturation by the agitation speed control. The overpressure in the fermenter was maintained at 300 g/cm². After 9 ± 1 hours, the culture was in stationary phase. The cells were separated from the culture broth by centrifugation at 5000g at 4°C for 15 minutes.

Flask cultivation of *Neisseiria meningitidis* serogroup B *cps-,* PorA- recombinant blebs: This was performed in two steps comprising preculture on solid medium followed by liquid cultivation._**Solid pre-culture**. A vial of seed was removed from freezer (-80°C), thawed to room temperature and 0.1 mL was streaked into a Petri dish containing 15 mL of FNE010AA (see above). The Petri dish was incubated at 37°C for 18 ± 2 hours. The surface growth was resuspended in 8 mL of FNE008AA (see above) supplemented with 200 mg/L of kanamycin. **Flask culture**. 2 mL of resuspended solid pre-culture were added to a 2 litre flask containing 400 mL of FNE008AA supplemented with 200 mg/L of kanamycin. The flask was placed on a shaking table (200 rpm) and incubated at 37°C for 16 ± 2 hours. The cells were separated from the culture broth by centrifugation at 5000g at 4°C for 15 minutes.

### Example 8: Isolation and purification of blebs from meningococci devoid of capsular polysaccharide

Recombinant blebs were purified as described below. The cell paste (42gr) was suspended in 211 ml of 0.1M Tris-Cl buffer pH 8.6 containing 10 mM EDTA and 0.5% Sodium Deoxycholate (DOC). The ratio of buffer to biomass was 5/1 (V/W). The biomass was extracted by magnetic stirring for 30 minutes at room temperature. Total extract was then centrifuged at 20,000g for 30 minutes at 4°C (13,000 rpm in a JA-20 rotor, Beckman J2-HS centrifuge). The pellet was discarded. The supernatant was ultracentrifuged at 125,000g for 2 hours at 4°C (40,000 rpm in a 50.2Ti rotor, Beckman L8-70M ultracentrifuge) in order to concentrate vesicles. The supernatant was discarded. The pellet was gently suspended in 25 ml of 50 mM Tris-Cl buffer pH 8.6 containing 2 mM EDTA, 1.2% DOC and 20% sucrose. After a second ultracentrifugation step at 125,000g for 2 hours at 4°C, vesicles were gently suspended in 44 ml of 3% sucrose and stored at 4°C. All solutions used for bleb extraction and purification contained 0.01% thiomersalate. As illustrated in Figure 8, this procedure yields protein preparations highly enriched in outer-membrane proteins such as PorA and PorB.

### Example 9: Identification of bacterial promoters suitable for up-regulation antigens-coding genes

The use of strong bacterial promoter elements is essential to obtain up-regulation of genes coding for outer membrane proteins. In that context, we have shown previously that up-regulating the *Neisseria meningitidis nspA, hsf,* and *omp85* genes using the *porA* promoter has allowed us to isolate recombinant blebs enriched in the corresponding NspA, Hsf and Omp85 proteins. Alternatives to the *porA* promoter may be useful to obtain different levels of up-regulation, to overcome potential *porA* phase variation and/or to achieve conditional gene expression (iron-regulated promoters). Here we describe a method allowing the identification of a precise transcriptional start site of strong promoter elements likely to confer high level of expression in bacteria. Since promoter regulatory elements are classically encompassed within 200 bp upstream and 50bp dowtream from the +1 site (Collado-Vides J, Magasanik B, Gralla JD, 1991, Microbiol Rev 55(3):371-94), the result of such an experiment allows us to identify DNA fragments of about 250 bp carrying strong promoter activities. Major outer membrane proteins such as *Neisseria meningitidis* PorA, PorB & Rmp, *Haemophilus influenzae* P1, P2, P5 & P6, *Moraxella catarrhalis* OmpCD, OmpE, as well as some cyoplasmic and/or iron regulated proteins of these bacteria possess strong promoter elements. As a validation of this general methodology, we mapped the transcriptional start site of the strong *Neisseria meningitidis porA* and *porB* promoters using rapid amplification of cDNA elements (5' RACE).

The principles of 5' RACE are the following: 1) Total RNA extraction using QIAGEN "RNeasy" Kit. Genomic DNA removing by DNase treatment followed by QIAGEN purification; 2) mRNA reverse transcription with a *porA* specific 3' end primer (named porA3). Expected cDNA size: 307 nt. RNA removing by alkaline hydrolysis; 3) Ligation of a single-stranded DNA oligo anchor (named DT88) to the 3' end of the cDNA using T4 RNA ligase. Expected product size: 335 nt. Amplification of the anchor-ligated cDNA using a combination of hemi-nested PCR; 4) PCR amplification of the anchor-ligated cDNA using a complementary-sequence anchor primer as the 5' end primer (named DT89) and a 3'end primer (named p1-2) which is internal to the 3'end RT primer porA3. Expected product size: 292 bp; 5) PCR amplification of previous PCR products using DT89 as 5'end primer and p1-1 as 3'end primer (internal to p1-2). Expected product size: 211bp; and 6) Sequencing with p1-1 primer (expected products size can be calculated because *porA* transcription start site is known: 59 nt before the "ATG" translation start site).

### Experimental procedure

Total RNA was extracted from approximately 10⁹ cells of *Neisseria meningitidis* serogroup B *cps- porA*+ strain. Extraction of 1 ml of a liquid culture at appropriate optical density (OD₆₀₀ = 1) was performed by the QIAGEN "RNAeasy" kit according to the manufacturer's instructions. Chromosomal DNA was removed by addition of 10U of RNase-free DNase (Roche Diagnostics, Mannheim, Germany) to the 30 µl of eluted RNA and was incubated at 37°C for 15 min. The DNA-free RNA was purified with the same QIAGEN kit according to instructions.

Reverse transcription reactions were performed using primer porA3 and 200U of SUPERSCRIPT II reverse transcriptase (Life Technologies). The RT reactions were performed in a 50µl volume containing: 5µl of 2mM dNTP, 20 pmol of porA3 pimer, 5µl of 10X SUPERSCRIPT II buffer, 9µl of 25mM MgCl2, 4µl of 0.1M DTT, 40U of recombinant ribonuclease inhibitor and 1 µg of total RNA. The porA3 primer was annealed stepwise (70°C for 2 min, 65°C for 1 min, 60°C for 1 min, 55°C for 1 min, 50°C for 1 min, and 45°C for 1 min) before the SUPERSCRIPT II was added. The RT reaction was performed at 42°C for 30 min, followed by 5 cycles (50°C for 1 min, 53°C for 1 min and 56°C for 1 min) to destabilize RNA secondary structure. Two parallel reactions were performed with the reverse transcriptase omitted from one reaction as negative control.

The RNA was removed by alkaline hydrolysis cleavage with the addition of 1 µl of 0.5M EDTA followed by 12.5 µl of 0.2 M NaOH before incubation at 68°C for 5 min. The reactions were neutralized by adding 12.5 µl of 1 M Tris-HCl (pH7.4) and precipitated by the addition of 20 µg of glycogen (Roche Molecular Biochemicals, Mannheim, Germany), 5 µl of 3 M sodium acetate and 60 µl of isopropanol. Both samples were resuspended in 20 µl of 10:1 TE (10 mM Tris-HCl, pH 7.4; 1 mM EDTA, pH8).

T4 RNA ligase was used to anchor a 5'-phosphorylated, 3'end ddCTP-blocked anchor oligonucleotide DT88 (see table below). Two parallel ligations were performed overnight at room temperature with each containing: 1.3 µl of 10X RNA ligase buffer (Roche Molecular Biochemicals), 0.4 µM DT88, 10 µl of either cDNA or RT control sample and 3 U of T4 RNA ligase. As negative controls, a second set of ligations reactions was performed, omitting the T4 RNA ligase. The resulting ligation-reaction mixtures were used directly without purification in the subsequent PCR.

The anchor-ligated cDNA was amplified using a combination of hemi-nested and hot-started PCR approaches to increase specificity and product yield. Four separate first-round PCR were performed on the RT/ligase reaction and controls in a 30 µl volume, each containing: 3 µl of 10X Taq Platinium buffer, 3µl of 25 mM MgCl₂, 1 µl of 10mM dNTP, 10 pmol of each primers and 1 µl of corresponding RNA ligation reaction. The PCR were hot started by the use of Taq Platinium (Life Technologies) DNA polymerase (2U added). The first ligation-anchored PCR (LA-PCR) was performed using 10 pmol of both the anchor-specific primer DT89 and the transcript-specific primer pl-2 (see table below) which is internal to the 3' end RT primer porA3. The PCR was performed using an initial 95°C for a 5 min step (for DNA polymerase activation) followed by 10 cycles at 95°C for 10 s and 70°C for 1 min (reducing one degree per cycle), 15 cycles at 95°C for 10 s and 60°C for 1 min. The second hemi-nested LA-PCR was performed under the same conditions using primer DT89 and the pl-2 internal primer, together with 10 pmol of pl-1 (see table below) and 1 µl of first-round PCR. Amplification products were purified using the QIAGEN "QIAquick PCR purification" kit according to manufacturer instructions before submitted to sequencing.

The CEQ™ Dye Terminator Cycle Sequencing kit (Beckman, France) was used to sequence the RACE PCR products using 10 pmol of primer p1-1. Sequencing reactions were performed according to the provided instructions and sequencing products were analyzed by the Ceq2000 DNA Analysis System (Beckman-Coulter).

| | |
|---|---|
| DT88 | 5'GAAGAGAAGGTGGAAATGGCGTTTTGGC3' |
| DT89 | 5' CCAAAACGCCATTTCCACCTTCTCTTC 3' |
| porA3 | 5' CCAAATCCTCGCTCCCCTTAAAGCC 3' |
| p1-2 | 5' CGCTGATTTTCGTCCTGATGCGGC 3' |
| p1-1 | 5' GGTCAATTGCGCCTGGATGTTCCTG 3' |

### Results for the Neisseria meningitidis porA promoter

The start of transcription for *Neisseria meningitidis* serogroup B (strain H44/76) *porA*-mRNA was mapped 59 bp upstream of the ATG start codon using the described 5'-RACE procedure. This result confirms the mapping performed by primer extension and published by van der Ende *et al* (1995). This result supports that a DNA fragment containing nucleotides -9 to -259 with regard to the *porA* ATG is suitable for driving strong gene expression in *Neisseria meningitidis* and possibly in other bacterial species such as *Haemophilus, Moraxella, Pseudomonas.*

### Results for the Neisseria meningitidis porB promoter

The same experimental strategy has been applied for *Neisseria meningitidis* serogroup B (strain H44/76) *porB* transcription start site mapping. Primers listed in the table below correspond to 3' end RT primer (porB3), transcript-specific primer that is internal to the porB3 (porB2) and internal to the porB2 (porB1). porB3, porB2 and porB1 are respectively located 265 bp, 195 bp and 150 bp downstream the ATG start codon.

| | |
|---|---|
| porB1 | 5' GGTAGCGGTTGTAACTTCAGTAACTT 3' |
| porB2 | 5' GTCTTCTTGGCCTTTGAAGCCGATT 3' |
| porB3 | 5' GGAGTCAGTACCGGCGATAGATGCT 3' |

Using porB1 and DT89 primers a ~200 bp PCR amplicon was obtained by performing 5' - RACE mapping. Since porB1 is located 150 bp from the *porB* ATG start codon, this result supports that the *porB* transcriptional start site is located about 50 bp (+/-30 bp) upstream of the porB ATG.

The exact nucleotide corresponding to transcription initiation is presently being determined by DNA sequencing. The above PCR result supports that a DNA fragment containing nucleotides -1 to -250 with regard to the *porB* ATG start codon is suitable for driving strong gene expression in *Neisseria meningitidis* and possibly in other bacterial species such as *Haemophilus, Moraxella, Pseudomonas.*

### Example 10: Up-regulation of the N. meningitidis serogroup B Omp85 gene by promoter replacement.

The aim of the experiment was to replace the endogenous promoter region of the D15/Omp85 gene by the strong *porA* promoter in order to up-regulate the production of the D15/Omp85 antigen. For that purpose, a promoter replacement plasmid was constructed using *E. coli* cloning methodologies. A DNA region (1000 bp) located upstream from the *D15*/*omp85* coding gene was discovered (SEQ ID NO:3) in the private Incyte PathoSeq data base containing unfinished genomic DNA sequences of the *Neisseria meningitidis* strain ATCC 13090. The main steps of this procedure are represented in Figure 9. Briefly, a DNA fragment (1000bp) covering nucleotides -48 to -983 with respect to the *D15*/*Omp85* gene start codon (ATG) was PCR amplified using oligonucleotides **ProD15-51X** (5'-GGG CGA ATT CGC GGC CGC CGT CAA CGG CAC ACC GTT G-3') and **ProD15-52** (5'-GCT CTA GAG CGG AAT GCG GTT TCA GAC G-3') containing *EcoRI* and *XbaI* restriction sites (underlined) respectively. This fragment was submitted to restriction and inserted in pUC18 plasmid restricted with the same enzymes. The construct obtained was submitted to *in vitro* mutagenesis using the Genome Priming system (using the pGPS2 donor plasmid) commercialized by New England Biolabs (MA, USA). Clones having inserted a mini-transposon (derived from Tn7 and harboring a chloramphenicol resistance gene) were selected. One clone containing a mini-transposon insertion located in the *D15*/*Omp85* 5' flanking region, 401 bp downstream from the *EcoRI* site was isolated and used for further studies. This plasmid was submitted to circle PCR mutagenesis (Jones & Winistofer (1992), Biotechniques12: 528-534) in order to (i) delete a repeated DNA sequence (Tn7R) generated by the transposition process, (ii) insert meningococcal uptake sequences required for transformation, and (iii) insert suitable restriction sites allowing cloning of foreign DNA material such as promoters. The circle PCR was performed using the **TnRD15-KpnI/XbaI** + US (5'-CGC CGG TAC CTC TAG AGC CGT CTG AAC CAC TCG TGG ACA ACC C-3') & **TnR03Cam(KpnI)** (5'-CGC CGG TAC CGC CGC TAA CTA TAA CGG TC-3') oligonucleotides containing uptake sequences and suitable restriction sites (*Kpn*I and *Xba*I) underlined. The resulting PCR fragment was gel-purified, digested with *Asp718* (isoschizomer of *Kpn*I) and ligated to a 184bp DNA fragment containing the *porA* promoter and generated by PCR using the **PorA-01** (5'-CGC CGG TAC CGA GGT CTG CGC TTG AAT TGT G-3') and **PorA02** (5'-CGC CGG TAC CTC TAG ACA TCG GGC AAA CAC CCG-3') oligonucleotides containing *Kpn*I restriction sites. Recombinant clones carrying a *porA* promoter inserted in the correct orientation (transcription proceeding in the *EcoRI* to *XbaI* direction) were selected and used to transform a strain of *Neisseria meningitidis* serogroup B lacking capsular polysaccharide (*cps*-) and one of the major outer membrane proteins - PorA (*porA*-). Recombinant *Neisseria meningitidis* clones resulting from a double crossing over event (PCR screening using oligonucleotides **Cam-05** (5'-GTA CTG CGA TGA GTG GCA GG-3') & **proD15-52**) were selected on GC medium containing 5µg/ml chloramphenicol and analyzed for D15/Omp85 expression. As represented in Figure 10, the production of D15/Omp85 was significantly increased in the total protein extracts of *Nm* strains resulting from promoter replacement, when compared to parental strain (*cps*-). This result was also observed when analyzing outer-membrane blebs prepared from the same strains (see Figure 17). These results are attributable to the replacement of the endogenous *D15* promoter by the strong *porA* promoter. In addition, it was surprisingly found that expression, where the porA promoter was introduced approximately 400 bp upstream of the initiator codon, was approximately 50 times greater than when the promoter was placed approximately 100 bp upstream. Altogether, these experiments support that the promoter replacement strategy works and allows the up-regulation of the synthesis of integral outer-membrane proteins in outer-membrane blebs.

Certain geographically isolated human populations (such as Cuba) are infected by a limited number of *Neisseiria meningitidis* isolates belonging largely to one or few outer membrane protein serotypes. Since PorA is a major outer-membrane protein antigen which can induce protective and strain-specific bactericidal antibodies, it may be possible to confer vaccine protection in such a population using a limited number of porA serotypes. Moreover, PorA may interact with or stabilize some other outer membrane proteins. In this context, the presence of PorA in outer membrane vesicles may be advantageous, strengthening the vaccine efficacy of such recombinant · improved blebs.

For such a reason, it may be desirable to up-regulate the expression of D15/Omp85 outer membrane protein in a *Neisseria meningitidis* serogroup B strain lacking functional *cps* genes but expressing PorA. Genomic DNA was extracted from the recombinant *Neisseria meningitidis* serogroup B *cps*-, *porA*-, D15/Omp85+ strain using the QIAGEN Genomic Tips 100-G kit. 10µgr of this material was linearized and used to transform *Neisseria meningitidis* serogroup B *cps-* following a classical transformation protocol. Recombinant *Neisseria* were obtained on GC agar plates containing 5µgr/ml chloramphenicol.

Integrations resulting from a double crossing-over upstream of the *D15* gene were screened by PCR as described previously. As homologous recombinations can occur everywhere in the chromosome, a second PCR screening was performed to control the integrity of the *porA* locus in the recombinant strain. For this purpose, internal *porA* primers **PPA1** (5- GCG GCC GTT GCC GAT GTC AGC C -3') and **PpA2** (5- GGC ATA GCT GAT GCG TGG AAC TGC -3') were used in a PCR screening experiment. The amplification of an 1170bp fragment confirms the presence of the *porA* gene in the recombinant bacteria.

Recombinant bacteria (corresponding to about 5.10⁸ bacteria) can be re-suspended in 50 µl of PAGE-SDS buffer, frozen(-20°C) / boiled (100°C) three times and then separated by PAGE-SDS electrophoresis on a 12.5 % gel. Gels can then be stained by Coomassie Brilliant blue R250 or transferred to a nitrocellulose membrane and probed either with an anti-porA monoclonal antibody or with an anti-D15/Omp85 rabbit polyclonal antibody. Analysis of outer-membrane blebs prepared from the same strains can also be performed.

### Example 11: Up-regulation of the Hsf protein antigen in a recombinant Neisseiria meningitidis serogroup B strain lacking functional cps genes but expressing PorA.

As described above, in certain countries, the presence of PorA in outer membrane vesicles may be advantageous, and can strengthen the vaccine efficacy of recombinant improved blebs. In the following example, we have used a modified pCMK(+) vector to up-regulate the expression of the Hsf protein antigen in a strain lacking functional *cps* genes but expressing PorA. The original pCMK(+) vector contains a chimeric *porA*/*lacO* promoter repressed in *E. coli* host expressing *lacl^{q}* but transcriptionally active in *Neisseria meningitidis.* In the modified pCMK(+), the native *porA* promoter was used to drive the transcription of the *hsf* gene. The gene coding for Hsf was PCR amplified using the HSF 01-*Nde*I and HSF 02-*Nhe*I oligonucleotide primers, presented in the table below. Because of the sequence of the HSF 01-*Nde*I primer the Hsf protein expressed will contain two methionine residues at the 5' end. The conditions used for PCR amplification were those described by the supplier (HiFi DNA polymerase, Boehringer Mannheim, GmbH). Thermal cycling was the following: 25 times (94°C 1min., 48°C 1min., 72°C 3min.) and 1 time (72°C 10min., 4°C up to recovery). The corresponding amplicon was subsequently cloned in the corresponding restriction sites of pCMK(+) delivery vector. In this recombinant plasmid, designed pCMK(+)-Hsf, we deleted the *lacO* present in the chimeric *porA*/*lacO* promoter by a recombinant PCR strategy (See Figure 12). The pCMK(+)-Hsf plasmid was used as a template to PCR amplify 2 separate DNA fragments:
- fragment 1 contains the *porA* 5' recombinogenic region, the Kanamycin resistance gene and the *porA* promoter. Oligonucleotide primers used, RP1(*Sac*II) and RP2, are presented in the table below. RP1 primer is homologous to the sequence just upstream of the *lac* operator.
- fragment 2 contains the Shine-Dalgarno sequence from the *porA* gene, the *hsf* gene and the *porA 3'* recombinogenic region. Oligonucleotide primers used, RP3 and *RP4(ApaI),* are presented in the table below. RP3 primer is homologous to the sequence just downstream of the *lac* operator. The 3' end of fragment 1 and the 5'end of fragment 2 halve 48 bases overlapping. 500ng of each PCR (1 and 2) were used for a final PCR reaction using primers RP1 and RP4. The final amplicon obtained was subcloned in pSL1180 vector restricted with *Sac*II and *Apa*I. The modified plasmid pCMK(+)-Hsf was purified at a large scale using the QIAGEN maxiprep kit and 2 µg of this material was used to transform a *Neisseiria meningitidis* serogroup B strain lacking functional *cps* genes (the strain described in example 1). In order to preserve the expression of *porA*, integration resulting from a single crossing-over was selected by a combination of PCR and Western blot screening procedures. Kanamycin resistant clones testing positive by *porA*-specific PCR and western blot were stored at -70°C as glycerol stocks and used for further studies. Bacteria (corresponding to about 5.10⁸ bacteria) were re-suspended in 50 µl of PAGE-SDS buffer, frozen (-20°C) / boiled (100°C) three times and then were separated by PAGE-SDS electrophoresis on a 12.5 % gel. The expression of Hsf was examined in Whole-cell bacterial lysates (WCBL) derived from *NmB* [Cps-, PorA+] or *NmB* [Cps-, PorA+, Hsf+]. Coomassie staining detected a significant increase in the expression of Hsf (with respect to the endogenous Hsf level) (See in Figure 13). This result confirms that the modified pCMK(+)-Hsf vector is functional and can be used successfully to up-regulate the expression of outer membrane proteins, without abolishing the production of the major PorA outer membrane protein antigen.

### Oligonucleotides used in this work

| **Oligonucleotides** | **Sequence** | **Remark(s)** |
|---|---|---|
| Hsf01-Nde | | *NdeI* **cloning site** |
| Hsf02-Nhe | | *Nhe* I **cloning site** |
| GFP-mut-Asn | | *Asn*I **cloning site** Compatible with *NdeI* |
| GFP-Spe | | *Spe*I **cloning site** Compatible with *Nhe*I |
| RP1 (SacII) | | *Sac*II **cloning site** |
| RP2 | | |
| RP3 | | |
| RP4(ApaI) | | *Apa*I **cloning site** |

### Example 12: Expression of the Green Fluorescent Protein in a recombinant Neisseria meningitidis serogroup B strain lacking functional cps genes but expressing PorA.

In the following example, the pCMK vector was used to test the expression of a cytoplasmic heterologous protein in *Neisseria meningitidis.* The Green Fluorescent Protein was amplified from the pKen-Gfpmut2 plasmid with the primers GFP-Asn-mut2 and GFP-Spe (see table in Example 11). *Asn*I gives cohesive ends compatible with *Nde*I, *Spe*I gives cohesive ends compatible with *Nhe*I. The conditions used for PCR amplification were those described by the supplier (HiFi DNA polymerase, Boehringer Mannheim, GmbH). Thermal cycling was the following: 25 times (94°C 1min., 48°C 1min., 72°C 3min.) and 1 time (724°C 10min., 4°C up to recovery). The corresponding amplicon was subsequently cloned in the pCMK(+) delivery vector digested with *NdeI* and *Nhe*I restriction enzymes. In this recombinant plasmid, designed pCMK(+)-GFP, we deleted the *lacO* present in the chimeric *porA*/*lacO* promoter by a recombinant PCR strategy. The pCMK(+)-GFP plasmid was used as template to PCR amplify 2 separate DNA fragments:
- fragment 1 contained the *porA* 5' recombinogenic region, the Kanamycin resistance gene and the *porA* promoter. Oligonucleotide primers used, RP1(*Sac*II) and RP2 (see table in example 11). RP1 primer is homologous to the sequence just upstream of the *lac* operator.
- fragment 2 contains the PorA Shine-Dalgamo sequence, the *gfp* gene and the *porA* 3' recombinogenic region. Oligonucleotide primers used, RP3 and *RP4(ApaI),* are presented in the table in example 11. RP3 primer is homologous to the sequence just downstream of the *lac* operator.

The 3'end of fragment 1 and the 5'end of fragment 2 have 48 bases overlapping. 500ng of each PCR (1 and 2) were used for a final PCR reaction using primers RP1 and RP4. Twenty µg of this PCR fragment were used to transform a *Neisseiria meningitidis* serogroup B strain lacking functional *cps* genes.

Transformation with linear DNA is less efficient than with circular plasmid DNA but all the recombinants obtained performed a double crossing-over (confirmed by a combination of PCR and Western blot screening procedures). Kanamycin resistant clones were stored at -70°C as glycerol stocks and used for further studies. Bacteria (corresponding to about 5.10⁸ bacteria) were re-suspended in 50 µl of PAGE-SDS buffer, frozen (-20°C) / boiled (100°C) three times and then were separated by PAGE-SDS electrophoresis on a 12.5 % gel.

The expression of GFP was examined in Whole-cell bacterial lysates (WCBL) derived from *NmB* [Cps-, PorA+] or *NmB* [Cps-, PorA-, GFP+]. Coomassie staining detected an expression of GFP absent in the recipient *Neisseria meningitidis* strain (see figure 14).

### Example 13: Up-regulation of the N. meningitidis serogroup B NspA gene by promoter replacement

The aim of the experiment was to replace the endogenous promoter region of the *NspA* gene by the strong *porA* promoter, in order to up-regulate the production of the NspA antigen. For that purpose, a promoter replacement plasmid was constructed using *E. coli* cloning methodologies. A DNA region (924bp) located upstream from the *NspA* coding gene was discovered (SEQ ID NO: 7) in the private Incyte PathoSeq data base containing unfinished genomic DNA sequences of the *Neisseria meningitidis* strain ATCC 13090. A DNA fragment (675bp) covering nucleotides -115 to -790 with respect to the *NspA* gene start codon (ATG) was PCR amplified using oligonucleotides **PNS1'** (5'-CCG CGA ATT CGA CGA AGC CGC CCT CGA C-3') and **PNS2** (5'-CGT CTA GAC GTA GCG GTA TCC GGC TGC -3') containing *Eco*RI and *Xba*I restriction sites (underlined) respectively. The PCR fragment was submitted to restriction with *Eco*RI and *Xba*I and inserted in pUC 18. This plasmid was submitted to circle PCR mutagenesis (Jones & Winistofer (1992), Biotechniques12: 528-534) in order to insert meningococcal uptake sequences required for transformation, and suitable restriction sites allowing cloning of a *CmR*/PorA promoter cassette. The circle PCR was performed using the **BAD01-2** (5'- GGC GCC CGG GCT CGA GCT TAT CGA TGG AAA ACG CAG C-3') & **BAD02-2** (5'-GGC GCC CGG GCT CGA GTT CAG ACG GCG CGC TTA TAT AGT GGA TTA AC -3') oligonucleotides containing uptake sequences and suitable restriction sites (*Xma*I and *Xho*I) underlined. The resulting PCR fragment was gel-purified and digested with *Xho*I. The *CmR*/PorA promoter cassette was amplified from the pUC D15/Omp85 plasmid previously described, using primers **BAD** 15-2 (5'-GGC GCC CGG GCT CGA GTC TAG ACA TCG GGC AAA CAC CCG-3') & **BAD 03-2** (5'- GGC GCC CGG GCT CGA GCA CTA GTA TTA CCC TGT TAT CCC-3') oligonucleotides containing suitable restriction sites (*Xma*I, *Xba*I, *Spe*I and *Xho*I) underlined. The PCR fragment obtained was submitted to digestion and inserted in the circle PCR plasmid restricted with the corresponding enzymes. 10 µg of the recombinant plasmid were linearized and used to transform a strain of *Neisseria meningitidis* serogroup B lacking capsular polysaccharide (*cps*-) and one of the major outer membrane proteins - PorA (*porA*-). Recombinant *Neisseria meningitidis* clones resulting from a double crossing over event PCR screening using oligonucleotides **BAD 25** (5'-GAG CGA AGC CGT CGA ACG C -3') & **BAD08** (5'-CTT AAG CGT CGG ACA TTT CC-3') were selected on GC agar plates containing 5µg/ml chloramphenicol and analyzed for NspA expression. Recombinant bacteria (corresponding to about 5.10⁸ bacteria) were re-suspended in 50 µl of PAGE-SDS buffer, frozen (-20°C) / boiled (100°C) three times and then were separated by PAGE-SDS electrophoresis on a 12.5 % gel. Gels were then stained by Coomassie Brilliant blue R250 or transferred to a nitrocellulose membrane and probed either with an anti-PorA monoclonal antibody or with anti-NspA polyclonal antibody (figure 17). As for Omp85, there is a surprising indication that insertion of the promoter approximately 400 bp upstream of the NspA initiation codon expresses more protein than if placed approximately 100 bp upstream.

The same recombinant pUC plasmid can be used to up-regulate the expression of NspA in a *Neisseria meningitidis* serogroup B strain lacking functional *cps* gene but still expressing PorA.

### Example 14: Up-regulation of the N. meninigitidis serogroup B pldA (omplA) gene by promoter replacement.

The aim of the experiment was to replace the endogenous promoter region of the *pldA* (*omplA*) gene by the strong *porA* promoter in order to up-regulate the production of the PldA (OmplA1) antigen. For that purpose, a promoter replacement plasmid was constructed using *E. coli* cloning methodologies. A DNA region (373bp) located upstream from the *pldA* coding sequence was discovered (SEQ ID NO: 18) in the private Incyte PathoSeq data base of the *Neisseria meningitidis* strain ATCC 13090. This DNA contains the sequence coding for a putative *rpsT* gene. The stop codon of *rpsT* is located 169bp upstream the *pldA* ATG. To avoid the disruption of this potentially important gene, we decided to insert the *CmR*/PorA promoter cassette just upstream of the ATG of *pldA.* For that purpose, a DNA fragment of 992 bp corresponding to the the *rpsT* gene, the 169 bp intergenic sequence and the 499 first nucleotides of *pldA* gene was PCR amplified from *Neisseria meningitidis* serogroup B genomic DNA using oligonucleotides **PLA1 Amo5** (5'-GCC GTC TGA ATT TAA AAT TGC GCG TTT ACA G-3') and **PLA1 Amo3** (5'-GTA GTC TAG ATT CAG ACG GCG CAA TTT GGT TTC CGC AC -3') containing uptake sequences (underlined). PLA1 Amo3 contains also a *Xba*I restriction site. This PCR fragment was cleaned with a High Pure Kit (Roche, Mannheim, Germany) and directly cloned in a pGemT vector (Promega, USA). This plasmid was submitted to circle PCR mutagenesis (Jones & Winistofer (1992)) in order to insert suitable restriction sites allowing cloning of a *CmR*/PorA promoter cassette. The circle PCR was performed using the **CIRCI-Bgl** (5'CCT AGA TCT CTC CGC CCC CCA TTG TCG -3') & either **CIRC1-XH-RBS/2** (5'-CCG CTC GAG TAC AAA AGG AAG CCG ATA TGA ATA TAC GGA ATA TGC G-3') or **CIRC2-XHO/2** (5'-CCG CTC GAG ATG AAT ATA CGG AAT -3') oligonucleotides containing suitable restriction sites (*Bgl*II and *XhoI)* underlined. The *CmR*/PorA promoter cassette was amplified from the pUC D15/Omp85 plasmid previously described, using primers **BAD20** (5'- TCC CCC GGG AGA TCT CAC TAG TAT TAC CCT GTT ATC CC-3') and **CM-PORA-3** (5'- CCG CTC GAG ATA AAA ACC TAA AAA CAT CGG GC-3') containing suitable restriction sites (*Bgl*II and *Xho*I) underlined. This PCR fragment was cloned in the circle PCR plasmid obtained with primers CIRC1-Bgl and CIRC1-XH-RBS/2. This plasmid can be used to transform *Neisseria meningitidis* serogroup B *cps-* and cps- porA- strains. Integration by double crossing-over in the upstream region of *pldA* will direct the insertion of the *porA* promoter directly upstream of the *pldA* ATG. Another cassette was amplified from the genomic DNA of the recombinant *Neisseria meningitidis* serogroup B *cps*-, *porA*-, D15/Omp85+ over-expressing D15/Omp85 by promoter replacement. This cassette contains the *cmR* gene, the *porA* promoter and 400bp corresponding to the 5' flanking sequence of the *D15*/*Omp85* gene. This sequence has been proven to be efficacious for up-regulation of the expression of D15/Omp85 in *Neisseria* and will be tested for the up-regulation of the expression of other *Neisseria* antigens. Primers used for the amplification were **BAD 20** and **CM-PORA-D15/3** (5'- CGG CTC GAG TGT CAG TTC CTT GTG GTG C-3') containing *Xho*I restriction sites (underlined). This PCR fragment was cloned in the circle PCR plasmid obtained with primers CIRC1-Bgl and CIRC2-XHO/2. This plasmid will be used to transform *Neisseria meningitidis* serogroup B *cps-* and *cps*-, *porA*- strains. Integration by double crossing-over in the upstream region of *pldA* will direct the insertion of the *porA* promoter 400bp upstream the *pldA* ATG.

### Example 15: Up-regulation of the N. meninizitidis serogroup B tbpA gene by promoter replacement.

The aim of the experiment was to replace the endogenous promoter region of the *tbpA* gene by the strong *porA* promoter, in order to up-regulate the production of the TbpA antigen. For that purpose, a promoter replacement plasmid was constructed using *E. coli* cloning methodologies. A DNA region (731bp) located upstream from the *tbpA* coding sequence was discovered (SEQ ID NO: 17) in the private Incyte PathoSeq data base of the *Neisseria meningitidis* strain ATCC 13090. This DNA contains the sequence coding for TbpB antigen. The genes are organized in an operon. The *tbpB* gene will be deleted and replaced by the *CmR*/porA promoter cassette. For that purpose, a DNA fragment of 3218bp corresponding to the 509bp 5' flanking region of *tbpB* gene, the 2139bp *tbpB* coding sequence, the 87bp intergenic sequence and the 483 first nucleotides of *tbpA* coding sequence was PCR amplified from *Neisseria meningitidis* serogroup B genomic DNA using oligonucleotides **BAD16** (5'-GGC CTA GCT AGC CGT CTG AAG CGA TTA GAG TTT CAA AAT TTA TTC-3') and **BAD17** (5'-GGC CAA GCT TCA GAC GGC GTT CGA CCG AGT TTG AGC CTT TGC-3') containing uptake sequences and *Nhe*I and *Hind*III restriction sites (underlined). This PCR fragment was cleaned with a High Pure Kit (Boerhinger Mannheim, Germany) and directly cloned in a pGemT vector (Promega, USA). This plasmid was submitted to circle PCR mutagenesis (Jones & Winistofer (1992)) in order to (i) insert suitable restriction sites allowing cloning of a *CmR*/PorA promoter cassette and (ii) to delete 209bp of the 5' flanking sequence of *tbpB* and the *tbpB* coding sequence. The circle PCR was performed using the **BAD 18** (5'-TCC CCC GGG AAG ATC TGG ACG AAA AAT CTC AAG AAA CCG-3') & the **BAD 19** (5'-GGA AGA TCT CCG CTC GAG CAA ATT TAC AAA AGG AAG CCG ATA TGC AAC AGC AAC ATT TGT TCC G -3') oligonucleotides containing suitable restriction sites *Xma*I, *Bgl*II and *XhoI* (underlined). The *CmR*/PorA promoter cassette was amplified from the pUC D15/Omp85 plasmid previously described, using primers **BAD21** (5'- GGA AGA TCT CCG CTC GAG ACA TCG GGC AAA CAC CCG-3') & **BAD20** (5'- TCC CCC GGG AGA TCT CAC TAG TAT TAC CCT GTT ATC CC-3') containing suitable restriction sites *Xma*I*, Spe*I*, Bgl*II and *XhoI* (underlined). This PCR fragment was cloned in the circle PCR plasmid. This plasmid will be used to transform *Neisseria meningitidis* serogroup B *cps-* and *cps- porA-* strains. Integration by double crossing-over in the upstream region of *tbpA* will direct the insertion of the *porA* promoter directly upstream of the *tbpA* ATG.

### Example 16: Up-regulation of the N. meningitidis serogroup B pilQ gene by promoter replacement.

The aim of the experiment was to replace the endogenous promoter region of the *pilQ* gene by the strong *porA* promoter, in order to up-regulate the production of the PilQ antigen. For that purpose, a promoter replacement plasmid was constructed using *E. coli* cloning methodologies. A DNA region (772bp) located upstream from the *pilQ* coding gene was discovered (SEQ ID NO: 12) in the private Incyte PathoSeq data base of the *Neisseria meningitidis* strain ATCC 13090. This DNA contains the sequence coding for PilP antigen. The *pilQ* gene is part of an operon we do not want to disturb, pilins being essential elements of the bacteria. The *CmR*/porA promoter cassette was introduced upstream the *pilQ* gene following the same strategy described for the up-regulation of the expression of the *pldA* gene. For that purpose, a DNA fragment of 866 bp corresponding to the 3' part of the *pilP coding sequence,* the 18bp intergenic sequence and the 392 first nucleotides of *pilQ* gene was PCR amplified from *Neisseria* serogroup B genomic DNA using **PQ-rec5-Nhe** (5'-CTA GCT AGC GCC GTC TGA ACG ACG CGA AGC CAA AGC-3') and **PQ-rec3-Hin** (GCC AAG CTT TTC AGA CGG CAC GGT ATC GTC CGA TTC G-3') oligonucleotides containing uptake sequences and *Nhe*I and *Hind*III restriction sites (underlined). This PCR fragment was directly cloned in a pGemT vector (Promega, USA). This plasmid was submitted to circle PCR mutagenesis (Jones & Winistofer (1992)) in order to insert suitable restriction sites allowing cloning of a *CmR*/PorA promoter cassette. The circle PCR was performed using the **CIRC1-PQ-Bgl** (5'-GGA AGA TCT AAT GGA GTA ATC CTC TTC TTA-3') & either **CIRC1-PQ-XHO** (5'-CCG CTC GAG TAC AAA AGG AAG CCG ATA TGA TTA CCA AAC TGA CAA AAA TC-3') or **CIRC2-PQ-X** (5'-CCG CTC GAG ATG AAT ACC AAA CTG ACA AAA ATC -3') oligonucleotides containing suitable restriction sites *Bgl*II and *XhoI* (underlined). The *CmR*/PorA promoter cassette was amplified from the pUC D15/Omp85 plasmid previously described, using primers **BAD20** (5'- TCC CCC GGG AGA TCT CAC TAG TAT TAC CCT GTT ATC CC-3') and **CM-PORA-3** (5'- CCG CTC GAG ATA AAA ACC TAA AAA CAT CGG GCA AAC ACC C-3') containing suitable restriction sites *Bgl*II and *XhoI* (underlined). This PCR fragment was cloned in the circle PCR plasmid obtained with primers CIRC1-PQ-Bgl and CIRC1-PQ-XHO. This plasmid can be used to transform *Neisseria meningitidis* serogroup B *cps-* and cps-, porA- strains. Integration by double crossing-over in the upstream region of *pilQ* will direct the insertion of the *porA* promoter directly upstream of the *pilQ* ATG.

Another cassette was amplified from the genomic DNA of the recombinant *Neisseria meningitidis* serogroup B *cps-, porA-, D15*/*Omp85*+ over-expressing D15/Omp85 by promoter replacement. This cassette contains the *cmR* gene, the *porA* promoter and 400bp corresponding to the 5' flanking sequence of the *D15*/*Omp85* gene. This sequence has been proven to be efficacious for up-regulation of the expression of D15/Omp85 in *Neisseria meningitidis* and will be tested for the up-regulation of the expression of other *Neisseria* antigens. Primers used for the amplification were **BAD 20** and **CM-PORA-D153** (5'- GGG CTC GAG TGT CAG TTC CTT GTG GTG C-3') containing *Xho*I restriction sites (underlined). This PCR fragment was cloned in the circle PCR plasmid obtained with primers CIRC1-PQ-Bgl and CIRC2-PQ-X. This plasmid can be used to transform *Neisseria meningitidis* serogroup B *cps-* and *cps-, porA-* strains. Integration by double crossing-over in the upstream region of *pilQ* will direct the insertion of the *porA* promoter 400bp upstream the *pilQ* ATG.

### Example 17: Construction of a kanR/sacB cassette for introducing "clean", unmarked mutations in the N. meningitidis chromosome.

The aim of the experiment is to construct a versatile DNA cassette containing a selectable marker for the positive screening of recombination in the chromosome of *Neisseria meningitidis* (ie*: kanR* gene), and a counter selectable marker to delete the cassette from the chromosome after recombination (ie: *sacB* gene). By this method, any heterologous DNA introduced during homologous recombination will be removed from the *Neisseria* chromosome.

A DNA fragment containing the *neoR* gene and the *sacB* gene expressed under the control of its own promoter was obtained by restriction of the pIB 279 plasmid (Blomfield IC, Vaughn V, Rest RF, Eisenstein BI (1991), Mol Microbiol 5:1447-57) with *BamH*I restriction enzyme. The recipient vector was derived from plasmid pCMK, previously described. The *kanR* gene of the pCMK was deleted by restriction with enzymes *Nru*I and *EcoR*V. This plasmid was named pCMKs. The *neoR*/*sacB* cassette was inserted in the pCMKs at a *Bgl*II restriction site compatible with *Bam*HI ends.

*E. coli* harboring the plasmid is unable to grow in the presence of 2% sucrose in the culture medium, confirming the functionality of the *sacB* promoter. This plasmid contains recombinogenic sequences allowing the insertion of the cassette at the *porA* locus in the chromosome of *Neisseria meningitidis* serogroup B. Recombinant *Neisseria* were obtained on GC agar plates containing 200µg/ml of kanamycin. Unfortunately, the *sacB* promoter was not functional in *Neisseria meningitidis:* no growth difference was observed on GC agar plates containing 2% sucrose.

A new cassette was constructed containing the *sacB* gene under the control of the *kanR* promoter. A circle PCR was performed using the plasmid pUC4K ((Amersham Pharmacia Biotech, USA)) as a template with **CIRC-Kan-Nco** (5'-CAT GCC ATG GTT AGA AAA ACT CAT CGA GCA TC-3') & **CIRC-Kan-Xba** (5'-CTA GTC TAG ATC AGA ATT GGT TAA TTG GTT G-3') oligonucleotides containing *NcoI* and *Xba*I restriction sites (underlined). The resulting PCR fragment was gel-purified, digested with *NcoI* and ligated to the *sacB* gene generated by PCR from the pIB279 plasmid with **SAC/NCO/NEW5** (5'-CAT GCC ATG GGA **GGA** TGA ACG ATG AAC ATC AAA AAG TTT GCA A-3') oligonucleotide containing a *NcoI* restriction site (underlined) and a RBS (bold) & **SAC/NCO/NEW3** (5'-GAT CCC ATG GTT ATT TGT TAA CTG TTA ATT GTC-3') oligonucleotide containing a *NcoI* restriction site (underlined). The recombinant *E. coli* clones can be tested for their sensitivity on agar plates containing 2% sucrose. The new *kanR*/*sacB* cassette can be subcloned in the pCMKs and used to transform a *Neisseria meningitidis* serogroup B *cps-* strain. The acquired sucrose sensitivity will be confirmed in *Neisseria.* The pCMKs plasmid will be used to transform the recombinant *kanR*/*SacB Neisseria* to delete the entire cassette inserted in the chromosome at the *porA* locus. Clean recombinant *Neisseria* will be obtained on GC agar plates containing 2% sucrose.

### Example 18: Use of small recombinogenic sequences (43bp) to allow homologous recombination in the chromosome of Neisseria meningitidis.

The aim of the experiment is to use small recombinogenic sequences (43bp) to drive insertions, modifications or deletions in the chromosome of *Neisseria.* The achievement of this experiment will greatly facilitate future work, in terms of avoiding subcloning steps of homologous sequences in *E. coli* (recombinogenic sequences of 43bp can easily be added in the PCR amplification primer). The *kanR* gene was PCR amplified from plasmid pUC4K with oligonucleotides **Kan-PorA-5** (5'-GCC GTC **TGA ACC CGT CAT TCC CGC GCA GGC GGG AAT CCA GTC CGT TCA GTT T**CG GGA AAG CCA CGT TGT GTC-3') containing 43bp homologous to the 5' flanking sequence of *Nm*B *porA* gene (bold) and an uptake sequence (underlined) & **Kan-PorA-3** (5'-TTC AGA CGG **CGC AGC AGG AAT TTA TCG GAA ATA ACT GAA ACC GAA CAG ACT AGG** CTG AGG TCT GCC TCG-3') containing 43bp homologous to the 3' flanking sequence of *Nm*B *porA* gene (bold) and an uptake sequence (underlined). The 1300bp DNA fragment obtained was cloned in pGemT vector (Promega, USA). This plasmid can be used to transform a *Neisseria meningitidis* serogroupB *cps-* strain. Recombinant *Neisseria* will be obtained on GC plates containing 200µg/ml kanamycin. Integrations resulting from a double crossing-over at the *porA* locus will be screened by PCR with primers PPA1 & PPA2 as described previously.

### Example 19: Active protection of mice immunized with WT and recombinant Neisseria meningitidis blebs

Animals were immunised three times (IP route) with 5 µg of the different CMVs adsorbed on Al(OH)3 on days 0, 14 and 28. Bleedings were donc on days 28 (day 14 Post II) and 35 (day 7 post III), and they were challenged on day 35 (IP route). The challenge dose was 20 x LD50 (~10⁷ CFU/mouse). Mortality rate was monitored for 7 days after challenge.

OMVs injected were:
Group1: Cps-, PorA+ blebs
Group2: Cps-, PorA- blebs
Group3: Cps-, PorA-, NspA+ blebs
Group4: Cps-, PorA-, Omp85+ blebs
Group 5: Cps-, PorA-, Hsf+ blebs

Figure 15 illustrates the pattern of these OMVs by analyzed SDS Page (Coomassie staining).

24 hours after the challenge, there was 100% mortality (8/8) in the negative control group (immunised with Al(OH)₃ alone) while mice immunised with the 5 different OMVs preparations were still alive (7 to 8/8 mice survived). Sickness was also monitored during the 7 days and the mice immunised with the NSPA over-expressed blebs appeared to be less sick than the other groups. PorA present in PorA+ blebs is likely to confer extensive protection against infection by the homologous strain. However, protection induced by PorA- up-regulated blebs is likely to be due at least to some extent, to the presence of increased amount ofNspA, Omp85 or Hsf.

### Example 20: Immunogenicity of recombinant blebs measured by whole cell & specific ELISA methods

To measure the ability of the antibodies to recognize the antigens present on the MenB cell surface, the pooled mice sera (from Example 19) were tested by whole cell ELISA (using tetracyclin inactivated cells), and titers were expressed as mid-point titers. All types of bleb antibodies induce a high whole cell Ab titer while the negative control group was clearly negative.

| **Bleb** | **WCE**(H44/76) mid-point titer | |
|---|---|---|
| | **14P2** | **14P3** |
| CPS(-) | | |
| PorA(+) | 23849 | 65539 |
| CPS(-) | | |
| PorA(-) | | 40150 |
| CPS(-) | | |
| PorA(-) | | |
| NSPA(+) | 8435 | 23846 |
| CPS(-) | | |
| PorA(-) | | |
| OMP85(+) | 4747 | 16116 |
| CPS(-) | | |
| PorA(-) | | |
| HSF(+) | 6964 | 22504 |
| (-) | 51 | 82 |

The specific Ab response to available recombinant HSF protein was carried out. Microplates were coated with 1 µg/ml full length HSF molecule.

The results illustrated in Figure 16 show that there was a good specific HSF response when HSF over-expressed OMVs were used to immunize mice (using purified recombinant HSF on the plates). The HSF over-expressed blebs induce a good level of specific antibodies.

## Claims

1. A genetically-engineered bleb preparation from a Neisserial, *Moraxella catarrhalis* or *Haemophilus influenzae* Gram-negative bacterial strain **characterized in that** said preparation is obtainable by employing the following process:
d) a process of modifying lipid A portion of bacterial LPS within the bleb preparation, comprising the steps of identifying a msbB gene involved in rendering the lipid A portion of LPS toxic, engineering a bacterial strain so as to reduce or switch off expression of said gene, and making blebs from said strain.

2. The genetically-engineered bleb preparation of claim 1, wherein the Neisserial strain is a meningococcal, gonococcal or N. meningitidis serogroup B strain.

3. The genetically-engineered bleb preparation of claim 1 or 2, wherein the msbB gene has a nucleotide sequence of SEQ ID NO: 79, 80 or 81

4. The genetically-engineered bleb preparation of claims 1-3, wherein the msbB gene is downregulated via point mutation or deletion.

5. The genetically-engineered bleb preparation of claim 1-3, wherein part or all of the msbB open reading frame or promoter is deleted.

6. A vaccine comprising the genetically-engineered bleb preparation of claims 1-5, and a pharmaceutically acceptable excipient.

7. The vaccine of claim 6 further comprising an adjuvant.

8. The vaccine of claim 7 wherein the adjuvant is: an aluminium salt, aluminium hydroxide gel, aluminium phosphate, a calcium salt, calcium carbonate, an iron salt, a zinc salt, an insoluble suspension of acylated tyrosine, or acylated sugars, cationically or anionically derivatised polysaccharides, or polyphosphazenes, a Th1 adjuvant system, Monophosphoryl lipid A (MPL), 3-de-I-acylated monophosphoryl lipid A (3D-MPL), a combination of MPL together with an aluminium salt, a combination of 3D-MPL together with an aluminium salt, a combination of a MPL and a saponin derivative, a combination of QS21 and 3D-MPL, a combination of QS21 and 3D-MPL here the QS21 is quenched with cholesterol, QS21 and 3D-MPL and tocopherol in an oil in water emulsion, a saponin, QS21, an oil in water emulsion and tocopherol, or unmethylated CpG containing oligonucleotides.

9. The genetically-engineered bleb preparation of claims 1-5 or the vaccine of claims 6-8 for use as an adjuvant in a combination vaccine comprising an other antigen.

10. The genetically-engineered bleb preparation of claims 1-5 for use in immunizing a human host against a disease caused by infection of: *Neisseria meningitidis, Neisseria gonorrhoeae, Haemophilus influenzae,* or *Moraxella catarrhalis.*

## Patentansprüche

1. Zubereitung genetisch manipulierter Bläschen aus einem Gram-negativen Neisseria-, Moraxella catarrhalis- oder Haemophilus influenzae-Bakterienstamm, **dadurch gekennzeichnet, dass** die Zubereitung erhältlich ist, indem das folgende Verfahren verwendet wird:
d) ein Verfahren der Modifizierung des Lipid A-Anteils von bakteriellem LPS in der Bläschen-Zubereitung, umfassend die Schritte aus Identifizieren eines msbB-Gens, das daran beteiligt ist, den Lipid A-Anteil von LPS toxisch zu machen, Manipulieren eines Bakterienstammes, um die Expression des Gens zu reduzieren oder abzuschalten, und Herstellen von Bläschen aus dem Stamm.

2. Zubereitung genetisch manipulierter Bläschen gemäß Anspruch 1, wobei der Neisseria-Stamm ein Meningokokken-, Gonokokken- oder N. meningitidis-Serogruppe B-Stamm ist.

3. Zubereitung genetisch manipulierter Bläschen gemäß Anspruch 1 oder 2, wobei das msbB-Gen eine Nukleotidsequenz von SEQ ID NO: 79, 80 oder 81 besitzt.

4. Zubereitung genetisch manipulierter Bläschen gemäß den Ansprüchen 1 bis 3, wobei das msbB-Gen über Punktmutation oder Deletion herunterreguliert ist.

5. Zubereitung genetisch manipulierter Bläschen gemäß den Ansprüchen 1 bis 3, wobei ein Teil oder alles des offenen Leserahmens oder Promotors von msbB deletiert ist.

6. Impfstoff, umfassend die Zubereitung genetisch manipulierter Bläschen gemäß den Ansprüchen 1 bis 5 und einen pharmazeutisch akzeptablen Exzipienten.

7. Impfstoff gemäß Anspruch 6, ferner umfassend ein Adjuvans.

8. Impfstoff gemäß Anspruch 7, wobei das Adjuvans ist: ein Aluminiumsalz, Aluminiumhydroxid-Gel, Aluminiumphosphat, ein Calciumsalz, Calciumcarbonat, ein Eisensalz, ein Zinksalz, eine unlösliche Suspension aus acyliertem Tyrosin oder acylierten Zuckern, kationisch oder anionisch derivatisierte Polysaccharide oder Polyphosphazene, ein Th1-Adjuvans-System, Monophosphoryllipid A (MPL), 3-de-O-acyliertes Monophosphoryllipid A (3D-MPL), eine Kombination aus MPL zusammen mit einem Aluminiumsalz, eine Kombination aus 3D-MPL zusammen mit einem Aluminiumsalz, eine Kombination aus einem MPL und einem Saponin-Derivat, eine Kombination aus QS21 und 3D-MPL, eine Kombination aus QS21 und 3D-MPL, wobei hier das QS21 mit Cholesterol gedämpft ist, QS21 und 3D-MPL und Tocopherol in einer Öl-in-Wasser-Emulsion, ein Saponin, QS21, eine Öl-in-Wasser-Emulsion und Tocopherol oder unmethyliertes CpG enthaltende Oligonukleotide.

9. Zubereitung genetisch manipulierter Bläschen gemäß den Ansprüchen 1 bis 5 oder Impfstoff gemäß den Ansprüchen 6 bis 8 zur Verwendung als Adjuvans in einem Kombinationsimpfstoff, oder ein anderes Antigen umfasst.

10. Zubereitung genetisch manipulierter Bläschen gemäß den Ansprüchen 1 bis 5 zur Verwendung in der Immunisierung eines menschlichen Wirts gegen eine Erkrankung, die durch Infektion mit Neisseria meningitidis, Neisseria gonorrhoeae, Haemophilus influenzae oder Moraxella catarrhalis verursacht wird.

## Revendications

1. Préparation à vésicule modifiée génétiquement à partir d'une souche bactérienne à gram négatif de *Neisseria, Moraxella catarrhalis* ou *Haemophilus* influenzae, **caractérisée en ce que** ladite préparation peut être obtenue en employant le procédé suivant :
d) procédé de modification de la partie de lipide A de LPS bactérien dans la préparation à vésicule, comprenant les étapes d'identification d'un gène msbB impliqué dans le fait de rendre la partie de lipide A de LPS toxique, de modification d'une souche bactérienne de façon à réduire ou éteindre l'expression dudit gène, et de production de vésicules à partir de ladite souche.

2. Préparation à vésicule modifiée génétiquement selon la revendication 1, dans laquelle la souche de *Neisseria* est une souche méningococcique, gonococcique ou de N. meningitidis de sérogroupe B.

3. Préparation à vésicule modifiée génétiquement selon la revendication 1 ou 2, dans laquelle le gène msbB a une séquence nucléotidique de SEQ ID N° : 79, 80 ou 81.

4. Préparation à vésicule modifiée génétiquement selon les revendications 1 à 3, dans laquelle le gène msbB est régulé négativement via une mutation ou une délétion ponctuelle.

5. Préparation à vésicule modifiée génétiquement selon les revendications 1 à 3, dans laquelle une partie ou la totalité du cadre de lecture ouvert de msbB ou d'un promoteur est supprimée.

6. Vaccin comprenant la préparation à vésicule modifiée génétiquement selon les revendications 1 à 5, et un excipient pharmaceutiquement acceptable.

7. Vaccin selon la revendication 8, comprenant en outre un adjuvant.

8. Vaccin selon la revendication 7, dans lequel l'adjuvant est : un sel d'aluminium, un gel d'hydroxyde d'aluminium, du phosphate d'aluminium, un sel de calcium, du carbonate de calcium, un sel de fer, un sel de zinc, une suspension insoluble de tyrosine acylée ou des sucres acylés, des polysaccharides dérivatisés cationiques ou anioniques ou des polyphosphazènes, un système d'adjuvant Th1, un lipide A monophosphoryle (MPL), un lipide A monophosphoryle 3-dés-1-acylé (3D-MPL), une combinaison de MPL et d'un sel d'aluminium, une combinaison de 3D-MPL et d'un sel d'aluminium, une combinaison d'un MPL et d'un dérivé de saponine, une combinaison de QS21 et de 3D-MPL, une combinaison de QS21 et de 3D-MPL, où ici, QS21 est inactivé avec du cholestérol, de QS21 et 3D-MPL et du tocophérol dans une émulsion huile dans eau, une saponine, QS21, une émulsion huile dans eau et du tocophérol ou CpG non méthylé contenant des oligonucléotides.

9. Préparation à vésicule modifiée génétiquement selon les revendications 1 à 5 ou vaccin selon les revendications 6 à 8 pour son utilisation comme adjuvant dans un vaccin combiné comprenant un autre antigène.

10. Préparation à vésicule modifiée génétiquement selon les revendications 1 à 5 pour son utilisation pour immuniser un hôte humain contre une maladie provoquée par une infection à *Neisseria meningitidis, Neisseria gonorrhoeae, Haemophilus influenzae* ou *Moraxella catarrhalis.*


### SEQUENCE LISTING

<110> 5mithKline Beecham Biologicals s.a.
<120> vaccine Composition
<130> 845189
<160> 156
<170> FastSEQ for Windows version 3.0
<210> 1
   <211> 5893
   <212> DNA
   <213> Artificial Sequence
<220>
   <223> pCMK(+) vector
<400> 1
   tcttccgctt cctcgctcac tgactcgctg cgctcggtcg ttcggctgcg gcgagcggta      60
   tcagctcact caaaggcggt aatacggtta tccacagaat caggggataa cgcaggaaag     120
   aacatgtgag caaaaggcca gcaaaaggcc aggaaccgta aaaaggccgc gttgctggcg     180
   tttttccata ggctccgccc ccctgacgag catcacaaaa atcgacgctc aagtcagagg     240
   tggcgaaacc cgacaggact ataaagatac caggcgtttc cccctggaag ctccctcgtg     300
   cgctctcctg ttccgaccct gccgcttacc ggatacctgt ccgcctttct cccttcggga     360
   agcgtggcgc tttctcatag ctcacgctgt aggtatctca gttcggtgta ggtcgttcgc     420
   tccaagctgg gctgtgtgca cgaacccccc gttcagcccg accgctgcgc cttatccggt     480
   aactatcgtc ttgagtccaa cccggtaaga cacgacttat cgccactggc agcagccact     540
   ggtaacagga ttagcagagc gaggtatgta ggcggtgcta cagagttctt gaagtggtgg     600
   cctaactacg gctacactag aagaacagta tttggtatct gcgctctgct gaagccagtt     660
   accttcggaa aaagagttgg tagctcttga tccggcaaac aaaccaccgc tggtagcggt     720
   ggtttttttg tttgcaagca gcagattacg cgcagaaaaa aaggatctca agaagatcct     780
   ttgatctttt ctacggggtc tgacgctcag tggaacgaaa actcacgtta agggattttg     840
   gtcatgagat tatcaaaaag gatcttcacc tagatccttt taaattaaaa atgaagtttt     900
   aaatcaatct aaagtatata tgagtaaact tggtctgaca gttaccaatg cttaatcagt     960
   gaggcaccta tctcagcgat ctgtctattt cgttcatcca tagttgcctg actccccgtc    1020
   gtgtagataa ctacgatacg ggagggctta ccatctggcc ccagtgctgc aatgataccg    1080
   cgagacccac gctcaccggc tccagattta tcagcaataa accagccagc cggaagggcc    1140
   gagcgcagaa gtggtcctgc aactttatcc gcctccatcc agtctattaa ttgttgccgg    1200
   gaagctagag taagtagttc gccagttaat agtttgcgca acgttgttgc cattgctaca    1260
   ggcatcgtgg tgtcacgctc gtcgtttggt atggcttcat tcagctccgg ttcccaacga    1320
   tcaaggcgag ttacatgatc ccccatgttg tgcaaaaaag cggttagctc cttcggtcct    1380
   ccgatcgttg tcagaagtaa gttggccgca gtgttatcac tcatggttat ggcagcactg    1440
   cataattctc ttactgtcat gccatccgta agatgctttt ctgtgactgg tgagtactca    1500
   accaagtcat tctgagaata gtgtatgcgg cgaccgagtt gctcttgccc ggcgtcaata    1560
   cgggataata ccgcgccaca tagcagaact ttaaaagtgc tcatcattgg aaaacgttct    1620
   tcggggcgaa aactctcaag gatcttaccg ctgttgagat ccagttcgat gtaacccact    1680
   cgtgcaccca actgatcttc agcatctttt actttcacca gcgtttctgg gtgagcaaaa    1740
   acaggaaggc aaaatgccgc aaaaaaggga ataagggcga cacggaaatg ttgaatactc    1800
   atactcttcc tttttcaata ttattgaagc atttatcagg gttattgtct catgagcgga    1860
   tacatatttg aatgtattta gaaaaataaa caaatagggg ttccgcgcac atttccccga    1920
   aaagtgccac ctgacgtcta agaaaccatt attatcatga cattaaccta taaaaatagg    1980
   cgtatcacga ggccctttcg tctcgcgcgt ttcggtgatg acggtgaaaa cctctgacac    2040
   atgcagctcc cggagacggt cacagcttgt ctgtaagcgg atgccgggag cagacaagcc    2100
   cgtcagggcg cgtcagcggg tgttggcggg tgtcggggct ggcttaacta tgcggcatca    2160
   gagcagattg tactgagagt gcaccataaa attgtaaacg ttaatatttt gttaaaattc    2220
   gcgttaaatt tttgttaaat cagctcattt tttaaccaat aggccgaaat cggcaaaatc    2280
   ccttataaat caaaagaata gcccgagata gggttgagtg ttgttccagt ttggaacaag    2340
   agtccactat taaagaacgt ggactccaac gtcaaagggc gaaaaaccgt ctatcagggc    2400
   gatggcccac tacgtgaacc atcacccaaa tcaagttttt tggggtcgag gtgccgtaaa    2460
   gcactaaatc ggaaccctaa agggagcccc cgatttagag cttgacgggg aaagccggcg    2520
   aacgtggcga gaaaggaagg gaagaaagcg aaaggagcgg gcgctagggc gctggcaagt    2580
   gtagcggtca cgctgcgcgt aaccaccaca cccgccgcgc ttaatgcgcc gctacagggc    2640
   gcgtactatg gttgctttga cgtatgcggt gtgaaatacc gcacagatgc gtaaggagaa    2700
   aataccgcat caggcgccat tcgccattca ggctgcgcaa ctgttgggaa gggcgatcgg    2760
   tgcgggcctc ttcgctatta cgccagctgg cgaaaggggg atgtgctgca aggcgattaa    2820
   gttgggtaac gccagggttt tcccagtcac gacgttgtaa aacgacggcc agtgccaagc    2880
   ttgccgtctg aatacatccc gtcattcctc aaaaacagaa aaccaaaatc agaaacctaa    2940
   aatcccgtca ttcccgcgca ggcgggaatc cagtccgttc agtttcggtc atttccgata    3000
   aattcctgct gcttttcatt tctagattcc cactttcgtg ggaatgacgg cggaagggtt    3060
   ttggtttttt ccgataaatt cttgaggcat tgaaattcta gattcccgcc tgcgcgggaa    3120
   tgacggctgt agatgcccga tggtctttat agcggattaa caaaaatcag gacaaggcga    3180
   cgaagccgca gacagtacag atagtacgga accgattcac ttggtgcttc agcaccttag    3240
   agaatcgttc tctttgagct aaggcgaggc aacgccgtac ttgtttttgt taatccacta    3300
   taaagtgccg cgtgtgtttt tttatggcgt tttaaaaagc cgagactgca tccgggcagc    3360
   agcgcatcgg cccgcacgag gtctctggag tcgcgagcat caagggcgaa ttctgcaggg    3420
   ggggggggga aagccacgtt gtgtctcaaa atctctgatg ttacattgca caagataaaa    3480
   atatatcatc atgaacaata aaactgtctg cttacataaa cagtaataca aggggtgtta    3540
   tgagccatat tcaacgggaa acgtcttgct cgaggccgcg attaaattcc aacatggatg    3600
   ctgatttata tgggtataaa tgggctcgcg ataatgtcgg gcaatcaggt gcgacaatct    3660
   atcgattgta tgggaagccc gatgcgccag agttgtttct gaaacatggc aaaggtagcg    3720
   ttgccaatga tgttacagat gagatggtca gactaaactg gctgacggaa tttatgcctc    3780
   ttccgaccat caagcatttt atccgtactc ctgatgatgc atggttactc accactgcga    3840
   tccccgggaa aacagcattc caggtattag aagaatatcc tgattcaggt gaaaatattg    3900
   ttgatgcgct ggcagtgttc ctgcgccggt tgcattcgat tcctgtttgt aattgtcctt    3960
   ttaacagcga tcgcgtattt cgtctcgctc aggcgcaatc acgaatgaat aacggtttgg    4020
   ttgatgcgag tgattttgat gacgagcgta atggctggcc tgttgaacaa gtctggaaag    4080
   aaatgcataa gcttttgcca ttctcaccgg attcagtcgt cactcatggt gatttctcac    4140
   ttgataacct tatttttgac gaggggaaat taataggttg tattgatgtt ggacgagtcg    4200
   gaatcgcaga ccgataccag gatcttgcca tcctatggaa ctgcctcggt gagttttctc    4260
   cttcattaca gaaacggctt tttcaaaaat atggtattga taatcctgat atgaataaat    4320
   tgcagtttca tttgatgctc gatgagtttt tctaatcaga attggttaat tggttgtaac    4380
   actggcagag cattacgctg acttgacggg acggcggctt tgttgaataa atcgaacttt    4440
   tgctgagttg aaggatcaga tcacgcatct tcccgacaac gcagaccgtt ccgtggcaaa    4500
   gcaaaagttc aaaatcacca actggtccac ctacaacaaa gctctcatca accgtggctc    4560
   cctcactttc tggctggatg atggggcgat tcaggcctgg tatgagtcag caacaccttc    4620
   ttcacgaggc agacctcagc gccccccccc ccctgcagga ggtctgcgct tgaattgtgt    4680
   tgtagaaaca caacgttttt gaaaaaataa gctattgttt tatatcaaaa tataatcatt    4740
   tttaaaataa aggttgcggc atttatcaga tatttgttct gaaaaatggt tttttgcggg    4800
   ggggggggta taattgaaga cgtatcgggt gtttgcccgg aattgtgagc ggataacaat    4860
   tcgatgtttt taggttttta tcaaatttac aaaaggaagc ccatatgcat cctaggccta    4920
   ttaatattcc ggagtatacg tagccggcta acgttaacaa ccggtacctc tagaactata    4980
   gctagcatgc gcaaatttaa agcgctgata tcgatcgcgc gcagatctga ttaaataggc    5040
   gaaaatacca gctacgatca aatcatcgcc ggcgttgatt atgatttttc caaacgcact    5100
   tccgccatcg tgtctggcgc ttggctgaaa cgcaataccg gcatcggcaa ctacactcaa    5160
   attaatgccg cctccgtcgg tttgcgccac aaattctaaa tatcggggcg gtgaagcgga    5220
   tagctttgtt tttgacggct tcgccttcat tctttgattg caatctgact gccaatctgc    5280
   ttcagcccca aacaaaaacc cggatacgga agaaaaacgg caataaagac agcaaatacc    5340
   gtctgaaaga ttttcagacg gtatttcgca tttttggctt ggtttgcaca tatagtgaga    5400
   ccttggcaaa aatagtctgt taacgaaatt tgacgcataa aaatgcgcca aaaaattttc    5460
   aattgcctaa aaccttccta atattgagca aaaagtagga aaaatcagaa aagttttgca    5520
   ttttgaaaat gagattgagc ataaaatttt agtaacctat gttattgcaa aggtctcgaa    5580
   ttgtcattcc cacgcaggcg ggaatctagt ctgttcggtt tcagttattt ccgataaatt    5640
   cctgctgcgc cgtctgaaga attcgtaatc atggtcatag ctgtttcctg tgtgaaattg    5700
   ttatccgctc acaattccac acaacatacg agccggaagc ataaagtgta aagcctgggg    5760
   tgcctaatga gtgagctaac tcacattaat tgcgttgcgc tcactgcccg ctttccagtc    5820
   gggaaacctg tcgtgccagc tgcattaatg aatcggccaa cgcgcgggga gaggcggttt    5880
   gcgtattggg cgc                                                        5893
<210> 2
   <211> 997
   <212> DNA
   <213> Neisseria meningitidis
<400> 2
   ggaaccgaac acgccgttcg gtcatacgcc gccgaaaggt ttgccgcaag acgaagccgc     60
   cctcgacatc gaagacgcgg tacacggcgc gctggaaagc gcgggttttg tccactacga    120
   aacatcggct tttgcgaaac cagccatgca gtgccgccac aatttgaact actggcagtt    180
   cggcgattat ttaggcatag gcgcgggcgc gcacggcaaa atttcctatc ccgaccgcat    240
   cgagcgcacc gtccgccgcc gccaccccaa cgactacctc gccttaatgc aaaaccgacc    300
   gagcgaagcc gtcgaacgca aaaccgtcgc cgccgaagat ttgccgttcg aattcatgat    360
   gaacgccctg cgcctgaccg acggcgtacc caccgcgatg ttgcaggagc gcacgggcgt    420
   accgagtgcc aaaatcatgg cgcaaatcga aacggcaagg caaaaaggcc tgctggaaac    480
   cgaccccgcc gtattccgcc cgaccgaaaa aggacgcttg tttttaaacg atttgctgca    540
   gtgtttttta tagtggatta acaaaaacca gtacggcgtt gcctcgcctt agctcaaaga    600
   gaacgattct ctaaggtgct gaagcaccaa gtgaatcggt tccgtactat ctgtactgtc    660
   tgcggcttcg tcgccttgtc ctgatttttg ttaatccact atataagcgc aaacaaatcg    720
   gcggccgccc gggaaaaccc ccccgaacgc gtccggaaaa tatgcttatc gatggaaaac    780
   gcagccgcat cccccgccgg gcgtttcaga cggcacagcc gccgccggaa atgtccgacg    840
   cttaaggcac agacgcacac aaaaaaccgt atgcctgcac ctgcaacaat ccgacagata    900
   ccgctgtttt ttccaaaccg tttgcaagtt tcacccatcc gccgcgtgat gccgccacca    960
   ccatttaaag gcaacgcgcg ggttaacggc tttgccg                             997
<210> 3
   <211> 1000
   <212> DNA
   <213> Neisseria meningitidis
<400> 3
   accattgccg cccgcgccgg cttccaaagc ggcgacaaaa tacaatccgt caacggcaca     60
   cccgttgcag attggggcag cgcgcaaacc gaaatcgtcc tcaacctcga agccggcaaa    120
   gtcgccgtcg ggttcagacg gcatcaggcg cgcaaaccgt ccgcaccatc gatgccgcag    180
   gcacgccgga agccggtaaa atcgcaaaaa accaaggcta catcggactg atgcccttta    240
   aaatcacaac cgttgccggt ggcgtggaaa aaggcagccc cgccgaaaaa gcaggcctga    300
   aaccgggcga caggctgact gccgccgacg gcaaacccat tacctcatgg caagaatggg    360
   caaacctgac ccgccaaagc cccggcaaaa aaatcaccct gaactacgaa cgcgccggac    420
   aaacccatac cgccgacatc cgccccgata ctgtcgaaca gcccgaccac accctgatcg    480
   ggcgcgtcgg cctccgtccg cagccggaca gggcgtggga cgcgcaaatc cgccgcagct    540
   accgtccgtc tgttatccgc gcattcggca tgggctggga aaaaaccgtt tcccactcgt    600
   ggacaaccct caaatttttc ggcaaactaa tcagcggcaa cgcctccgtc agccatattt    660
   ccgggccgct gaccattgcc gacattgccg gacagtccgc cgaactcggc ttgcaaagtt    720
   atttggaatt tttggcactg gtcagcatca gcctcggcgt gctgaacctg ctgcccgtcc    780
   ccgttttgga cggcggccac ctcgtgtttt atactgccga atggatacgc ggcaaacctt    840
   tgggcgaacg cgtccaaaac atcggtttgc gcttcgggct tgccctcatg atgctgatga    900
   tggcggtcgc cttcttcaac gacgttaccc ggctgctcgg ttagatttta cgtttcggaa    960
   tgccgtctga aaccgcattc cgcaccacaa ggaactgaca                         1000
<210> 4
   <211> 1036
   <212> DNA
   <213> Neisseria meningitidis
<400> 4
   attcccgcgc aggcgggaat ccagaaacgc aacgcaacag gaatttatcg gaaaaaacag      60
   aaacctcacc gccgtcattc ccgcaaaagc gggaatctag aaacacaacg cggcaggact     120
   ttatcagaaa aaacagaaac cccaccgccg tcattcccgc aaaagcggga atccagaccc     180
   gtcggcacgg aaacttaccg gataaaacag tttccttaga ttccacgtcc tagattcccg     240
   ctttcgcggg aatgacgaga ttttagatta tgggaattta tcaggaatga ttgaatccat     300
   agaaaaacca caggaatcta tcagaaaaaa cagaaacccc caccgcgtca ttcccgcgca     360
   ggcgggaatc cagaaacaca acgcggcagg actttatcgg aaaaaaccga aaccccaccg     420
   accgtcattc ccgcaaaagt tggaatccaa aaacgcaacg caacaggaat ttatcggaaa     480
   aaacagaaac ccccaccgcg tcattcccgc gcaggcggga atccagaaac acaacgcaac     540
   aggaatttat cggaaaaaac agaaacccca ccgaccgtca ttcccgcaaa agcgggaatc     600
   cagcaaccga aaaaccacag gaatctatca gcaaaaacag aaacccccac cgaccgtcat     660
   tcccgcgcag gcgggaatcc agaaacacaa cgcggcagga ctttatcgga aaaaacagaa     720
   accccaccga ccgtcattcc cgcaaaagct ggaatccaaa aacgcaacgc aacaggaatt     780
   tatcggaaaa aacagaaacc ccaccgccgt cattcccgca aaagcgggaa tccagacccg     840
   tcggcacgga aacttaccgg ataaaacagt ttccttagat tccacgtccc agattcccgc     900
   cttcgcggga atgacgagat tttaagttgg gggaatttat cagaaaaccc ccaaccccca     960
   aaaaccgggc ggatgccgca ccatccgccc ccaaaccccg atttaaccat tcaaacaaac    1020
   caaaagaaaa aacaaa                                                    1036
<210> 5
   <211> 772
   <212> DNA
   <213> Neisseria meningitidis
<400> 5
   gcgatgtcgg gaagccttct cccgaatcat taccccttga gtcgctgaaa atcgcccaat      60
   ctccggaaaa cggcggcaat catgacggca agagcagcat cctgaacctc agtgccattg     120
   ccaccaccta ccaagcaaaa tccgtagaag agcttgccgc agaagcggca caaaatgccg     180
   agcaaaaata acttacgtta gggaaaccat gaaacactat gccttactca tcagctttct     240
   ggctctctcc gcgtgttccc aaggttctga ggacctaaac gaatggatgg cacaaacgcg     300
   acgcgaagcc aaagcagaaa tcataccttt ccaagcacct accctgccgg ttgcgccggt     360
   atacagcccg ccgcagctta cagggccgaa cgcattcgac ttccgccgca tggaaaccga     420
   caaaaaaggg gaaaatgccc ccgacaccaa gcgtattaaa gaaacgctgg aaaaattcag     480
   tttggaaaat atgcgttatg tcggcatttt gaagtctgga cagaaagtct ccggcttcat     540
   cgaggctgaa ggttatgtct acactgtcgg tgtcggcaac tatttgggac aaaactacgg     600
   tagaatcgaa agcattaccg acgacagcat cgtcctgaac gagctgatag aagacagcac     660
   gggcaactgg gtttcccgta aagcagaact gctgttgaat tcttccgaca aaaacaccga     720
   acaagcggca gcacctgccg cagaacaaaa ttaagaagag gattactcca tt             772
<210> 6
   <211> 1057
   <212> DNA
   <213> Neisseria meningitidis
<400> 6
   gtgcggcaaa aaacagcaaa agcccgctgt cgattgcctg accgtccgcg tccgtaaaat      60
   cagcataggt tgccacgcgc ggcttgggcg ttttcccaca caaagcctct gccatcggca     120
   gcaggttttt ccccgatatg cgtatcacgc ccacgccgcc gcgcccgggt gcggtagcga     180
   ctgccgcaat cgttggaacg ttatccgaca taaaaccccc gaaaattcaa aacagccgcg     240
   attatagcaa atgccgtctg aagtccgacg gtttggcttt cagacggcat aaaaccgcaa     300
   aaatgcttga taaatccgtc cgcctgacct aatataacca tatggaaaaa cgaaacacat     360
   acgccttcct gctcggtata ggctcgctgc tgggtctgtt ccatcccgca aaaaccgcca     420
   tccgccccaa tcccgccgac gatctcaaaa acatcggcgg cgattttcaa cgcgccatag     480
   agaaagcgcg aaaatgaccg aaaacgcaca ggacaaggcg cggcaggctg tcgaaaccgt     540
   cgtcaaatcc ccggagcttg tcgagcaaat cctgtccgac gagtacgtgc aaataatgat     600
   agcccggcgt ttccattcgg gatcgttgcc gccgccgtcc gacttggcgc aatacaacga     660
   cattatcagc aacggggcag accgcattat ggcaatggcg gaaaaagaac aagccgtccg     720
   gcacgaaacc atacggcaag accaaacctt caacaggcgc gggcaactgt acggcttcat     780
   cagcgtcatc ctgatactgc tttttgccgt cttcctcgta tggagcggct accccgcaac     840
   cgccgcctcc cttgccggcg gcacagtggt tgccttggcg ggtgctttcg tgattggaag     900
   aagccgagac caaggcaaaa attaattgca aatcctaggg cgtgcttcat atccgcccga     960
   acgccgaacc gcacatatag gcacatcccg cgcgccgccg gaagcggaag ccgcgccctc    1020
   ccaaacaaac ccgaatcccg tcagataagg aaaaata                            1057
<210> 7
   <211> 924
   <212> DNA
   <213> Neisseria meningitidis
<400> 7
   ggaaccgaac acgccgttcg gtcatacgcc gccgaaaggt ttgccgcaag acgaagccgc      60
   cctcgacatc gaagacgcgg tacacggcgc gctggaaggc gcgggttttg tccactacga     120
   aacatcggct tttgcgaaac cagccatgca gtgccgccac aatttgaact actggcagtt     180
   cggcgattat ttaggcatag gcgcgggcgc tcacggcaaa atttcctatc ccgaccgcat     240
   cgagcgcacc gtccgccgcc gccaccccaa cgactacctc gccttaatgc aaagccaacc     300
   gagtgaagcc gtcgaacgca aaaccgttgc cgccgaagat ttgccgtttg agttcatgat     360
   gaacgccctg cgcctgaccg acgcgtaccc gccgcgatgt tgcaggagcg cacgggcgta     420
   ccgagtgcca aaatcatggc gcaaatcgaa acggcaaggc aaaaaggcct gctggaaacc     480
   gaccccgccg tattccgccc gaccgaaaaa ggacgcttgt ttttaaacga tttgctgcag     540
   tgttttttat agtggattaa caaaaaccag tacggcgttg cctcgcctta gctcaaagag     600
   aacgattctc taaggtgctg aagcaccaag tgaatcggtt ccgtactatt tgtactgtct     660
   gcggcttcgt cgccttgtcc tgatttttgt taatccacta tataagcgca aacaaatcgg     720
   cggccgcccg ggaaaacccg ccccgaacgc gtccggaaaa tatgcttatc gatggaaaac     780
   gcagccgcat cccccgccgg gcgtttcaga cggcacagcc gccgccggaa atgtccgacg     840
   cttaaggcac agacgcacac aaaaccgtat gcctgcacct gcaacaatcc gacagatacc     900
   gctgtttttt ccaaaccgtt tgca                                           924
<210> 8
   <211> 1000
   <212> DNA
   <213> Neisseria meningitidis
<400> 8
   aagtgggaat ctaaaaatga aaagcaacag gaatttatcg gaaatgaccg aaactgaacg      60
   gactggattc ccgctttcgc gggaatgacg gcgacagggt tgctgttata gtggatgaac     120
   aaaaaccagt acgtcgttgc ctcgccttag ctcaaagaga acgattctct aaggtgctga     180
   agcaccaagt gaatcggttc cgtcctattt gtactgtctg cggcttcgtc gccttgtcct     240
   gatttctgtt cgttttcggt tattcccgat aaattaccgc cgtttctcgt catttcttta     300
   acccttcgtc attcccgcgc aggcgggaat ctagtttttt tgagttccag ttgtttctga     360
   taaattcttg cagctttgag ttcctagatt cccactttcg tgggaatgac ggtggaaaag     420
   ttgccgtgat ttcggataaa ttttcgtaac gcataatttc cgttttaccc gataaatgcc     480
   cgcaatctca aatcccgtca ttccccaaaa acaaaaaatc aaaaacagaa atatcgtcat     540
   tcccgcgcag gcgggaatct agaccttaga acaacagcaa tattcaaaga ttatctgaaa     600
   gtccgagatt ctagattccc actttcgtgg gaatgacgaa ttttaggttt ctgtttttgg     660
   ttttctgtcc ttgcgggaat gatgaaattt taagttttag gaatttatcg gaaaaaacag     720
   aaaccgctcc gccgtcattc ccgcacaggc ttcgtcattc ccgcgcaggc ttcgtcattc     780
   ccgcatttgt taatccacta tattcccgcc gttttttaca tttccgacaa aacctgtcaa      840
   caaaaaacaa cacttcgcaa ataaaaacga taatcagctt tgcaaaaatc ccccccccct      900
   gttaatataa ataaaaataa ttaattaatt atttttctta tcctgccaaa tcttaacggt     960
   ttggatttac ttcccttcat acactcaaga ggacgattga                          1000
<210> 9
   <211> 1000
   <212> DNA
   <213> Neisseria meningitidis
<400> 9
   ctataaagat gtaaataaaa atctcggtaa cggtaacact ttggctcagc aaggcagcta      60
   caccaaaaca gacggtacaa ccgcaaaaat gggggattta cttttagcag ccgacaatct     120
   gcacagccgc ttcacgaaca aaatgctatc cattagccat gttcgggaaa acacgatttc     180
   cccgtttgtt ttaggctgtc taaacaaata accataaatg tatatcatta tttaaaataa     240
   ataaaagtat ttaactatta ttgacgaaat tttagagaaa gagtagactg tcgattaaat     300
   gacaaacaat agtgagaaag gaaatattta ctatccgagc acagagcata ttttaggtag     360
   cctgtaactg ttcctgctgg cggaagagga tgaaggtgga cttacccgag aataaatgtc     420
   ctgttgtgtg atatggatgc catgccgcga agcaattgat gcaatcacgg cagtcctact     480
   tgaatgaaac ctgtcgttgc agaatttgaa aacgctattt ttaagaaagg ataaagggag     540
   aaagaatttt tggtttttaa gctgcatgaa accgtgttgg aataaatgca cacctacgat     600
   aattaataat tttcgttttt tattctacaa gctatttata tatgattgct aaaagtttat     660
   tttttagatg ccaaaaaata tattttatat acttcatatt gtttatatgt ctttatttga     720
   atatatctta cgatggggaa atatttatat attttataat aaattttact catttgctaa     780
   tatgtcatgg aatattactt gtattttgta gaatttttcc atatgaaaat attccattta     840
   ctatttttct gaactttatt agtttatttt taatattttt acctcttata tttaccataa     900
   gagagctaat tgattcatat tatattgagt cgataattaa tttattctta attttaattc     960
   ctcacgttat ttttttaatt tacttgaaag gaaagcagat                          1000
<210> 10
   <211> 1000
   <212> DNA
   <213> Neisseria meningitidis
<400> 10
   ggaaacagag aaaaaagttt ctcttctatc ttggataaat atatttaccc tcagtttagt      60
   taagtattgg aatttatacc taagtagtaa aagttagtaa attattttta actaaagagt     120
   tagtatctac cataatatat tctttaacta atttctaggc ttgaaattat gagaccatat     180
   gctactacca tttatcaact ttttattttg tttattggga gtgtttttac tatgacctca     240
   tgtgaacctg tgaatgaaaa gacagatcaa aaagcagtaa gtgcgcaaca ggctaaagaa     300
   caaaccagtt tcaacaatcc cgagccaatg acaggatttg aacatacggt tacatttgat     360
   tttcagggca ccaaaatggt tatcccctat ggctatcttg cacggtatac gcaagacaat     420
   gccacaaaat ggctttccga cacgcccggg caggatgctt actccattaa tttgatagag     480
   attagcgtct attacaaaaa aaccgaccaa ggctgggttc ttgagccata caaccagcaa     540
   aacaaagcac actttatcca atttctacgc gacggtttgg atagcgtgga cgatattgtt     600
   atccgaaaag atgcgtgtag tttaagtacg actatgggag aaagattgct tacttacggg     660
   gttaaaaaaa tgccatctgc ctatcctgaa tacgaggctt atgaagataa aagacatatt     720
   cctgaaaatc catattttca tgaattttac tatattaaaa aaggagaaaa tccggcgatt     780
   attactcatc ggaataatcg aataaaccaa actgaagaag atagttatag cactagcgta     840
   ggttcctgta ttaacggttt cacggtacag tattacccgt ttattcggga aaagcagcag     900
   ctcacacagc aggagttggt aggttatcac caacaagtag agcaattggt acagagtttt     960
   gtaaacaatt caaataaaaa ataatttaaa ggatcttatt                          1000
<210> 11
   <211> 1000
   <212> DNA
   <213> Neisseria meningitidis
<400> 11
   acgtccgaac cgtgattccg caacgccgcg cccaaaacca aagcccaagc caaaatgccg      60
   atatagttgg cattggcaat cgcgttaatc gggttggcga ccaggttcat cagcagcgat     120
   ttcaacactt ccacaatgcc ggaaggcggc gcggcggaca catcgcccgc gcccgccaaa     180
   acaatgtgcg tcgggaaaac cataccggcg atgacggcgg tcagggctgc ggaaaacgta     240
   ccaatgaggt aaaggatgat aatcggcctg atatgcgcct tgttgccttt ttggtgctgc     300
   gcgattgtgg ccgccaccaa aataaatacc aaaaccggcg cgaccgcttt gagcgcgccg     360
   acaaacaggc tgccgaacaa gcctgccgcc aagcccagtt gcggggaaac cgaaccgatt     420
   acgatgccca acgccaaacc ggcggcaatc tgcctgacca ggctgacgcg gccgatcgca     480
   tgaaataagg atttgccgaa cgccataatt cttccttatg ttgtgatatg ttaaaaaatg     540
   ttgtatttta aaagaaaact cattctctgt gtttttttta tttttcggct gtgttttaag     600
   gttgcgttga tttgccctat gcagtgccgg acaggctttg ctttatcatt cggcgcaacg     660
   gtttaattta ttgaacgaaa ataaatttat ttaatcctgc ctattttccg gcactattcc     720
   gaaacgcagc ctgttttcca tatgcggatt ggaaacaaaa taccttaaaa caagcagata     780
   catttccggc gggccgcaac ctccgaaata ccggcggcag tatgccgtct gaagtgtccc     840
   gccccgtccg aacaacacaa aaacagccgt tcgaaaccct gtccgaacag tgttagaatc     900
   gaaatctgcc acaccgatgc acgacacccg taccatgatg atcaaaccga ccgccctgct     960
   cctgccggct ttatttttct ttccgcacgc atacgcgcct                          1000
<210> 12
   <211> 772
   <212> DNA
   <213> Neisseria meningitidis
<400> 12
   gcgatgtcgg gaagccttct cccgaatcat taccccttga gtcgctgaaa atcgcccaat      60
   ctccggaaaa cggcggcaat catgacggca agagcagcat cctgaacctc agtgccattg     120
   ccaccaccta ccaagcaaaa tccgtagaag agcttgccgc agaagcggca caaaatgccg     180
   agcaaaaata acttacgtta gggaaaccat gaaacactat gccttactca tcagctttct     240
   ggctctctcc gcgtgttccc aaggttctga ggacctaaac gaatggatgg cacaaacgcg     300
   acgcgaagcc aaagcagaaa tcataccttt ccaagcacct accctgccgg ttgcgccggt     360
   atacagcccg ccgcagctta cagggccgaa cgcattcgac ttccgccgca tggaaaccga     420
   caaaaaaggg gaaaatgccc ccgacaccaa gcgtattaaa gaaacgctgg aaaaattcag     480
   tttggaaaat atgcgttatg tcggcatttt gaagtctgga cagaaagtct ccggcttcat     540
   cgaggctgaa ggttatgtct acactgtcgg tgtcggcaac tatttgggac aaaactacgg     600
   tagaatcgaa agcattaccg acgacagcat cgtcctgaac gagctgatag aagacagcac     660
   gggcaactgg gtttcccgta aagcagaact gctgttgaat tcttccgaca aaaacaccga     720
   acaagcggca gcacctgccg cagaacaaaa ttaagaagag gattactcca tt             772
<210> 13
   <211> 1000
   <212> DNA
   <213> Neisseria meningitidis
<400> 13
   tttgtttttt cttttggttt gtttgaatgg ttaaatcggg gtttgggggc ggatggtgcg      60
   gcatccgccc ggtttttggg ggttgggggt tttctgataa attcccccaa cttaaaatct     120
   cgtcattccc gcgaaggcgg gaatctggga cgtggaatct aaggaaactg ttttatccgg     180
   taagtttccg tgccgacggg tctggattcc cgcttttgcg ggaatgacgg cggtggggtt     240
   tctgtttttt ccgataaatt cctgttgcgt tgcgtttttg gattccagct tttgcgggaa     300
   tgacggtcgg tggggtttct gttttttccg ataaagtcct gccgcgttgt gtttctggat     360
   tcccgcctgc gcgggaatga cggtcggtgg gggtttctgt ttttgctgat agattcctgt     420
   ggtttttcgg ttgctggatt cccgcttttg cgggaatgac ggtcggtggg gtttctgttt     480
   tttccgataa attcctgttg cgttgtgttt ctggattccc gcctgcgcgg gaatgacgcg     540
   gtgggggttt ctgttttttc cgataaattc ctgttgcgtt gcgtttttgg attccaactt     600
   ttgcgggaat gacggtcggt ggggtttcgg ttttttccga taaagtcctg ccgcgttgtg     660
   tttctggatt cccgcctgcg cgggaatgac gcggtggggg tttctgtttt ttctgataga     720
   ttcctgtggt ttttctatgg attcaatcat tcctgataaa ttcccataat ctaaaatctc     780
   gtcattcccg cgaaagcggg aatctaggac gtggaatcta aggaaactgt tttatccggt     840
   aagtttccgt gccgacgggt ctggattccc gcttttgcgg gaatgacggc ggtggggttt     900
   ctgttttttc tgataaagtc ctgccgcgtt gtgtttctag attcccgctt ttgcgggaat    960
   gacggcggtg aggtttctgt tttttccgat aaattcctgt                           1000
<210> 14
   <211> 1000
   <212> DNA
   <213> Neisseria meningitidis
<400> 14
   aatcagcata ggttgccacg cgcggcttgg gcgttttccc acacaaagcc tctgccatcg      60
   gcagcaggtt tttccccgat atgcgtatca cgcccacgcc gccgcgcccg ggtgcggtag     120
   cgactgccgc aatcgttgga acgttatccg acataaaacc cccgaaaatt caaaacagcc     180
   gcgattatag caaatgccgt ctgaagtccg acggtttggc tttcagacgg cataaaaccg     240
   caaaaatgct tgataaatcc gtccgcctga cctaatataa ccatatggaa aaacgaaaca     300
   catacgcctt cctgctcggt ataggctcgc tgctgggtct gttccatccc gcaaaaaccg     360
   ccatccgccc caatcccgcc gacgatctca aaaacatcgg cggcgatttt caacgcgcca     420
   tagagaaagc gcgaaaatga ccgaaaacgc acaggacaag gcgcggcagg ctgtcgaaac     480
   cgtcgtcaaa tccccggagc ttgtcgagca aatcctgtcc gacgagtacg tgcaaataat     540
   gatagcccgg cgtttccatt cgggatcgtt gccgccgccg tccgacttgg cgcaatacaa     600
   cgacattatc agcaacgggg cagaccgcat tatggcaatg gcggaaaaag aacaagccgt     660
   ccggcacgaa accatacggc aagaccaaac cttcaacagg cgcgggcaac tgtacggctt     720
   catcagcgtc atcctgatac tgctttttgc cgtcttcctc gtatggagcg gctaccccgc     780
   aaccgccgcc tcccttgccg gcggcacagt ggttgccttg gcgggtgctt tcgtgattgg     840
   aagaagccga gaccaaggca aaaattaatt gcaaatccta gggcgtgctt catatccgcc     900
   cgaacgccga accgcacata taggcacatc ccgcgcgccg ccggaagcgg aagccgcgcc     960
   ctcccaaaca aacccgaatc ccgtcagata aggaaaaata                           1000
<210> 15
   <211> 1000
   <212> DNA
   <213> Neisseria meningitidis
<400> 15
   gattttggtc atcccgacaa gcttcttgtc gaagggcgtg aaattccttt ggttagccaa      60
   gagaaaacca tcaagcttgc cgatggcagg gaaatgaccg tccgtgcttg ttgcgacttt     120
   ttgacctatg tgaaactcgg acggataaaa accgaacgcc cggcaagtaa accaaaggcg     180
   gaagataaaa gggaggatga agagagtgca ggcgttggta acgtcgaaga aggcgaaggc     240
   gaagtttccg aagatgaagg cgaagaagcc gaagaaatcg tcgaagaaga acccgaagaa     300
   gaagctgaag aggaagaagc tgaacccaaa gaagttgaag aaaccgaaga aaaatcgccg     360
   acagaagaaa gcggcagcgg ttcaaacgcc atcctgcctg cctcggaagc ctctaaaggc     420
   agggacatcg accttttcct gaaaggtatc cgcacggcgg aagccgacat tccaagaacc    480
   ggaaaagcac actataccgg cacttgggaa gcgcgtatcg gcacacccat tcaatgggac     540
   aatcaggcgg ataaagaagc ggcaaaagca gaatttaccg ttaatttcgg cgagaaatcg     600
   atttccggaa cgctgacgga gaaaaacggt gtacaacctg ctttctatat tgaaaacggc     660
   aagattgagg gcaacggttt ccacgcaaca gcacgcactc gtgagaacgg catcaatctt     720
   tcgggaaatg gttcgaccaa ccccagaacc ttccaagcta gtgatcttcg tgtagaagga     780
   ggattttacg gcccgcagcg gaggaattgg gcggtattat tttcaataag gatgggaaat     840
   ctcttggtat aactgaaggt actgaaaata aagttgaagt tgaagctgaa gttgaagttg     900
   aagctgaaac tggtgttgtc gaacagttag aacctgatga agttaaaccc caattcggcg     960
   tggtattcgg tgcgaagaaa gataataaag aggtggaaaa                          1000
<210> 16
   <211> 1000
   <212> DNA
   <213> Neisseria meningitidis
<400> 16
   cggcgttaga gtttagggca gtaagggcgc gtccgccctt agatctgtaa gttacgattc      60
   cgttaaataa cttttactga ctttgagttt tttgacctaa gggtgaaagc acccttactg     120
   cttaaagtcc aacgacaaaa accaaaagac aaaaacactt ttattaccct aaaatcgaac     180
   acccataaat gacctttttt gtctttggcg aggcggcagt aagggcgcgt ccgcccttag     240
   atctgtaagt tatgattccg ttaaatagcc tttactgact ttgagttttt tgacctaagg     300
   gcggacgcgc ccttactgct tcaccttcaa tgggctttga attttgttcg ctttggcttg     360
   cttgacctaa gggtgaaagc acccttactg ccgcctcgcc aaagacgaaa agggttattt     420
   acgggggttg gattttaggc agtaagggcg cgtccgccct tagatctgta agttatgatt      480
   ccgttaaata gcctttactg actttgagtt ttttgaccta agggtgaaag cacccttact     540
   gcttcacctt caatgggctt tgaattttgt tcgctttggc ttgcttgatc taagggtgaa     600
   agcaccctta ctgccgtctc gccgaagaca acgagggcta tttacggcgt tagagtttag     660
   ggcagtaagg gcgcgtccgc ccttagatcc agacagtcac gcctttgaat agtccatttt     720
   gccaaagaac tctaaaacgc aggacctaag ggtgaaagca cccttactgc cttacatcca     780
   agcaccctta ctgcaccacg tccacgcacc cttactgccc tacgtccacg cacccttact     840
   gccctacatc caagcaccct tactgcctta catagacatg acagacgccg agcagcggaa     900
   caggactaaa aacaattaag tgatattttt gcccaactat aatagacatg tataattata     960
   ttactattaa taataattag tttatcctcc ttttcatccc                          1000
<210> 17
   <211> 731
   <212> DNA
   <213> Neisseria meningitidis
<400> 17
   tatgaagtcg aagtctgctg ttccaccttc aattatctga attacggaat gttgacgcgc      60
   aaaaacagca agtccgcgat gcaggcagga gaaagcagta gtcaagctga tgctaaaacg     120
   gaacaagttg gacaaagtat gttcctccaa ggcgagcgca ccgatgaaaa agagattcca     180
   aacgaccaaa acgtcgttta tcgggggtct tggtacgggc atattgccaa cggcacaagc     240
   tggagcggca atgcttccga taaagagggc ggcaacaggg cggactttac tgtgaatttc     300
   ggtacgaaaa aaattaacgg cacgttaacc gctgacaaca ggcaggcggc aacctttacc     360
   attgtgggcg atattgaggg caacggtttt tccggtacgg cgaaaactgc tgactcaggt     420
   tttgatctcg atcaaagcaa taacacccgc acgcctaagg catatatcac aaacgccaag     480
   gtgcagggcg gtttttacgg gcccaaagcc gaagagttgg gcggatggtt tgcctattcg     540
   gacgataaac aaacgaaaaa tgcaacagat gcatccggca atggaaattc agcaagcagt     600
   gcaactgtcg tattcggtgc gaaacgccaa aagcctgtgc aataagcacg gttgccgaac     660
   aatcaagaat aaggcctcag acggcaccgc tccttccgat accgtctgaa agcgaagagt     720
   agggaaacac t                                                          731
<210> 18
   <211> 373
   <212> DNA
   <213> Neisseria meningitidis
<400> 18
   cgtaccgcat tccgcactgc agtgaaaaaa gtattgaaag cagtcgaagc aggcgataaa      60
   gctgccgcac aagcggttta ccaagagtcc gtcaaagtca tcgaccgcat cgccgacaag     120
   ggcgtgttcc ataaaaacaa agcggctcgc cacaaaaccc gtttgtctca aaaagtaaaa     180
   ccttggcttg atttttgcaa aacctgcaat ccggttttca tcgtcgattc cgaaaacccc     240
   tgaagcccga cggtttcggg gttttctgta ttgcggggac aaaatcccga aatggcggaa     300
   agggtgcggt tttttatccg aatccgctat aaaatgccgt ctgaaaacca atatgccgac     360
   aatgggggtg gag                                                        373
<210> 19
   <211> 1000
   <212> DNA
   <213> Neisseria meningitidis
<400> 19
   ttttggcttc cagcgtttca ttgttttcgt acaagtcgta agtcagcttc agattgttgg      60
   cttttttaaa gtcttcgacc gtactctcat caacatagtt cgaccagttg tagatgttca    120
   gagtatcggt ggcagcggct tcggcattgg cagcagacgc agcgtctgct tgaggttgca     180
   cggcgttttt ttcgctgccg ccgcaggctg ccagagacag cgcggccaaa acggctaata     240
   cggatttttt catacgggca gattcctgat gaaagaggtt ggaaaaaaag aaatccccgc     300
   gccccatcgt taccccggcg caaggtttgg gcattgtaaa gtaaatttgt gcaaactcaa     360
   agcgatattg gactgatttt cctaaaaaat tatcctgttt ccaaaagggg agaaaaacgt     420
   ccgcccgatt ttgccgtttt tttgcgctgt cagggtgtcc gacgggcgga tagagagaaa     480
   aggcttgcat ataatgtaaa ccccctttaa aattgcgcgt ttacagaatt tatttttctt     540
   ccaggagatt ccaatatggc aaacagcgca caagcacgca aacgtgcccg ccagtccgtc     600
   aaacaacgcg cccacaatgc tagcctgcgt accgcattcc gcaccgcagt gaaaaaagta     660
   ttgaaagcag tcgaagcagg cgataaagct gccgcacaag cggtttacca agagtccgtc     720
   aaagtcatcg accgcatcgc cgacaagggc gtgttccaca aaaacaaagc ggcacgccac     780
   aaaagccgtc tgtctgcaaa agtaaaagcc ttggcttgat ttttgcaaaa ccgccaaggc     840
   ggttgatacg cgataagcgg aaaaccctga agcccgacgg tttcggggtt ttctgtattg     900
   cgggggcaaa atcccgaaat ggcggaaagg gtgcgatttt ttatccgaat ccgctataaa     960
   atgccgtttg aaaaccaata tgccgacaat gggggcggag                          1000
<210> 20
   <211> 1000
   <212> DNA
   <213> Neisseria meningitidis
<400> 20
   tacggaaact gcaagcggat ccagaagtta cagcgtgcat tattcggtgc ccgtaaaaaa 60
   atggctgttt tcttttaatc acaatggaca tcgttaccac gaagcaaccg aaggctattc 120
   cgtcaattac gattacaacg gcaaacaata tcagagcagc ctggccgccg agcgcatgct 180
   ttggcgtaac agacttcata aaacttcagt cggaatgaaa ttatggacac gccaaaccta 240
   taaatacatc gacgatgccg aaatcgaagt gcaacgccgc cgctctgcag gctgggaagc 300
   cgaattgcgc caccgtgctt acctcaaccg ttggcagctt gacggcaagt tgtcttacaa 360
   acgcgggacc ggcatgcgcc aaagtatgcc tgcaccggaa gaaaacggcg gcgatattct 420
   tccaggtaca tctcgtatga aaatcattac tgccggtttg gacgcagccg ccccatttat 480
   tttaggcaaa cagcagtttt tctacgcaac cgccattcaa gctcaatgga acaaaacgcc 540
   gttggttgcc caagataaat tgtcaatcgg cagccgctac accgttcgcg gatttgatgg 600
   ggagcagagt cttttcggag agcgaggttt ctactggcag aatactttaa cttggtattt 660
   tcatccgaac catcagttct atctcggtgc ggactatggc cgcgtatttg gcgaaagtgc 720
   acaatatgta tcgggcaagc agctgatggg tgcagtggtc ggcttcagag gagggcataa 780
   agtaggcggt atgtttgctt atgatctgtt tgccggcaag ccgcttcata aacccaaagg 840
   ctttcagacg accaacaccg tttacggctt caacttgaat tacagtttct aacctctgaa 900
   ttttttactg atatttagac ggtctttcct tatcctcaga ccgtcaaact ttacctacgt 960
   acttggcgcg cagtacgttc atcttcaaaa tggaatagac 1000
<210> 21
   <211> 1000
   <212> DNA
   <213> Neisseria meningitidis
<400> 21
   ttatcttggt gcaaaacttt gtcggggtcg gactggctac ggctttgggt ttggacccgc 60
   tcatcggtct gattaccggt tcggtgtcgc tgacgggcgg acacggtacg tcaggtgcgt 120
   ggggacctaa ttttgaaacg caatacggct tggtcggcgc aaccggtttg ggtattgcat 180
   cggctacttt cgggctggtg ttcggcggcc tgatcggcgg gccggttgcg cgccgcctga 240
   tcaacaaaat gggccgcaaa ccggttgaaa acaaaaaaca ggatcaggac gacaacgcgg 300
   acgacgtgtt cgagcaggca aaacgcaccc gcctgattac ggcggaatct gccgttgaaa 360
   cgcttgccat gtttgccgcg tgtttggcgt ttgccgagat tatggacggc ttcgacaaag 420
   aatatctgtt cgacctgccc aaattcgtgt ggtgtctgtt tggcggcgtg gtcatccgca 480
   acatcctcac tgccgcattc aaggtcaata tgttcgaccg cgccatcgat gtgttcggca 540
   atgcttcgct ttcgcttttc ttggcaatgg cgttgctgaa tttgaaactg tgggagctga 600
   ccggtttggc ggggcctgta accgtgattc ttgccgtaca aaccgtggtg atggttttgt 660
   acgcgacttt tgttacctat gtctttatgg ggcgcgacta tgatgcggca gtattggctg 720
   ccggccattg cggtttcggc ttgggtgcaa cgccgacggc ggtggcaaat atgcagtccg 780
   tcacgcatac tttcggcgcg tcgcataagg cgtttttgat tgtgcctatg gtcggcgcgt 840
   tcttcgtcga tttgattaat gccgcgattc tcaccggttt tgtgaatttc tttaaaggct 900
   gattttccgc ctttccgaca aagcacctgc aaggtttacc gcctgcaggt gcttttgcta 960
   tgatagccgc tatcggtctg caccgtttgg aaggaacatc 1000
<210> 22
   <211> 1000
   <212> DNA
   <213> Neisseria meningitidis
<400> 22
   cctactccac cgattccaat atgctcggcg cgacccacga agccaaagac ttggaatttt      60
   tgaactcggg catcaaaatc gtcaaaccca ttatgggcgt tgccttttgg gacgaaaacg     120
   ttgaagtcag ccccgaagaa gtcagcgtgc gctttgaaga aggcgtgccg gttgcactga     180
   acggcaaaga atacgccgac cccgtcgaac tcttcctcga agccaaccgc atcggcggcc     240
   gccacggctt gggtatgagc gaccaaatcg aaaaccgcat catcgaagcc aaatcgcgcg     300
   gcatctacga agccccgggt atggcgttgt tccacatcgc ctacgaacgc ttggtgaccg     360
   gcatccacaa cgaagacacc atcgaacaat accgcatcaa cggcctgcgc ctcggccgtt     420
   tgctctacca aggccgctgg ttcgacagcc aagccttgat gttgcgcgaa accgcccaac     480
   gctgggtcgc caaagccgtt accggcgaag ttaccctcga actgcggcgc ggcaacgact     540
   actcgattct gaacaccgaa tcgcccaacc tgacctacca acccgaacgc ctgagtatgg     600
   aaaaagtcga aggtgcggcg tttaccccgc tcgaccgcat cggacagctc acgatgcgca     660
   acctcgacat caccgacacc cgcgccaaac tgggcatcta ctcgcaaagc ggtttgctgt     720
   cgctgggcga aggctcggta ttaccgcagt tgggcaataa gaaataaggt ttgctgtttt     780
   gcatcattag caacttaagg ggtcgtctga aaagatgatc ccttatgtta aaaggaatcc     840
   tatgaaagaa tacaaagtcg tcatttatca ggaaagccag ttgtccagcc tgtttttcgg     900
   cgcggcaaag gtcaaccccg tcaatttcag cgcgttcctc aacaaacaaa ccccccgaag     960
   gctggcgggt cgagaccttt gcaataacat aggttactaa                          1000
<210> 23
   <211> 1000
   <212> DNA
   <213> Neisseria meningitidis
<400> 23
   gaatgacaat tcataagttt cccgaaattc caacataacc gaaacctgac aataaccgta      60
   gcaactgaac cgtcattccc gcaaaagcgg gaatccagtc cgttcagttt cggtcatttc     120
   cgataaatgc ctgttgcttt tcatttctag attcccactt tcgtgggaat gacggcggaa     180
   gggttttggt tttttccgat aaattcttga ggcattgaaa ttccaaattc ccgcctgcgc     240
   gggaatgacg gctgcagatg cccgacggtc tttatagtgg attaacaaaa atcaggacaa     300
   ggcgacgagc tgcagacagt acagatagta cggaaccgat tcacttagtg cttcagtatc     360
   ttagagaatc gttctctttg agctaaggcg aggcaacgtc gtactggttt ttgttcatcc     420
   actatatatg acacggaaaa cgccgccgtc caaaccatgc cgtctgaaga aaactacaca     480
   gataccgccg cttatattac aatcgccgcc ccgtggttcg aaaacctccc acactaaaaa     540
   actaaggaaa ccctatgtcc cgcaacaacg aagagctgca aggtatctcg cttttgggta     600
   atcaaaaaac ccaatatccg gccgaatacg cgcccgaaat tttggaagcg ttcgacaaca     660
   aacatcccga caacgactat ttcgtcaaat tcgtctgccc agagttcacc agcctctgcc     720
   ccatgaccgg gcagcccgac ttcgccacca tcgtcatccg ctacattccg cacatcaaaa     780
   tggtggaaag caaatccctg aaactctacc tcttcagctt ccgcaaccac ggcgattttc     840
   atgaagactg cgtcaacatc atcatgaaag acctcattgc cctgatggat ccgaaataca     900
   tcgaagtatt cggcgagttc acaccgcgcg gcggcatcgc cattcatcct ttcgccaatt     960
   acggcaaagc aggcaccgag tttgaagcat tggcgcgtaa                          1000
<210> 24
   <211> 228
   <212> DNA
   <213> Neisseria meningitidis
<400> 24
   gatatcgagg tctgcgcttg aattgtgttg tagaaacaca acgtttttga aaaaataagc      60
   tattgtttta tatcaaaata taatcatttt taaaataaag gttgcggcat ttatcagata     120
   tttgttctga aaaatggttt tttgcggggg ggggggtata attgaagacg tatcgggtgt      180
   ttgcccgatg tttttaggtt tttatcaaat ttacaaaagg aagcccat                  228
<210> 25
   <211> 1000
   <212> DNA
   <213> Neisseria meningitidis
<400> 25
   gttttctgtt tttgagggaa tgacgggatg taggttcgta agaatgacgg gatataggtt      60
   tccgtgcgga tggattcgtc attcccgcgc aggcgggaat ctagaacgtg gaatctaaga     120
   aaccgtttta tccgataagt ttccgtgcgg acaagtttgg attcccgcct gcgcgggaat     180
   gacgggattt taggtttcta attttggttt tctgtttttg agggaatgac gggatgtagg     240
   ttcgtaggaa tgacgggata taggtttccg tgcggatgga ttcgtcattc ccgcgcaggc     300
   gggaatctag accttagaac aacagcaata ttcaaagatt atctgaaagt ccgagattct     360
   agattcccgc ctgagcggga atgacgaaaa gtggcgggaa tgacggttag cgttgcctcg     420
   ccttagctca aagagaacga ttctctaagg tgctgaagca ccaagtgaat cggttccgta     480
   ctatttgtac tgtctgcggc ttcgtcgcct tgtcctgatt tttgttaatc cactatctcc     540
   tgccgcaggg gcgggttttg catccgcccg ttccgaaaga aaccgcgtgt gcgttttttg     600
   ccgtctttat aacccccggt ttgcaatgcc ctccaatacc ctcccgagta agtgttgtaa    660
   aaatgcaaat cttaaaaaat ttaaataacc atatgttata aaacaaaaaa tacccataat     720
   atctctatcc gtccttcaaa atgcacatcg aattccacac aaaaacaggc agaagtttgt     780
   tttttcagac aggaacatct atagtttcag acatgtaatc gccgagcccc tcggcggtaa     840
   atgcaaagct aagcggcttg gaaagcccgg cctgcttaaa tttcttaacc aaaaaaggaa     900
   tacagcaatg aaaaaatccc tgattgccct gactttggca gcccttcctg ttgcagcaat     960
   ggctgacgtt accctgtacg gcaccatcaa aaccggcgta                          1000
<210> 26
   <211> 537
   <212> DNA
   <213> Neisseria meningitidis
<400> 26
   gttttctgtt tttgagggaa tgacgggatg taggttcgta agaatgacgg gatataggtt      60
   tccgtgcgga tggattcgtc attcccgcgc aggcgggaat ctagaacgtg gaatctaaga     120
   aaccgtttta tccgataagt tttccgtgcg gacaagtttg gattcccgcc tgcgcgggaa     180
   tgacgggatt ttaggtttct aattttggtt ttctgttttt gagggaatga cgggatgtag     240
   gttcgtagga atgacgggat ataggtttcc gtgcggatgg attcgtcatt cccgcgcagg     300
   cgggaatcca gaccttagaa caacagcaat attcaaagat tatctgaaag tccgagattc     360
   tagattcccg cctgagcggg aatgacgaaa agtggcggga atgacggtta gcgttgcctc     420
   gccttagctc aaagagaacg attctctaag gtgctgaagc actaagtgaa tcggttccgt     480
   actatttgta ctgtctgcgg cttcgtcgcc ttgtcctgat ttttgttaat ccactat         537
<210> 27
   <211> 1000
   <212> DNA
   <213> Neisseria meningitidis
<400> 27
   atacggccaa tggcttcaga aagcgataag cctctggctg aaaaaccgat ttcttgtgtt      60
   ctccccaccg cacccataga cgtaaaggta tagggattgg taatcatggt aaccacatca     120
   ccgcgacgca gcaaaatatt ttgtcgcgga tttgcaacta aatcttccaa ggcaacagtt     180
   cgtactacat tgccacgtgt cagctgcaca ttcgtatcct gcacatttgc cgttgaacca     240
   cctaccgcag ccaccgcatc caacacacgc tcaccggctg ccgtcagcgg catacgcaca     300
   ctattcccag cacgaatcac cgacacattc gccgcattat tctgcaccaa acgcaccatc     360
   acttgtggct gattggccat ttttttcagg cggcctttaa taatttcctg aacctgacca     420
   ggcgttttac cgaccaccga aatatcgcca acaaacggca cagaaaccgt accacgtgcc     480
   gtgaccaact gctctggcaa cttagtttga tgcgcactac ccgagcccat cgaagaaagg     540
   ccaccaccaa acaatactgc cggcggcgct tcccaaatca taatatccaa tacatcacca     600
   atatttagcg taccagccga agcataacca tcgccaaact gagtgaatga ctgatttatc     660
   tgagccttat ataataactg agcaaccgta tgattcacat caatcagctc cacttcagga     720
   atttgaactt cagattgttg ccctaaagag acaatttttt ttgcgctggg gcctgatgaa     780
   ggaatcgcag agcatcctac aattaaactt ccacacaata ataatactgc gtgacgaata     840
   taaaatttca ctttaaacac aagccaaatc ctaatataat tataaatggc ctaattatag     900
   cacttaatcg aaataaattt atgagtacgt agagtataat tagtattctt ctttccaact     960
   tccttatact tatatatata tacttataga ttctaaaatc                          1000
<210> 28
   <211> 1000
   <212> DNA
   <213> Neisseria meningitidis
<400> 28
   gccaaagcat tgggcgcgga tgccgccgct gccgaacgcg ccgcgcgtct tgccaaagcc      60
   gacttggtaa ccgaaatggt cggcgagttc cccgaactgc aaggcacgat gggcaaatac     120
   tatgcctgtt tggacggcga aaccgaagaa attgccgaag ccgtcgagca gcactatcag     180
   ccgcgttttg ccggcgacaa gctgcccgaa agcaaaattg ccgccgccgt ggcactggcc     240
   gacaaactag aaaccttggt cggcatttgg ggcatcggtc tgattccgac cggcgacaaa     300
   gacccctacg ccctgcgccg cgctgccttg ggtattttgc gtatgctgat gcagtatggt     360
   ttggacgtga acgaactgat tcagacggca ttcgacagct tccccaaagg tttgctcaac     420
   gaaaaaacgc cgtctgaaac cgccgacttt atgcaggcgc gccttgccgt gttgctgcaa     480
   aacgattatc cgcaagacat cgttgccgcc gtactcgcca aacagccgcg ccgtttggac     540
   gatttgaccg ccaaactgca ggccgttgcc gcgttcaaac aactgcccga agccgccgcg     600
   ctcgccgccg ccaacaaacg cgtgcaaaac ctgctgaaaa aagccgatgc cgagttgggc     660
   gcggttaacg aaagcctgtt gcaacaggac gaagaaaaag ccctctttgc cgccgcgcaa     720
   ggcttgcagc cgaaaatcgc cgccgccgtc gccgaaggca atttccaaac cgccttgtcc     780
   gaactggctt ccgtcaaacc gcaagtcgat gcattctttg acggcgtgat ggtaatggcg     840
   gaagatgccg ccgtaaaaca aaaccgcctg aacctgctga accgcttggc agagcaaatg     900
   aacgcggtag ccgacatcgc gcttttgggc gagtaaccgt tgtacagtcc aaatgccgtc     960
   tgaagccttc agacggcatc gtgcctatcg ggagaataaa                          1000
<210> 29
   <211> 1000
   <212> DNA
   <213> Neisseria meningitidis
<400> 29
   gaacgaaccg gattcccact ttcgtgggaa tgacgaattt caggttactg tttttggttt      60
   tctgtttttg tgaaaataat gggatttcag cttgtgggta tttaccggaa aaaacagaaa     120
   ccgctccgcc gtcattcccg cgcaggcggg aatctaggtc tgtcggtgcg gaaacttatc     180
   ggataaaacg gtttcttgag atttttcgtc ctggattccc actttcgtgg gaatgacgcg     240
   aacagaaacc gctccgccgt cattcccgcg caggcgggaa tctagacatt caatgctaag     300
   gcaatttatc gggaatgact gaaactcaaa aaactggatt cccactttcg tgggaatgac     360
   gtggtgcagg tttccgtatg gatggattcg tcattcccgc gcaggcggga atctagacct     420
   tcaatactaa ggcaatttat cggaaatgac tgaaactcga aaaactggat tcccactttt     480
   gtgggaatga cgcgattaga gtttcaaaat ttattctaaa tagctgaaac tcaacacact     540
   ggattcccgc ctgcgcggga atgacgaagt ggaagttacc cgaaacttaa aacaagcgaa     600
   accgaacgaa ctggattccc actttcgtgg gaatgacgga atgtaggttc gtgggaatga     660
   cggcggagcg gtttctgctt tttccaataa atgaccccaa cttaaaatcc cgtcattccc     720
   gcgcaggcgg gaatctaggt ctgtcggtgc ggaaacttat cgggtaaaac ggtttcttga     780
   gattttgcgt cctggattcc cactttcgtg ggaatgacgg aatgtaggtt cgtgggaatg     840
   acgggatata ggtttccgtg cggacgcgtt cggattcatg actgcgcggg aatgacggga     900
   ttttggtgta ttccctaaaa aaataaaaaa gtatttgcaa atttgttaaa aataaataaa     960
   ataataatcc ttatcattct ttaattgaat tggatttatt                          1000
<210> 30
   <211> 1000
   <212> DNA
   <213> Neisseria meningitidis
<400> 30
   caaaggctac gacagtgcgg aaaaccggca acatctggaa gaacatcagt tgttggacgg      60
   cattatgcgc aaagcctgcc gcaaccgtcc gctgtcggaa acgcaaacca aacgcaaccg     120
   gtatttgtcg aagacccgtt atagtggatt aaatttaaat caggacaagg cgacgaagcc     180
   gcagacagta caaatagtac ggcaaggcga ggcaacgccg tactggttta aatttaatcc     240
   actatatgtg gtcgaacaga gcttcggtac gctgcaccgt aaattccgct atgcgcgggc     300
   agcctatttc ggactgatta aagtgagtgc gcaaagccat ctgaaggcga tgtgtttgaa     360
   cctgttgaaa gccgccaaca agctaagtgc gcccgctgcc gcctaaaagg agaccggatg     420
   cctgattatc gggtatccgg ggagggttaa gggggtattt gggtaaaatt aggaggtatt     480
   tggggcgaaa atagacgaaa acctgtgttt gggtttcggc tgtcgggagg gaaaggaatt     540
   ttgcaaagat ctcatcctgt tattttcaca aaaacagaaa accaaaaaca gcaacctgaa 600
   attcgtcatt cccgcgcagg cgggaatcca gacccccaac gcggcaggaa tctatcggaa 660
   ataaccgaaa ccggacgaac ctagattccc gctttcgcgg gaatgacggc agagtggttt 720
   cagttgctcc cgataaatgc cgccatctca agtctcgtca ttcccttaaa acagaaaacc 780
   gaaatcagaa acctaaaatt tcgtcattcc cataaaaaac agaaaaccaa gtgagaataa 840
   caattcgttg taaacaaata actatttgtt aatttttatt aatatatgta aaatcccccc 900
   cccccccccc cgaaagctta agaatataat tgtaagcgta acgattattt acgttatgtt 960
   accatatccg actacaatcc aaattttgga gattttaact 1000
<210> 31
   <211> 1000
   <212> DNA
   <213> Neisseria meningitidis
<400> 31
   ataatgcagg cgctgaagtt gttaaacatc aaacacacat cgttgaagac gaaatgtctg 60
   atgaggccaa acaagtcatt ccaggcaatg cagatgtctc tatttatgaa attatggaac 120
   gttgcgccct gaatgaagaa gatgagatta aattaaaaga atacgtagag agtaagggta 180
   tgatttttat cagtactcct ttctctcgtg cagctgcttt acgattacaa cgtatggata 240
   ttccagcata taaaatcggc tctggcgaat gtaataacta cccattaatt aaactggtgg 300
   cctcttttgg taagcctatt attctctcta ccggcatgaa ttctattgaa agcatcaaaa 360
   agtcggtaga aattattcga gaagcagggg taccttatgc tttgcttcac tgtaccaaca 420
   tctacccaac cccttacgaa gatgttcgat tgggtggtat gaacgattta tctgaagcct 480
   ttccagacgc aatcattggc ctgtctgacc ataccttaga taactatgct tgcttaggag 540
   cagtagcttt aggcggttcg attttagagc gtcactttac tgaccgcatg gatcgcccag 600
   gtccggatat tgtatgctct atgaatccgg atacttttaa agagctcaag caaggcgctc 660
   atgctttaaa attggcacgc ggcggcaaaa aagacacgat tatcgcggga gaaaagccaa 720
   ctaaagattt cgcctttgca tctgtcgtag cagataaaga cattaaaaaa ggagaactgt 780
   tgtccggaga taacctatgg gttaaacgcc caggcaatgg agacttcagc gtcaacgaat 840
   atgaaacatt atttggtaag gtcgctgctt gcaatattcg caaaggtgct caaatcaaaa 900
   aaactgatat tgaataatgc ttattaactt agttacttta ttaacagagg attggctatt 960
   acatatagct aattctcatt aatttttaag agatacaata 1000
<210> 32
   <211> 1000
   <212> DNA
   <213> Neisseria meningitidis
<400> 32
   atacctgcac ttgagttgcc gaccataaat ttagcatgtt tcaataagac taaaaaatat 60
   tcaaatcgaa tggaaggaaa tgcaataaat ttatcagatt gatattttaa taattcttgc 120
   agaatacttt cagtgccagt gtcattatta gggtagatgc taatgatatt ttggccactt 180
   aattctaatg ctttgaaata ttgggccgca tattgtggca ttaaatgtgc ttctgtagtc 240
   acggggtgaa acatagaaat accataattt tcgtatggta aaccgtaata ttctttgact 300
   tcttctaagg atgggagggt ggaagaggcc ataacatcta aatcggggga gccgatgatg 360
   tgaatatgct ttcttttttc tcccatttgc actaggcgag tgacagcttg ttcatttgct 420
   accaagtgga tatgagaaag tttactaata gaatgacgaa tggagtcatc tactgtacca 480
   gatagttcac caccttcgat atggcaaact aaacggctgc ttaatgcacc tacagctgcg 540
   cctgctagtg cttctaaacg gtcgccgtga atcatgacca tatcaggttc aatttcatca 600
   gatagacgag agataaacgt aatggtattg cctaaaacgg cacccattgg ttcaccttgg 660
   atttgatttg aaaacagata tgtatgttga tagttttctc gagttacttc cttgtaggtt 720
   ctgccatatg ttttcatcat atgcatacca gttacaatca aatgcaattc aaggtctggg 780
   tgattttcaa tataggctaa taaaggtttt agcttgccga agtcggctct ggtacctgta 840
   atgcaaagaa ttcttttcat gattttagaa tctataagta tatatatata agtataagga 900
   agttggaaag aagaatacta attatactct acgtactcat aaatttattt cgattaagtg 960
   ctataattag gccatttata attatattag gatttggctt 1000
<210> 33
   <211> 1000
   <212> DNA
   <213> Neisseria meningitidis
<400> 33
   tctttttcgg actgaaagga cgcatcatcc cgacatcgag cgcgtgttcg tccggcagcc     60
   aaggcatagg ttatgcctac gaagccatca aatacggtct gaccgatatg atgctggcgg    120
   gcggaggcga agaatttttc ccgtccgaag tgtatgtttt cgactcgctt tatgccgcca    180
   gccgccgcaa cggcgaaccg gaaaaaaccc cgcgcccata cgacgcgaac cgcgacgggc    240
   tggtcatcgg cgaaggcgcg gggattttcg tgctggaaga attggaacac gccaaacggc    300
   gcggtgcgat aatttacgcc gaactcgtcg gctacggagc caacagcgat gcctaccata    360
   tttccacgcc ccgccccgac gcgcaaggcg caatccttgc ctttcagacg gcattgcaac    420
   acgcagacct tgcgcccgaa gacatcggct ggattaatct gcacggcacc gggacgcacc    480
   acaacgacag tatggaaagc cgcgccgttg cagcggtttt cggcaacaat acgccctgca    540
   cgtccaccaa gccgcaaacc ggacacacgc tgggcgcggc gggcgcaatc gaagccgcgt    600
   tcgcgtgggg cattgctgac cggaaaagca atcccgaagg gaaacttccg ccccagcttt    660
   gggacgggca gaacgatccc gaccttcccg ccatcaacct gaccggcagc ggcagccgct    720
   gggaaaccga aaaacgcatt gccgccagct cgtcgtttgc cttcggagga agcaactgcg    780
   ttttactcat cggatgaaat aagtttgtca atcccaccgc tatgctatac aatacgcgcc    840
   tactcttgat gggtctgtag ctcaggggtt agagcagggg actcataatc ccttggtcgt    900
   gggttcgagc cccaccggac ccaccaattc ccaagcccgg acgtatgttt gggctttttt    960
   gccgccctgt gaaaccaaaa tgctttgaga aaccttgata                         1000
<210> 34
   <211> 1000
   <212> DNA
   <213> Neisseria meningitidis
<400> 34
   tagaaaaata tttcgcccaa tcattagccg ccgtcgtgaa tcagacttgg cgcaacttgg     60
   agattttgat tgtcgatgac ggctcgacag acggtacgct tgccattgcc aaggattttc    120
   aaaagcggga cagccgtatc aaaatccttg cacaagctca aaattccggc ctgattccct    180
   ctttaaacat cggcctggac gaattggcaa agtcaggaat gggggaatat attgcacgca    240
   ccgatgccga cgatattgcc gcccccgact ggattgagaa aatcgtgggc gagatggaaa    300
   aagaccgcag catcatcgcg atgggcgcgt ggctggaagt tttgtcggaa gaaaaggacg    360
   gcaaccggct ggcgcggcat cacaggcacg gcaaaatttg gaaaaagccg acccggcacg    420
   aagatattgc cgactttttc cctttcggca accccataca caacaacacg atgattatga    480
   ggcgcagcgt cattgacggc ggtttgcgtt acaacaccga gcgggattgg gcggaagatt    540
   accaattttg gtacgatgtc agcaaattgg gcaggctggc ttattatccc gaagccttgg    600
   tcaaataccg ccttcacgcc aatcaggttt catccaaata cagcatccgc caacacgaaa    660
   tcgcgcaagg catccaaaaa accgccagaa acgatttttt gcagtctatg ggttttaaaa    720
   cccggttcga cagccttgaa taccgccaaa taaaagcagt agcgtatgaa ttgctggaga    780
   aacatttgcc ggaagaagat tttgaacgcg cccgccggtt tttgtaccaa tgcttcaaac    840
   ggacggacac gctgcccgcc ggcgcgtggc tggattttgc ggcagacggc aggatgcggc    900
   ggctgtttac cttgaggcaa tacttcggca ttttgcaccg attgctgaaa aaccgttgaa    960
   aaacgccgct ttatccaaca gacaaaaaac aggataaatt                         1000
<210> 35
   <211> 806
   <212> DNA
   <213> Neisseria meningitidis
<400> 35
   gcgcacggct ttttcttcat cggtttgagg gtcggcagga taatcgggga cggcaaagcc     60
   tttagactgc aattctttaa tcgcggcggt cagttgaggt acggatgcgc tgatgttcgg    120
   cagtttgatt acgtttgcat cgggctgttt caccagttcg cccaattcgg caagcgcgtc    180
   gggtacgcgc tgcgcttcgg tcagatattc ggggaatgcc gccaaaatac ggccggacag    240
   ggaaatgtcg gcagttttga catcaatatc ggcgtggcgg gcaaacgcct gcacaatcgg    300
   cagcagcgat tgggtcgcca gcgcgggggc ttcgtcggta tgggtataaa caatggtgga    360
   tttttgagtc ataggattat tctcttgtag gttggttttt tcttttggaa cacattgcgc    420
   ggggaatgtg cgcggctatt atggcatatt ttggcggctt tgttcgcgct ttgttcgatc    480
   ttggcgtgtt tgaacgcggc agcgtgaaag gaagggggaa atggttttcc cgcgtttggc    540
   ggcggtgtcg gaggtgctgt gcctgatgtg cggcggcata ttttcggtga aattgatttt    600
   atagtggttt aaatttaaac cagtacagcg ttgcctcgcc ttgtcgtact atctgtactg    660
   tctgcggctt cgttgccttg tcctgattta aatttaaacc actataatat tcggtaactg    720
   tcggaatatc tgctaaaatt ccgcattttt ccgcctcggg acactcgggg cgtatgttta    780
   atttgtcgga atggagtttt agggat                                         806
<210> 36
   <211> 1000
   <212> DNA
   <213> Neisseria meningitidis
<400> 36
   gcccgacggc gaacagacac gtcgtgaaat caaccgcttg gacagtacgg cggcgcaata     60
   cgacatgctt gcaggttatc ttgaaagact tgccggaaaa accgaccgtt gggcgtgcgc    120
   ctaccgccaa aatgccgtct gaacacccga ttatcctttt gaaagcgcga ttatgcccca    180
   tacccttccc gatatttccc aatgtatcag acaaaatttg gaacaatatt tcaaagacct    240
   gaacggtacc gaaccttgcg gcgtgtacga tatggtcttg catcaggtgg aaaaaccgct    300
   gctggtgtgc gtgatggaac aatgcggcgg caaccagtcc aaagcctccg tcatgttggg    360
   actgaaccgc aatactttgc gtaaaaaact gattcaacac ggtttgctgt gaatatgtcg    420
   gcaaccgtcc gtatcttggg tattgacccg ggcagtcgcg taacgggttt cggtgtcatc    480
   gatgtcaggg ggcgcgatca tttttacgtc gcctccggct gcatcaaaac gcctgccgat    540
   gcgcctctgg cagacaggat tgccgtgatt gtgcggcata tcggcgaagt cgttaccgtt    600
   tacaagcctc aacaggcggc agtggaacag gtgttcgtca acgtcaatcc ggcatcgacg    660
   ctgatgctcg gtcaggctag gggcgcggca ttggcggcat tggtcagcca taagctgccc    720
   gtttcggaat acacggcctt gcaggtcaaa caggcggtag tcggcaaggg caaggcggca    780
   aaagaacagg tgcagcatat ggtggtgcag atgctggggc tttcgggaac gccgcaggan    840
   tggcggcgga cggtcttgcc gtcgcgctga cccacgcctt acgcaaccac gggcttgccg    900
   ccaaactcaa tccttcgggg atgcaggtca agcgcggcag gtttcaatag tttcagacgg    960
   catttgtatt ttgccgtctg aaaagaaaat gtgtatcgag                          1000
<210> 37
   <211> 1000
   <212> DNA
   <213> Neisseria meningitidis
<400> 37
   ccgccaagcg tttccccctt tgtcgggctt aacatttgct ttgtacggca gactttttcc 60
   cttcataacg ccgcctttcc gaaaagacga tggtaggcgc gacgtaattc tcaaccctta 120
   aggtacggtt ggacgaaaag ttttcctttt cattccacct gccaactttt cggctacacc 180
   gagtggtctc gttaggtttg ggcgaactac gcccttaaaa aaacggacat tctttgcatg 240
   cccgtctcta aggtttcacg gtaagtttac ccttataaag agttgactta ccatacttat 300
   ccctttaaaa cgatataaag ggcgacagct gtaatacaag tatgttgtac ggcagacttc 360
   ttctaccaaa caaaaagttc cttttagagt tactcgctta tagacaaatg aaggcttagc 420
   cataggcttc cggtaggcct atttcaacgg ctggttcaca ggctacgcta aaacctacgg 480
   tagaaccgcg ttctggggtt tcgcgcacag cggcgtcttt ggaaccagtt gtgtccgaac 540
   acgcataacc gcccgcttta atggtggtgg cgggttcacc tgatgtagtt tcagcgtgcg 600
   ctttggtagt ttgcgtagcc gatgttgagg aggctcgacc cgaaactacg gttgccgacg 660
   cgccagccgc acatgatgct ggtcgttaga ggcctgtagc gggttccgca cttgcttccg 720
   cttccgtaac tgaacttggt tccgcgaccg ctggttccaa actacaagcc gatacggacg 780
   ctgctttggg gctgggacta cggcaaacgg tagataatgt cggtggcgga ctacgtcgca 840
   gtttcgctta atgcgtttct gccggaggac ggaaccgacg cagggctgcg ttttcgggtt 900
   gactggcacc aaatgctatc gcttaggccg tttcattttg cgtaactatg gcagcaggag 960
   agatacgttg tgctgggcct ttagccaata cttctcaact 1000
<210> 38
   <211> 1000
   <212> DNA
   <213> Neisseria meningitidis
<400> 38
   cacaaaaacc aagttatgac gggaataagg tacagcagcc aaaccaaggc ctcgccctgc 60
   gtcggatggt cggtatagcc gaaaaatccg ccgagcagca cgcccaacgg gctgtcttcg 120
   tgcaaatatt ttgatgagtc gaacacaatg tcctgaagcg cgttccaaat gcctgcttcg 180
   tgcagcgcac gcagcgaacc ggcaagcaga ccagcggcaa cgataatcag aaacgcccct 240
   gtccaacgga aaaacttcgc cagattcagg cgcatcccac cctgataaat caacgcgcca 300
   atcacggcgg cagccaaaac ccccgctacc gcaccggccg gcatctgcca cgtcgggctc 360
   tgtttgaata cggcaagcag gaaaaaaacg ctctccaaac cttcgcgcgc cacggcaaga 420
   aacgccatac cgaccaaggc ccatccttga ccgctgccac ggttcaaagc cgcctgcaca 480
   gaatcctgaa gctgccgctt catcgaacgg gcggcttttt tcatccataa aatcatataa 540
   gtcagcatcg cgacagcaac caaaccgata atgccgacga cgaactcctg ctgcttctgg 600
   ggaatctcgc ccgttgccga atggattccg taccccagcc ccaaacacat caaagaagca 660
   agaacaaccc cgaaccagac cttaggcatc agtttggaat gtccggactg tttcagaaaa 720
   ccggcaacga tgccgacgat gagcgcggct tcgataccct cgcgcaacat aattaaaaaa 780
   gcgaccagca taaacgcgaa cgaacaagga tgatgaataa tatattatcg gaatattttc 840
   attgcttgta aatacaaatg caagttattt ttatctgcag taccgcgcgg cggaaagttc 900
   cgcagctgca gctgcgccct gtgttaaaat cccctctcca cggctgccgc aacgccgccc 960
   gaaaccatct ttcttattac tgccggcaac attgtccatt 1000
<210> 39
   <211> 1000
   <212> DNA
   <213> Moraxella catarrhalis
<400> 39
   gctgatttgt gagcaagcgg gcgcatcagg gattaccttg catttgcgag aagatcgtcg      60
   acatattcaa gatgaagatg tttatgaatt gattgggcaa ttgacaacac gcatgaatct     120
   tgagatggca gtcactgatg agatgctaaa tattgcccta aaggtacgac cagcatgggt     180
   gtgtttagta ccagaaaaac gccaagagct gactacagaa ggtgggcttg atatcgccaa     240
   tttatcaaat attcaagcat ttatacacag tcttcagcag gcggatatta aggtttcttt     300
   attcatcgat ccagatccgc atcaaattga tgctgcaatt gctttgggtg ctgatgcgat     360
   tgagctgcat acgggagctt atgctcaagc gactttacaa aataatcaaa agcttgttga     420
   taaagagctt gaccgtattc aaaaagccgt tgcaatggca caaaaaaaat catcattatt     480
   gattaatgca ggtcatggtt tgacgcgtga taatgttgca gcgattgccc aaattgatgg     540
   tattcatgag ctgaatatcg ggcatgcatt gatttcagat gcgatattta tggggcttga     600
   taatgcagtc aaggcaatga aaatggcttt tattcaagat aaaacgacca atcattgatg     660
   cgttagaaag aaaatcgtaa ataatgatga ctattgtgta atattatgta tttttgttca     720
   aaaaaaggtt gtaaaaaaat tcatttacca ttaagctaag cccacaagcc acaatgaata     780
   cctattggtt tgactcatta gtcactaaga atctgcaaaa ttttgtaaca gattattggc     840
   aggtcttgga tcgctatgct aaaataggtg cggtaatctt gaaaaaccaa ccattccttg     900
   gaggaattta tgaaaaaggg atataaacgc tcttgcggtc atcgcagccg ttgcagctcc     960
   agttgcagct ccagttgctg ctcaagctgg tgtgacagtc                          1000
<210> 40
   <211> 1000
   <212> DNA
   <213> Moraxella catarrhalis
<400> 40
   gatgctgtta aagtgggtat tggtcctggt tctatttgta caacccgtat tgttgcaggc      60
   attggcgtcc cgcagataag tgccattgat agtgtggcaa gtgcgttaaa agatcgcatt     120
   cctttgattg ccgatggcgg tattcgtttt tcgggtgata tcgccaaagc catcgcagca     180
   ggcgcttcat gtattatggt gggtagcttg ttggcaggta ccgaagaagc acctggtgag     240
   gtggaattat tccaaggtcg ttattataag gcttatcgtg gtatgggcag cttgggggca     300
   atgtctggtc aaaatggctc atcggatcgt tattttcaag atgccaaaga tggtgttgaa     360
   aaactggttc cagagggtat cgaaggccgt gttccttata aaggccctgt ggcaggcatc     420
   atcggtcaat tggcaggtgg tctaagatca tccatgggtt atacaggttg ccagaccatc     480
   gaacagatgc gtaagaatac cagctttgtc aaagtgactt ccgcaggcat gaaggaatcg     540
   catgtacacg atgtacagat taccaaagaa gcacccaatt atcgccaaaa ttaactctat     600
   taatagcaaa tacaagcact cattagatag ggtgggtgct ttttagagca taaaaaataa     660
   actgacacat gacttattgt catattttta aaatgctttt aatttagatt tttaatttag     720
   ataatggcta aaaataacag aatattaatt taaagttttc aaaatcaagc gattagatga     780
   aattatgaaa ataaataaca ataattctga tttattttaa ccaataatat caattatcat     840
   ttacaagaaa aatttttttt gataaaattc ttacttgtac cttgctattt tttcttattt     900
   atcatttttg gcggtatttt cgttgatttt agtaagtaga tgagcaaggg ataatttgac     960
   aaaaacaaat ttgatttcaa gcctcataat cggagttatt                          1000
<210> 41
   <211> 1000
   <212> DNA
   <213> Moraxella catarrhalis
<400> 41
   aaaactggtg atgtcttcac tgctattcat ggtgaaccaa tcaatgattg gctaagtgcc      60
   accaagatta ttcaggcaaa tccagaaacc atgcttgatg tgacagtcat gcgtcaaggt     120
   aagcaggttg atttaaaatt aatgccccgt ggtgtaaaga cacaaaacgg cgtagtcggt     180
   caactgggta ttcgccccca gattgatatc gatacgctca ttcctgatga atatcgtatg     240
   acgattcaat atgatgtcgg tgaggcattt actcaagcca tccgacgaac ttatgattta     300
   tcaataatga ccttagatgc gatgggtaag atgattacag gattgattgg cattgaaaat     360
   ctatcaggtc ccattgccat tgccgatgtt tctaagacca gttttgagtt gggatttcaa     420
   gaagtgttat cgacagccgc aatcatcagt ttaagcttgg cagtactgaa tcttttaccc     480
   attccagtgt tagatggcgg gcatttggta ttttatactt atgaatggat tatgggcaaa     540
   tctatgaatg aagcggtgca gatggcagca tttaaagcgg gtgcgttatt gcttttttgt     600
   ttcatgttac ttgcaatcag taacgatatc atgcgatttt ttggctaagt tctgatttat     660
   cgtaccatta acaaaatttt tggctttttt aagctgaaat acttgccaaa tttaactttt     720
   tggcttacct ttacacaata taaatttggg tgtagaaaat tttggataca tttttatacc     780
   ttatttttag aaattttaaa aattaagttt ggatagactt atgcgtaatt catattttaa     840
   aggttttcag gtcagtgcaa tgacaatggc tgtcatgatg gtaatgtcaa ctcatgcaca     900
   agcggcggat tttatggcaa atgacattgc catcacagga ctacagcgag tgaccattga     960
   aagcttacaa agcgtgctgc cgtttcgctt gggtcaagtg                         1000
<210> 42
   <211> 1000
   <212> DNA .
   <213> Moraxella catarrhalis
<400> 42
   acttggcgaa aataccattt atatcgattg tgatgttata caggcagatg gcggtacacg      60
   cacagccagt atcagtggtg ctgcggtggc acttattgat gctttagaac acttgcagcg     120
   tcgtaaaaag cttacccaag atccgctttt gggcttggtg gcagcggttt ctgtgggtgt     180
   taatcaaggc cgtgtattgc ttgatttgga ttatgctgaa gattcaactt gtgataccga     240
   tttaaatgtg gtcatgacgc aggcaggtgg gtttattgag attcaaggca cagcagaaga     300
   aaagccattt actcgtgctg aagctaatgc gatgcttgat ttggcagagc tgggaattgg     360
   gcagattatc gaagcccaaa agcaagtatt aggctggtga tatgctaatc gttgaagata     420
   atggcgtgat catcacatta aatggacaag taaaagaccc attattttgg tggtcgatga     480
   tattgctgct gctgggtgtc ttggtggcaa tcatttgttt gattgcaccc gttttttatg     540
   caatcggtgc gttggcttta tttgcagttg tggtatttgt gtttaatatt caaaggcaaa     600
   aagccaaaac ttgtcatatg ttttcacaag gtcgcttgaa gattacgtcc aaacgctttg     660
   agattcataa caaatcacta accttatcag catcggcaac aatatctgct aaagataaca     720
   aaatgacaat tgttgatcgg ggcattgaat atcattttac aggttttgct gatgaccgtg     780
   aaattaatat agccaaacag gtacttttgg gaaagtcaat caaaaccaat gcggtggcgg     840
   taacattggc taagtagttg ttgtgataca gacaggttgg atggtcttta actccaccca     900
   cctaactttt tctttgtttg gatttaagag tatgttatga tgggcaggat tttattttaa     960
   gtcatcattt aatgcaatca gttgtccaga gtagccgttc                         1000
<210> 43
   <211> 1000
   <212> DNA
   <213> Moraxella catarrhalis
<400> 43
   gtgatcggca acaccccacc attcaggagc aaccaaaatt gcccgtgcct tgcctgtctt      60
   ggtggtatca tttggcaggg caatgtggct aagtagtggt gtgccatcag gtgcggtggt     120
   ggtgagtgta cgattcgtta ttgtcataaa attatccttt tgggttggat gatatcaatg     180
   aaatacccta cggttgtatg gaattttatc cattgtacca cggtattggt ctttttaaat     240
   taacaagcag cttctagcaa gtcaaagttt ttatgcctat tttttcagat tttaaggtac     300
   aataaagcca attgttaata atatggtatt gtcatgattt atgatgaatt gcgaccaaaa     360
   ttttgggaaa attatccctt agatgcgtta acagatgctg aatgggaagc attatgtgac     420
   ggatgtggcg cgtgttgttt ggtgaaattt cttgatgatg acaatgttaa attgaccgaa     480
   tataccgatg ttgcctgcca gctattggat tgctcaacag gattttgcca aaactatgcc     540
   aagcgtcaaa cgattgtgcc agattgtatt cgcttaacac ctgatatgct gcctgatatg     600
   ctgtggttgc cacgccattg tgcttataag cggttgtatc ttgggcaaaa tctgccagca     660
   tggcacaggc tcattaaaca tagccaaaac catggtgcag gatttgcgaa agtttcaact     720
   gctgggcgat gtgtgagtga gcttggtatg agtgatgaag acatagaaag gcgagtggtg     780
   aaatgggtta aaccttgaca tgattgttga catgattgac agacaataaa aattggcaaa     840
   tttgataaaa ttggtgtatg tgtgtgattt tatcaaaagc acttgaataa aaccgagtga     900
   tacgctaaat tgtagcaaac caatcaattc atcataattt taatgaacac gaggttaaat     960
   tatactgtct atgtctgatg acaattcaag cacttggtcg                         1000

<210> 44
   <211> 1000
   <212> DNA
   <213> Moraxella catarrhalis
<400> 44
   taacaaaggc aacccaacac gcagttattt tgtgcaaggc ggtcaagcgg atgtcagtac     60
   tcagctgccc agtgcaggta aattcaccta taatggtctt tgggcaggct acctgaccca    120
   gaaaaaagac aaaggttata gcaaagatga ggataccatc aagcaaaaag gtcttaaaga    180
   ttatatattg accaaagact ttatcccaca agatgacgat gacgatgacg atgacgatag    240
   tttgaccgca tctgatgatt cacaagatga taatacacat ggcgatgatg atttgattgc    300
   atctgatgat tcacaagatg atgacgcaga tggcgatgac gattcagatg atttgggtga    360
   tggtgcagat gatgacgccg caggcaaagt gtatcatgca ggtaatattc gccctgaatt    420
   tgaaaacaaa tacttgccca ttaatgagcc tactcatgaa aaaacctttg ccctagatgg    480
   taaaaataag gctaagtttg atgtaaactt tgacaccaac agcctaactg gtaaattaaa    540
   cgatgagaga ggtgatatcg tctttgatat caaaaatggc aaaattgatg gcacaggatt    600
   taccgccaaa gccgatgtgc caaactatcg tgaagaagtg ggtaacaacc aaggtggcgg    660
   tttcttatac aacatcaaag atattgatgt taaggggcaa ttttttggca caaatggcga     720
   agagttggca ggacggttac atcatgacaa aggcgatggc atcactgaca ccgccgaaaa    780
   agcaggggct gtctttgggg ctgttaaaga taaataaagc ccccctcatc atcgtttagt    840
   cgcttgaccg acagttgatg acgcccttgg caatgtctta aaacagcact ttgaaacagt    900
   gccttgggcg aattcttgga taaatgcacc agatttgcct cgggctaata tcttgataaa    960
   acatcgccat aaaatagaaa ataaagttta ggattttttt                         1000
<210> 45
   <211> 1000
   <212> DNA
   <213> Moraxella catarrhalis
<400> 45
   cagcttgtac catttggtga atatatacca tttggtggtt tgttggatat tttaccaggg     60
   cttgagggtg tcgctagcct aagccgtggc gatgataagc aaccaccgct caaattgggc    120
   ggcggcgtgg gcgatacgat tggtgcggca atttgttatg aggtggcata tcctgagacg    180
   acgcgtaaaa atgcacttgg cagtaatttt ttattaaccg tctcaaacga tgcttggttt    240
   ggtacaacag caggtccttt gcagcattta caaatggtgc aaatgcgaag cttggagacg    300
   gggcgatggt ttgtgcgtgc aacaaacaac ggagtgactg cattaattga ccatcaagga    360
   cggattatca agcagatacc gcagtttcag cgagatattt tgcgaggtga tgtacccagt    420
   tatgttggac acacgcctta tatggtttgg gggcattatc ccatgttggg gttttctttg    480
   gtgctgattt ttcttagtat catggcaaag aaaatgaaaa ataccaccgc caaacgagaa    540
   aaattttata ccgctgatgg tgtggtagac cgctgaattg tgccactttg ggcgttagag    600
   catgagcaag attaggcgtt gggtgagctt tggttgtatt actcatcagc ctacccgaaa    660
   cctgccaaac atcaccgccc aaaacctaaa catacaatgg ctaaaaatat cagaaaataa    720
   cttgctgtat tgtaaattct tatgttatca tgtgataata attatcatta gtaccaagat    780
   atccattact aaacttcatc ccccatctta acagttacca agcggtgagc ggattatccg    840
   attgacagca agcttagcat gatggcatcg gctgattgtc tttttgcctt gttgtgtgtt    900
   tgtgggagtt gattgtactt accttagtgg tggatgcttg ggctgattta attaaatttg    960
   atcaaagcgg tcttcacaac acaccaaacg agatatcacc                         1000
<210> 46
   <211> 1000
   <212> DNA
   <213> Moraxella catarrhalis
<400> 46
   agtttgccct gattttgaga gccactgcca tcatgaattt gttggcgtaa acaccactcg      60
   tattcttctt cggtttcccc tttccatgca aacacaggga taccagcggc cgccatggca     120
   gcggcggcgt ggtcttgggt gctaaaaata ttgcatgatg tccagcgaac ttctgcaccc     180
   aaggcaacca aagtctcaat cagcaccgct gtttgaatgg tcatgtggat acagcctagg     240
   attttagcac ccttaagtgg ttgctggtct tgatagcgtt ttcttaaccc catcagggct     300
   ggcatctcag cttctgccaa ggcaatctca cggcgaccat aatcggctaa acggatatca     360
   gcgactttat aatcggtgaa gttttgggtg gtacttggat tgattgaggt aggcatatct     420
   ttattcctaa gctattttaa agtattttta acaataattt tgatgaattt gagataattg     480
   atgctaaaag gttgaatgac caaaccatcg ctaacaatca agaaaagaca ttttaagcat     540
   aaaaagcaaa tgtgtcttga tggcttatta taacagttat tatgataaat ttgggtagaa     600
   agttaaatgg atcgttgggt aagtttgttg gctatcctta attaattata attttttaat      660
   aatgctttta ctttatttta aaaatagagt aaaaaatggt tggctttggg tttttatctc     720
   actatggtag ataaaattga tacaaaatgg tttgtattat cacttgtatt tgtattataa     780
   ttttacttat ttttacaaac tatacactaa aatcaaaaat taatcacttt ggttgggtgg     840
   ttttagcaag caaatggtta ttttggtaaa caattaagtt cttaaaaacg atacacgctc     900
   ataaacagat ggtttttggc atctgcaatt tgatgcctgc cttgtgattg gttggggtgt     960
   atcggtgtat caaagtgcaa aagccaacag gtggtcattg                         1000
<210> 47
   <211> 1000
   <212> DNA
   <213> Moraxella catarrhalis
<400> 47
   ttgggggcgg ataaaaagtg gtctttgccc aaaggggcat atgtgggagc gaacacccaa      60
   atctatggca aacatcatca aaatcacaaa aaatacaacg accattgggg cagactgggg     120
   gcaaatttgg gctttgctga tgccaaaaaa gaccttagca ttgagaccta tggtgaaaaa     180
   agattttatg ggcatgagcg ttataccgac accatcggca tacgcatgtc ggttgattat     240
   agaatcaacc caaaatttca aagcctaaac gccatagaca tatcacgcct aaccaaccat     300
   cggacgccca gggctgacag taataacact ttatacagca catcattgat ttattaccca     360
   aatgccacac gctattatct tttgggggca gacttttatg atgaaaaagt gccacaagac     420
   ccatctgaca gctatgagcg tcgtggcata cgcacagcgt gggggcaaga atgggcgggt     480
   ggtctttcaa gccgtgccca aatcagcatc aacaaacgcc attaccaagg ggcaaaccta     540
   accagtggcg gacaaattcg ccatgataaa cagatgcaag cgtctttatc gctttggcac     600
   agagacattc acaaatgggg catcacgcca cggctgacca tcagtacaaa catcaataaa     660
   agcaatgaca tcaaggcaaa ttatcacaaa aatcaaatgt ttgttgagtt tagtcgcatt     720
   ttttgatggg ataagcacgc cctacttttg tttttgtaaa aaaatgtgcc atcatagaca     780
   atatcaagaa aaaatcaaga aaaaaagatt acaaatttaa tgataattgt tattgtttat     840
   gttattattt atcaatgtaa atttgccgta ttttgtccat cacaaacgca tttatcatca     900
   atgcccagac aaatacgcca aatgcacatt gtcaacatgc caaaataggc attaacagac     960
   ttttttagat aataccatca acccatcaga ggattatttt                           1000
<210> 48
   <211> 1000
   <212> DNA
   <213> Moraxella catarrhalis
<400> 48
   aaagacatta cacatcatca ttcaaacgcc caaccatgta cctctgcccc gtggtcgcac      60
   gccaacgctt tttgatgcgg tgcgttgggt tcagatggct tgtcaatcat ttggttttat     120
   taaaattcat acctttggta gtttggcttt acctgatatg tcatttgatt atcgaaacaa     180
   tacgcagttg accaaacatc aatttttagc catttgccaa gcactcaata ttaccgctca     240
   tacgaccatg cttggtatta aatcatcaca taaagatact ttacatccat ttgaattgac     300
   attacccaaa tacggccatg cctcaaatta tgatgatgaa ttggtgcaaa acaatccatt     360
   ggcttatttt catcaactgt ctgccgtctg ccgatatttt tatacccaaa cggtttgtat     420
   tgttggcggt gaaagctcag ggaaaactac cttggtgcaa aaacttgcca attattatgg     480
   tgccagcatc gcacctgaaa tgggtcgatt atacacacac tcccatctcg gcggtagcga     540
   acttgccctt caatacagcg actacgcatc cattgccatc aatcacgcca acgctatcga     600
   aaccgctcgt accactgcca gctctgctgt tacactgatt gatactgatt ttgcgacaac     660
   gcaagcattt tgtgaaattt atgaagggcg aacgcatccg cttgtcgcag aatttgctaa     720
   acaaatgcga ttggatttta cgatttattt agataataat gttgcttggg tcgctgatgg     780
   catgcgtagg cttggtgatg atcatcaacg cagtttgttc gccaataaat tgcttgagat     840
   tttggcacga tatgatatta gttatcatat cattaatgac accgactacc acaaacgcta     900
   tctacaagca ttaagcttga tagacaatca tatttttaat cattttacaa aaattcatga     960
   caattaatta gggaaaatct gatgaaaatt gatattttag                          1000
<210> 49
   <211> 1000
   <212> DNA
   <213> Moraxella catarrhalis
<400> 49
   ggatgtggca tatctgccca tcgacccaat acacatcggt cgaggctatc aagatgtggt      60
   acgaattaat agccagtcag gtaagggcgg tgctgcgtat atcttgcagc ggcattttgg     120
   ttttaattta ccacgctgga cacagattga ttttgctcgt gtggtacagg cttatgcaga     180
   aagtatggcg cgtgaactaa aaactgatga gctgcttgaa atttttaccc aagcgtatct     240
   taagcaagat aaattccgcc taagtgacta taccatcagc aataaaggcg atgctgtcag     300
   cttccaaggc caagtagcga cacccaaagc ggtgtttgag gtgattggtc aaggcaatgg     360
   tgcgttatct gcgttcattg atggcttggt gaaatccaca ggcagacaga ttcatgtcac     420
   caattacgcc gaacacgcca tcgataacaa aacccatcaa aaaaccgata cggataacca     480
   aaccgatgcc gccgtgccgc ttatatccag ctgtcggtag aggggcagat ttattcaggc     540
   atcgccactt gccatagcac cgtatccgcc atgctaaaag gtgcattatc cgctttggca     600
   caggcgtggt aatctgaccc aatcaaaatc ctgcatgatg gcaggatttt attatttagt     660
   gggctgccca acaatgatga tcatcagcat gtgagcaaat gactggcgta aatgactgat     720
   gagtgtctat ttaatgaaag atatcaatat ataaaagttg actatagcga tgcaatacag     780
   taaaatttgt tacggctaaa cataacgacg gtccaagatg gcggatatcg ccatttacca     840
   acctgataat cagtttgata gccattagcg atggcatcaa gttgtgttgt tgtattgtca     900
   tataaacggt aaatttggtt tggtggatgc cccatctgat ttaccgtccc cctaataagt     960
   gagggggggg gagaccccag tcatttatta ggagactaag                          1000
<210> 50
   <211> 1000
   <212> DNA
   <213> Moraxella catarrhalis
<400> 50
   ccccaagctt tccgtttgtg tgcctgctgg tgtcgggcgg tcataccatg ctggtgcgtg      60
   ccgatggtgt gggcgtgtat cagatattgg gcgagtctat cgatgatgcg gtgggtgaat     120
   gctttgataa aacggcaaaa atgctcaaac tgccctatcc tggtggccca aatatcgaaa     180
   aattagccaa aaacggcaac ccacacgcct atgagctgcc aagacccatg cagcataaag     240
   ggctggattt ttcgttcagt ggcatgaaaa ccgccattca taatctcatc aaagacacac     300
   caaacgccca aagcgacccc gccacacgag cagacatcgc cgcaagcttt gagtatgcgg     360
   tggtggatac tttggtcaaa aaatgcacca aagcactaca gatgacaggc attcgccagc     420
   tggtggtcgc agggggcgtc tctgccaatc agatgctacg ccgcaccctg accgagacgc     480
   tccgccaaat cgatgcgtcg gtgtactatg ccccgaccga gctatgcacg gataatggtg     540
   cgatgatcgc ctatgctggc ttttgtcggc tcagctgtgg acagtcggat gacttggcgg     600
   ttcgctgtat tccccgatgg gatatgacga cgcttggcgt atcggctcat agatagccac     660
   atcaatcata ccaaccaaat cgtacaaacg gttgatacat gccaaaaata ccatattgaa     720
   agtagggttt gggtattatt tatgtaactt atatctaatt tggtgttgat actttgataa     780
   agccttgcta tactgtaacc taaatggata tgatagagat ttttccattt atgccagcaa     840
   aagagataga tagatagata gatagataga actctgtctt ttatctgtcc gctgatgctt     900
   tctgcctgcc accgatgata tcatttatct gctttttagg catcagttat ttcaccgtga     960
   tgactgatgt gatgacttaa ccaccaaaag agagtgctaa                         1000
<210> 51
   <211> 1000
   <212> DNA
   <213> Moraxella catarrhalis
<400> 51
   gagtgaactt tattgtaaaa tatgattcat taaagtatca aaatcatcaa acgcagcatc      60
   agggtttgct aaatcaattt tttcaccata attatagcca taacgcacag caagcgtagt     120
   tatgccagcg gcttgccctg ataaaatatc atttttggaa tcaccaacca taatggcatc     180
   agtcggtgcg atgcccagtg attgacacag gtataataaa ggcgttgggt cgggcttttt     240
   gacgctgagc gtatcaccgc caatcacttg gtcaaacagt gtcagccatc caaaatgtga     300
   taaaatttta ggcaaataac gctcaggctt attggtacaa attgccaaat aaaaccccgc     360
   tgcttttaat cgttcaagcc cttgtataac ccctgcatag ctttgcgtat tttcaattgt     420
   tttatgggca tattctgcca aaaataactc atgggcatgg tgaatcatag tcgtatcata     480
   gatatgatgt gcttgcattg ctcgctcaac caattttagc gaaccattgc ccacccagct     540
   tttgatgata tcaattggca taggcggtaa gttaagcttg gcatacatgc cattgaccgc     600
   cgccgccaaa tcaggggcac tatcgataag cgtaccatcc aaatcaaata taatcagttt     660
   tttgccagtc attgacagtg tttgcatgct ttttccttat tcttaaaatt ggcggctgtt     720
   tggtattttt taaatcagtc aatttttacc atttgtcata taatgacaaa gtacaaattt     780
   agcaatattt tagtgcattt tttggcgaag ttttatgaaa actggtcatt ggttgcaaaa     840
   ctttacacag tacctataaa acttgcacag ttaataagaa atattttgtt actatagggg     900
   cgtcatttgg aacaagacag ttatttgtaa atagttattt gcaaaagacg gctaaaagac     960
   agaacagcgt ttgtttcagt gattaactag gagaaaaaca                         1000
<210> 52
   <211> 1000
   <212> DNA
   <213> Moraxella catarrhalis
<400> 52
   ttgatcggtt ttgccccact gtttcatgat ttactcaaaa caggcggctt gatcgtgctg 60
   gcaggtctga cccaaaacca aacccaagcg gtcatcgatg cctactcgcc ttatgttacg 120
   cttgatacgc cattttgtta tgcagatgcc caagactgcc attggcaacg cctaagcggc 180
   atcaaaccta ccaacccata agcgatatgc catgagccac aaacctaagc caacaccgct 240
   atatcaacaa gttgagcaga ccgccaagcg ttattttgag acattgggcg atgctcatac 300
   tcatgatgtc tatgccactt ttttggccga atttgaaaaa ccgctgctca tcgccgcact 360
   caatcacacg cacggcaatc agtcaaaaac cgcccaaatc cttggtatca atcgtggcac 420
   attacgcacc aaaatgaaaa cccatcactt actttagacc gccagttatc gccatggata 480
   tgggcaggtg tgctcgcctg ccgtatgatg gcgatgacac cccatttgcc ccatatctgc 540
   acgatttgac atgatttaac atgtgatatg atttaacatg tgacatgatt taacattgtt 600
   taatactgtt gccatcatta ccataattta gtaacgcatt tgtaaaaatc attgccccct 660
   ttttttatgt gtatcatatg aatagaatat tatgattgta tctgattatt gtatcagaat 720
   ggtgatgcct acgagttgat ttgggttaat cactctatta tttgatatgt tttgaaacta 780
   atctattgac ttaaatcacc atatggttat aatttagcat aatggtaggc tttttgtaaa 840
   aatcacatcg caatattgtt ctactgttac caccatgctt gaatgacgat ccaaatcacc 900
   agattcattc aagtgatgtg tttgtatacg caccatttac cctaattatt tcaatcaaat 960
   gcctatgtca gcatgtatca tttttttaag gtaaaccacc 1000
<210> 53
   <211> 1001
   <212> DNA
   <213> Moraxella catarrhalis
<400> 53
   actattctgc tttttgtttt tcacgaatgc gaatgcccaa ctcacgcaac tggcgattat 60
   caacttcagc aggtgcttcg gtcaatgggc aatctgccgt cttggttttt gggaaggcga 120
   tcacatcacg gattgagctg gcaccaacca tcagcataat caggcgatct agaccaaatg 180
   ccaaaccacc gtgcggcggt gcaccaaaac gcaatgcatc catcaaaaac ttaaacttaa 240
   gctctgcttc ttctttagaa atacccaagg catcaaatac cgcctcttgc atgtcaaccg 300
   tattaatacg cagcgaaccg ccaccaattt ctgtgccatt tagtaccatg tcataggcaa 360
   tggatagggc ggtttcggga ctttgtttga gttcctcaac cgagcctttt gggcgtgtaa 420
   aaggatgatg aactgatgtc cacttaccat catcagtttc ctcaaacatt ggaaaatcaa 480
   cgacccaaag cggtgcccat tcacaggtaa ataaatttaa atcagtaccg attttaacac 540
   gcaatgcacc catagcatca ttgacgattt tggctttatc ggcaccaaag aaaatgatat 600
   cgccagtttg ggcatcggta cgctcaatca gctcaatcaa aacctcatcg gtcatatttt 660
   taatgatggg tgattgtaat cctgattctt tttcaacgcc attattgata ttgcttgcgt 720
   cattgacctt aatatatgcc aatccacgag cgccataaat accaacaaat ttggtgtact 780
   catcaatctg cttgcgactc atgttaccgc catttggaat gcgtaaggca acaacacggc 840
   ctttaggatc ttgggcgggc cctgaaaata ctttaaattc aacatgttgc atgatgtcag 900
   caacatcaat aagttttaag ggaatgcgta aatcaggctt atctgaggca taatcacgca 960
   tggcatctgc gtaagtcatg cgggggaagg tatcaaactc a 1001
<210> 54
   <211> 1001
   <212> DNA
   <213> Moraxella catarrhalis
<400> 54
   tggatcatat tctttattaa tggtactgtt taaacctgta ttttaaagtt tattgggtca 60
   tattttcaag ctcatcccat cgctcaagct tcatcatcaa aagctcatca atctctacca 120
   atcgctcacc agccttcgtt gctgccgcca aatcggtatt aaaccatgaa ccatcttcaa 180
   tctttttggc aagctgtgcc tgctcttgtt caagtgcagc aatttcatta ggcaaatctt 240
   caagttcacg ctgctcttta tagctgagtt tgcgtttttg ggcaacgcct gattgaggtg 300
   gtttgatttg gatgggttca gcgggttttg tcgccttagg tttattgtct gtggcgtgat 360
   gagcaagcca tctttcatgc tgttgtacat agtcttcata accgccaaca tattccaaaa 420
   cgataccgtc gccgtactta tcagtatcaa atacccaagt ttgggtaaca acattatcca 480
   taaaagcacg gtcatggctg atgagtaata ccgtgccttt aaaattgacc acaaaatctt 540
   ctaaaagctc aagtgttgcc atatccaaat cattggtagg ctcatcaagc accaaaacat 600
   tggcaggttt tagcaataat ttggccaata aaacgcgtgc tttttcaccg cctgatagtg 660
   ctttaacagg tgtgcgagca cgatttggcg tgaataaaaa atcttgcaaa tagcttaaaa 720
   tgtgcgtagt ttttccacca acatcgacat ggtcagagcc ttctgaaaca ttatctgcga 780
   tagatttttc agggtctagg tcgtctttga gttggtcaaa aaaagcaata tttagattgg 840
   tgccaagctt aactgaacct gactgaatcg ctgaatcatc caaacccaaa atgcttttaa 900
   ttaaggttgt tttaccaacg ccatttttgc caatgatacc aactttatca ccacgaacaa 960
   gcagcgttga aaaatcctta actaaggttt tattgtcgta t 1001
<210> 55
   <211> 1000
   <212> DNA
   <213> Moraxella catarrhalis
<400> 55
   caacttgaaa atcagctcaa tgctctgcca cgcacagcac cgatgagcga gattatcgga 60
   atgataaata ccaaagcaca agcggttaat gtgcaggtgg tgagtgcatc agttcaagca 120
   ggtcgtgaac aggattatta taccgaacgc cctatcgcag tgagtgcgac aggggattat 180
   catgctttgg gtcgatggtt acttgagttg tcagaggcta accatttgct gacagtgcat 240
   gattttgatc tgaaggctgg tttgaaccat cagctgatga tgattgttca gatgaaaact 300
   tatcaagcga acaaacgccc aaaaccagtt gctcagcagg tgcctgatgt tcaatgaata 360
   ttatcggtgg ggcattttgg gtgcttggat ttgggttggg attggatgtg ctgatagcac 420
   cagtcaagtt gttgatgata agcttgcaca tattacccat gaagagcgta tggcgatcag 480
   tgagcctgtg ccgataccct tatctgtgcc gatgatatat cagcaaggca aagatccttt 540
   tatcaatcct tatagaaatg ttgaggttct tgataccaat catgccgctg atcagcaaga 600
   tgagccaaaa accgaatcta ccaaagcttg gcctatggca gacactatgc catctcagcc 660
   atctgatact catcagtctg ccaaggctca ggcacaagtc ttcaaaggcg atccgatagt 720
   cattgatacc aaccgtgttc gagagccttt agaaagctat gagttatcaa gcctacgcta 780
   tcatggtcgt atttttgatg atgttagact tgtggcactc attatgagtc ctgatggcat 840
   cgttcatcgt gtgagtactg gacaatatct tggtaaaaat cacggaaaaa ttacccatat 900
   tgacagtcgt acgatacatc tgattgaagc ggtcgctgat acacaaggtg gctattatcg 960
   ccgtgatgta aacattcatt ttattcataa gcaatgacac 1000
<210> 56
   <211> 1001
   <212> DNA
   <213> Moraxella catarrhalis
<400> 56
   ttcatgcaac aagcgaccat cttggccgat gataccatcc tgctcaccta agaaaatcag 60
   tttatcagct tgcagggcaa tggctgtggt cagtgctaca tcttctgcca atagattaaa 120
   aatttcgccc gtaaccgaaa aacctgtcgg tcctagtagg acaatatggt cattatccaa 180
   attatggcga atggcatcga catcaattga gcgtacctca cctgtcatct gataatccat 240
   accatctctg atgccgtaag ggcgagcggt gacaaaatta cccgaaatgg catcaatacg 300
   agatccgtac attggggagt tagcaagccc catcgacagc cgagcttcga tttgtagacg 360
   aattgagccg actgcctcca agatggcagg catagattca tacggtgtta cacgcacatt 420
   ctcatgtagg tttgatatca gcttgcgatt ttgtaaattt ttttccactt gtgggcgtac 480
   accatgcaca agcaccaatt tgatgcccaa gctgtgtagc agtgcaaaat catgaatcag 540
   cgtactaaaa ttgtcacgag cgaccgcctc atcaccaaac ataaccacaa aggttttgcc 600
   acgatgggtg ttaatgtacg gggcagaatt acgaaaccaa tgcacaggtg tgagtgcagg 660
   agtgttctga taggtgctga cagaattcat gaatgctcca aagagtcaat ggctggtaaa 720
   ataagaatgg cgaacaatat atggcgagag cgtctgatgt tggtcaaatg tcccattaat 780
   aactatcaag ataccatcat accatagcaa agttttgggc agatgccaag cgaatttatc 840
   agcttgataa ggttggcata tgataaaatc taccatcatc gtcgccagtt ttgagcatgt 900
   gtaagtagtt accataatta aacagtcaag aaattcacac cgtcaatcag ctgtgctatg 960
   cttatgggca cataaaactt gaccaacaca ggataaattt a 1001
<210> 57
   <211> 1001
   <212> DNA
   <213> Moraxella catarrhalis
<400> 57
   ggcatacttt tgccatgctt tattttggca taactgctat aagcccattg ctacttttta      60
   tcatttatcc atatgtccaa taatgtgctt tatgtaattt aggcacacta ttaactcgtg     120
   ccactgttaa cattcagcat aaaaatctta acaatgaatc aaagcatcgt attggctgtt     180
   aaatgataag cttatattta tttaaattca gactaaatga ttgtaatatg gacatatcaa     240
   ggttgaaatc aaaaattttg gagagttatg tacgataatg ataaaaaatt gaccaccatc     300
   gtaggggtgt tgtatacggt gtcttatatt gccatatggt tggtcagtgg ctatatttta     360
   tggggctgga ttggtgtgac aggatttact cgtgcgatac tttggctgat cgcttggatg     420
   attgtgggta cgattgctga tagaattctg ataccgatta ttttgaccgt cgtggttggg     480
   ttattttcta tcttttttga aaaaaggcga taatttggtt attttttcac aaaaaatcat     540
   gatttttttt gtaaactatc taaaatatca attatgttat attatgtgat aaaagatggg     600
   catgcttaag ttttggattg caaaaatcct aatatcatca ctgaccaaag ctgtgatgat     660
   atcaaaactt tatcaaagtt cttagggtat tatcaagata tcataccaaa tgaatactta     720
   cccaacttac tataaaaatc aaatgatatg actgtgattt tattatcata gatacaaaaa     780
   tcaaaacgca tgagccaaag gtatgatgaa tgaatacaaa atttcgcaca cattatgaca     840
   atctaaatgt cgccagaaac gctgacattg cggtgatttg gtgggatagg ggtcaagcca     900
   gtgcgattaa gctaaatttt tatgtgggca atcgctgact ttattttatt tgtgccagtt     960
   ggaacaattc gtggtctaat gtatttattt taaggagata a                        1001
<210> 58
   <211> 1001
   <212> DNA
   <213> Moraxella catarrhalis
<400> 58
   tctggtctac atcccaaact atttacacaa gaaacactaa agacagtgga gcagatgacg      60
   ctcaaaaagg catcttatag taatttgaca gttaattttc gtcaagtgct tgtacaaaaa     120
   tacaccatcg tgcaagaagt ttgtaccaat ttaagcacaa tcattttggc acacactgtc     180
   aagcaatgct tcaggcaaat tagctgctgg taaagatact tgggtcatca tgcaatcgca     240
   tcaacccttc ttgctgcgtt gaagcgataa gtttgccatc ttgccaaaat tgaccatggt     300
   ttagaccctt ggcgtggctt gtggtatcgc tccacatgtc gtagagtaga tattcggtca     360
   tatcaaaagg gcgatggaaa tgtatggaat ggtcaatact agccatttgt agaccttgtg     420
   tcatcaggct tagcccatga ctcattaaac ctgtgctgac caaataataa tcagacacaa     480
   acgcaagtag tgcttgatga atggcaactg gctgctcccc aatatcagcg atacgcaccc     540
   aattggcttg gcgtggacgc tcaggcttgg gtgtcacagg gtctcgtggt gtgacggggc     600
   ggatttcgac atgacgctga cgcataaatc ttgctttgag tggttcggga attttatgta     660
   aataatccgc tttgagttct tgctcggttt ttaggctttc agggggtgga taatcaggca     720
   tggtttcttg gtaatcaagc ccgccttcca tgggtgaaaa tgaggcaatc atcgaaaaaa     780
   tgacctgttc attggtcgta tgattaccgt ttttgtcggt ggttggcaca tattgcaccg     840
   caatgacttc tcgagctgat aaactgcgtc catcacgtaa gcggcgtact tgatagatga     900
   ctggtagacg aatatcgcca cctcgtaaaa aataaccatg taggctatga caaggtttat     960
   caatcgttaa tgtgttagca ccagcaagca gcgcttgggc a                        1001
<210> 59
   <211> 1001
   <212> DNA
   <213> Moraxella catarrhalis
<400> 59
   taaaatgacc ttacaaaata aaattatatg ttcaaaaatc gcttaagtat tgaaaaaagc      60
   tataaaaact tatctattaa agcataaaag atattaaagc ataaaagacg agaaaagagc     120
   aagcgtcaat gatgatattt catataaaaa cttatgaaat ttttcaattt tttatcgatt     180
   gattcagctt ggctatcggt ggtcaacttt ggctgccaag acatcgccgg ctttttgaaa     240
   aatcatcaca atggcaacaa tgatgatggt tgaaatccac ttgacatata ccatgttgcg     300
   atgctcacca tagttaatcg caaggcttcc caagccacca ccgccaacca cacctgccat     360
   tgcagaataa ccaatcaaag acaccaaggt caatgtgacc gcattaatca aaatgggcag     420
   gctttcagca aaatagtatt tgctgacaac ctgccaatgc gttgcaccca tagatttggc     480
   agcttcggtc agtcctgtgg gtacttctaa taaagcattg gcactcaagc gtgcaaaaaa     540
   tggaattgct gccacactca aagggacgat ggcggctgtt gtgccaaggg ttgttcccac     600
   caaaaatcgt gtgactggca tgagaataat gagcaaaata ataaaaggaa cggagcgacc     660
   aatattaata ataacatcca aaattacaaa tacactgcga ttttcaagga tacgcccttt     720
   atcggttaaa aatgccaaaa accctatcgg tagcccaacc aaaacagcga tggcagtggc     780
   agcaagcccc atatagatgg tttcccaagt ggattgggca accatctccc acattcttgg     840
   gtgcatttca ctgacaaatt ttgtgacgat ttcattccac atagccgata atctcaatat     900
   tgacccgatg ggtggttaaa aattctattg cttgcatgac cgaggtgcct tcaccgataa     960
   gctcagcaat ggtaaagcca aattttatat cacctgcata a                        1001
<210> 60
   <211> 1001
   <212> DNA
   <213> Moraxella catarrhalis
<400> 60
   agtaaacaat ggtaacaaat acagcagtgt cgcacagtcc tcagtacgat gattctgaat      60
   ttgaatatgc aggattttgg atacgatttg tggcatgtct tgtcgataat ttaattgtta     120
   tgattataat tgcaccgtat tggttttata attatcagca aatgatggcc atgcctgctg     180
   accaaatacc gttttatagt gttggggatg ccatccttta tagtgctggg gatgctatcc     240
   taaacttagt gatggcggcg gcggttgttt ggttttgggt aaaaaaaggt gcaacaccag     300
   gtaaaatgct ctttgggctg caagtccgtg atgccaaaac agggcaattt atcagtgtgc     360
   caagggcatt attgcgatat tttagttatc tgatttcatc cgtgattctt tgtttgggac     420
   ttatttgggt tggttttgat aagaaaaaac aaggctggca tgataaaatt gccaaaactg     480
   ttgtggtaaa acgcattcgc tgatgggtcg ccagttaaac aataaaacca tcaaacgcaa     540
   gcagggcgat gtgtttgagc agttggcggt agataagcta aaacaagcag gctatgaaat     600
   tattttaacc aactttacca ccccatttgt tggtgagatt gatattatcg ccagacagcc     660
   tttggagcaa tcgcaccgtt tggtgcagcc aagattttgt acggtatttg ttgaagtgcg     720
   tagccgaaca agttctgtgt atggtacagc gcttgagagt gttacctcaa aaaagcaggc     780
   aaaaatctac cgaacagcag aacgattttt aatcaattat cccaaatata ttgatgatgc     840
   ataccgtttt gatgtcatgg tttttgattt ggttgatgga ttgattgaac atgaatggat     900
   aaaaaatgcg ttttgattgg ctcaatggtc gtgaattaaa atcaatcaag caatccgtag     960
   ctttactata agatatatcc cagtaatatg gaaacatagc a                        1001
<210> 61
   <211> 1001
   <212> DNA
   <213> Moraxella catarrhalis
<400> 61
   cgtttagctt catacgcaga ccttgtgcac cttcgggcaa ccgaagcatc acgccagcat      60
   cacgcatccg cacaaaaccc atcatgccat caatttcgct gctgatatga tataccccca     120
   ccaaagtaaa ccgcttaaat cgtggaataa cgcctgctgc tgagggtgag gcttcaggca     180
   aaaccaaggt aaccttatcc cccaacttaa gtcccatgtc agagacaatg gactcaccta     240
   atataatacc aaactcgccg atatgtaaat catccaaatt gcctgcggtc atatgctcat     300
   caatgataga aacttgcttt tcgtaatcag gctcaatgcc agaaaccacg attccagtca     360
   cctgaccttc agcggttaac ataccttgta gttgaatata aggggcaact gcttgcactt     420
   ctggattttg cattttgatt ttttcggcaa gttcttgcca atttgtcaaa atttctgttg     480
   aggtaactga agcttgaggc accatgccaa gaatgcgtga tttaatttca cggtcaaagc     540
   cattcatgac cgacaaaacc gtgataagca ctgcaacccc aagcgtaagc ccaatggttg     600
   agataaaaga aataaaggaa ataaagccat ttttacgctt agctttggta tatctaagcc     660
   caataaataa cgccaaggga cgaaacataa gctgtgttcc aaacgaccca accgtgctag     720
   tttagcactt ttttggacaa ataccaaaca tcacataaca aatgaatcat caggttggtt     780
   ttgttgcgct tgtgtatctg tatgataagt ttcttgctaa aacagctttt ttatgtcaga     840
   atacagaaaa ggtatatact tatattttta actttaaata gatctgcttt tttataccga     900
   tgatttggca tgaagtttat cggtctgata tgctggatat aagtttatcg gcttgatata     960
   aattttaatt aatcatcaaa tttttaagga atttatcatt a                        1001
<210> 62
   <211> 1001
   <212> DNA
   <213> Moraxella catarrhalis
<400> 62
   taaggatacc agattttggc ttgtcaatcg ttgtgttaat cattgtaacg gtttatagtg      60
   attgtcaatt aataagggta aaaaagtatt tatcaagtaa taatctttct tatatgtgaa     120
   tataatgaca aatttatcac atttttacaa ggatttttta tcaagattag gatatgttcc     180
   agcttaatta ttagtgatga gcgtgtgatt atttggcatc gttaaattta tgagtgctaa     240
   aattgccaaa tgattaaaat tttgctaaca tgatagcccc tttggtaggc tttatttggt     300
   attgatgagc aataataata taccgagtta aatggattaa cttaacatac gccaaaaact     360
   taacaacgaa aagtagatga ttatgacaga tacagtacaa aaagatacag cacagtcccc     420
   caaaaaagtt tatctaaaag actacacgcc gccagtatat gcagttaata aagtggattt     480
   ggatatccgc ttgtttgatg atcatgctgt cgttggtgcc aaacttaaaa tgacacgagc     540
   acacgcaggc gagcttcggc ttcttgggcg agatttaaag cttaaaagca ttcacctaaa     600
   tggtcaggaa ttagagtcgc aggcgtatca tcttgataag gaaggcttaa caattttaga     660
   tgcaccagat gtcgcagtga ttgagacatt ggttgagatt tcaccacaaa ccaacacaac     720
   acttgaaggg ctatatcaag caggaacagg tgatgataag atgtttgtga cacaatgcga     780
   acctgagggt tttcgcaaaa tcaccttttt ccctgaccgc cctgatgttt tgacagaata     840
   caccacacgc ctagaagcac caaagcattt taaaaccttg cttgccaatg gtaatttggt     900
   tgagtcagga gatgtggatg aaaatcgcca ttataccatt tggcatgatc ctaccaaaaa     960
   acccagctat ctattcgccg ctgtcattgc caatctagaa g                        1001
<210> 63
   <211> 1001
   <212> DNA
   <213> Haemophilus influenzae
<400> 63
   aaatcaagcg cctgtgcctg ctggtgatgg ttgtggagac gaattatatt cttggtttga      60
   accgccaaaa ccaggcactt cagtgagcaa acctaaagtt acaccgcctg agccgttttt     120
   gtgccaacag attttgaact caccgaatcg gagagaatgg ttagaatagc attgaggtaa     180
   atcaatatgg atatcggcat tgatctttta gcaatattgt tttgtgttgg ttttgtcgca     240
   tcatttatcg atgcaattgc tggcggtggt ggattaatca ccattccagc gttactcatg     300
   acaggtatgc caccagcaat ggcgttaggc accaacaaat tgcaagctat gggcggtgca     360
   ttatccgcaa gcctttattt cttgcgaaaa agagcggtca atttacgcga tatttggttt     420
   attttgattt gggttttctt aggttctgcc ctaggtacat tattaattca atcaattgac     480
   gtggcgattt tcaaaaaaat gcttcctttt ttgattttag ccattggtct atatttttta      540
   tttactccta aattaggtga tgaagatcga aaacaacgat taagttatct gttatttggt     600
   cttttagtta gcccattttt aggtttttat gatggcttct ttgggccagg gactggctca     660
   atcatgagtt tagcctgtgt tactttgcta ggatttaatc tcccgaaagc ggcagcacat     720
   gcaaaagtga tgaacttcac ttcgaacctt gcttcttttg cacttttctt attgggcgga     780
   caaattcttt ggaaagtggg tttcgtgatg atggctggga gcattttagg tgcaaattta     840
   ggtgccaaaa tggtgatgac gaaaggtaaa accttgattc gaccgatggt tgttatcatg     900
   tcttttatga tgacggctaa aatggtttac gatcagggtt ggtttcattt ttaattcgga     960
   aagcgcgcaa aagtgcggtt aaaattaatt acattttatt a                         1001
<210> 64
   <211> 1001
   <212> DNA
   <213> Haemophilus influenzae
<400> 64
   ttgaagtccc caatttaccc accacaattc ctgcggcaac attggctagg taacaagatt      60
   cttcgaaaga acgtccatct gctaatgtgg ttgctaatac actaatgaca gtgtcaccgg     120
   ctcccgtcac atcaaacact tcttttgcaa cggttggcaa atgataaggc tcttgatttg     180
   ggcgtaataa tgtcatgcct ttttcagaac gcgtcaccaa aagtgcggtt aattcaatat     240
   cagaaattaa ttttaaacct ttcttaataa tctcttcttc tgtattacat ttacctacaa     300
   cggcttcaaa ttcagacata ttgggtgtca ataatgtagc cccacgataa cgttcaaaat     360
   cagttccctt tggatcgatc aacacaggca cattcgcttt gcgtgcaatt tgaatcattt     420
   tctgaacatc tttaagcgtg cctttgccgt aatcagaaag aatcaaagca ccgtaatttt     480
   tcaccgcact ttctaacttc gctaataaat ccttgcaatc tacattattg aaatcttctt     540
   caaaatcaag gcggagcagc tgttgatgac gagataaaat acgtaattta gtaatggttg     600
   gatgggtttc taatgcaaca aaattacaat caatcttttg tttttctaat aagtgggaaa     660
   gtgcagaacc tgtctcatct tgtccaatca atcccattaa ctgaacgggt acattgagtg     720
   aagcaatatt catcgccaca tttgcagcac cgcccgcgcg ttcttcattt tcttgtacgc     780
   gaactactgg cactggtgct tctggtgaaa tacggttggt tgcaccgaac caataacgat     840
   caagcatcac atcgcctaat acaagtactt ttgcttgctt aaattctgct gaatattgag     900
   ccattttaaa atctctctat ttgaataacc aaaattgtgg cgattttacc acaactcaaa     960
   tttacgataa actacgcccc taacttacgt ggaaagaaca a                         1001
<210> 65
   <211> 1000
   <212> DNA
   <213> Haemophilus influenzae
<400> 65
   agcaataatt atagctggaa tattctttaa agatgaaaga gatcgtataa gacaaaaaga      60
   attttatatt ggagaattat tagcaattat tggttcgcta atattcgtaa taaatagttc     120
   aaataatgat ggaaatacag acttttttct tggggcaata tttcttttta cagctatttt     180
   tattcaatct gtacagaatt taattgtaaa aaaagtagcc aaaaagataa atgctgttgt     240
   aataagtgca tcgacagcaa caatttcagg agtattattt ttatgtttag cttttaatac    300
   taaacaaata tatttattac aagatgttgg cattggaatg ttgataggtt tagtttgcgc    360
   tggcttttat gggatgctaa cagggatgtt gatggctttt tatattgttc aaaaacaggg    420
   aatcactgtt tttaacattt tgcaattatt aattcctctt tcaactgcga taataggtta    480
   cttaacatta gatgaaagaa taaatatcta tcagggaatt agcggtatta ttgtaattat    540
   tggttgtgta ttggcattaa aaagaaaaaa caaggagtgt tgatatataa agtagatgat    600
   gttggtggaa taggtatagt taaatatctg gttcaattgg ttttattaag ggcgttagca    660
   attctccatt taagtttatg tttgaattag atattttggg aaaagatgga agaataaagc    720
   tgttaaataa tgctgaaaca tatgaactat accaatactc aaataaaaat aattctgctg    780
   gaaatgatta taaatctcta attctaactt gtagagagga taatgactat caatcagaaa    840
   gaatgattaa agccattaaa aatattattc attgtatgac taataatcat caacctattt    900
   caagtgctga aacatcttta gaaactatta aaattattca cggaataatt aattctgtta    960
   aaataggtaa tgatcctaac aatatataag gagaataagt                          1000
<210> 66
   <211> 1001
   <212> DNA
   <213> Haemophilus influenzae
<400> 66
   taaatactcc aaaataaatt tcagataacg tggtctgtaa gacaaaaaaa taaaaaaaat     60
   gttcaataag aggagagcaa attatcttgt ttaaaaggaa atcggagcag tacaaaaacg    120
   gtcttacaag tagcaaattc tataaattta tgttctaata cgcgcaattt tctagtcaat    180
   aaaaaggtca aaaaatgagc tggattaacc gaatttttag taaaagtcct tcttcttcca    240
   ctcgaaaagc caatgtgcca gaaggcgtat ggacaaaatg tactgcttgt gaacaagtac    300
   tttatagtga agaactcaaa cgtaatctgt atgtttgccc gaaatgtggt catcatatgc    360
   gtattgatgc tcgtgagcgt ttattaaatt tattggacga agattcaagc caagaaattg    420
   cggcagattt agaaccaaaa gatattttaa aattcaaaga tttaaagaaa tataaagatc    480
   gtatcaatgc ggcgcaaaaa gaaacgggcg agaaagatgc gctaattact atgacaggta    540
   cactttataa tatgccaatc gttgtggctg catcgaactt tgcttttatg ggcggttcaa    600
   tgggttctgt agttggtgca aaatttgtta aagcggctga aaaagcgatg gaaatgaatt    660
   gtccatttgt gtgtttctct gcgagtggtg gtgctcgtat gcaggaagca ttattctctt    720
   taatgcaaat ggcaaaaact agtgccgtac ttgctcaaat gcgtgaaaag ggtgtgccat    780
   ttatttcagt attaacggat ccgactttag gcggcgtatc agccagtttt gcgatgttag    840
   gggatttaaa tattgccgag ccaaaagcct taattggttt tgcagggcca cgcgttattg    900
   aacaaactgt gcgtgaaaaa ttgccagaag gtttccaacg tagtgagttt ctacttgaga    960
   aaggggcaat tgatatgatc gtgaaacgtt cagaaatgcg t                        1001
<210> 67
   <211> 1001
   <212> DNA
   <213> Haemophilus influenzae
<400> 67
   tcacttaatt caagcgcatc aatgttttct aaaacatcaa cagaattgac cgcacttgta     60
   tctaaaattt cgccatttat taagactgcg cgtaatgcca aaacatgatt agaggtttta    120
   ccatattgca atgagccttg cccagaggca tcggtgttaa tcattccacc taaagtcgct    180
   cgattgctgg tggacagttc tggggcaaag aacaaaccat gtggttttaa aaattgatta    240
   agttgatctt ttactacgcc tgcttgtact cgaacccaac gttcttttac attgagttct    300
   aagatggctg tcatatgacg agaaagatcc actattatat tgttattgat ggattgccca    360
   tttgtgccag tgcctccacc gcgaggcgta aagctgattg attgatattc aggtaaattt    420
   gccaattttg ttatccgcac tatatcagca accgttttcg gaaaaagaat tgcttgtgga    480
   agttgttggt aaacgctgtt atccgtagcc agacttaatc tatctgcata gtttgtcgca    540
   atatccccct caaaatgttg gcattgaaga tcatcaagat aatcaagtac atattgttca    600
   acttgaggaa tgcgatttag atttggcaac atagtatttg acccatttaa acatatcaga    660
   tggaggcttt gataatatcc taaggctaga ataatgtcga ttaggaaaga gagaggagaa    720
   agtaaaaagt ctgtttaaga aagtgttatt ttggataaaa actaaacaaa aaattcaaaa    780
   gaatttgatc ttttcaattt ttataggata ataagcgcac ttttgaacgt tcctttgggg    840
   taaacataag caaaggaatt gaatttgtca aaaggtaata aagtagggca aattcaaaac    900
   cctagttaag tgactgttta taatgtagct ttaattaaaa gttcagtata aacaaggaca    960
   ctttttatta ctattcgatc actaaataga ggacatcaaa a                        1001
<210> 68
   <211> 1001
   <212> DNA
   <213> Haemophilus influenzae
<400> 68
   tcgattgtat cctatataaa ttatagacgt aaaaaatcat taaataatgc aaacaccgtt      60
   aagcttaata acagtgctgc gccaattcga taacagatgc tttgcacccg ctcagaaaca     120
   ggttttcctt taacagcttc cattgttaaa aaaactaaat gaccgccatc taatactggt     180
   aatggaaata aattcataat ccctaaattt acactaatca atgccataaa acttaaaaaa     240
   tacaccaatc caatatttgc tgatgcgcca gcaccttttg caatagaaat tggcccactt     300
   aaattattta atgacaaatc gccagtaagt aatttcccta atattttcaa ggttaaaagg     360
   gaaagctgtc ctgttttttc aatgcctttt tgtaaagatt caagaatacc atattttaat     420
   tcagtacggt attcatccgc taattttgtt aaggctgggc taaccccaac aaaccatttg     480
   ccattttgat tacgcactgg agttaggact ttgtcaaatg tttctccatt acgttcaact     540
   ttaatagaaa aagattcgcc ttgttcgacc tgttttataa aatcttgcca aggaagtgcg     600
   gttaaatttt cttttaaaat tttatcaccg atttgtaaac cagctttctc agcgggagaa     660
   ttttgaacaa ctttagaaag caccatttca attttaggac gcataggcat aatccctaat     720
   gcctcaaaag cactttcttt ttcaggatcg aatgtccaat ttgtaagatt taaagtccgt     780
   tgttgttcaa tattagaatt gaaaggagaa aggctaatct caacattagg ctcccccatt     840
   tttgtggcaa gtagcatatt gatggtttcc caatcttgag tttcttcgcc atcaattgta     900
   agaatttgcg tattgggttc aatgtgggct tgtgctgcga ttgagtttgg tgttattgat     960
   tcaatcactg gtttaaccgt tggcattcca taaaggtaaa t                        1001
<210> 69
   <211> 1001
   <212> DNA
   <213> Haemophilus influenzae
<400> 69
   tttgataaat atccttaatt aaatgatggg tttaatattt tctctgccca attaaattag      60
   gcagagaacg ttgtttttga gttctgatga agaaaaaagt tcaatttatt agaaagaacc     120
   tccaatacta aattggaact gttcgacatc atcattttca tattttttaa ttggtttggc     180
   ataagagaat accaatggcc caataggaga ttgccattgg aatccgacac ctgtagaggc     240
   gcgaatacgg cttgatttgc cataatcggg taagcttttt aatacattgt tatctaaccc     300
   actcttatcc gatttccact tagtattcca aacacttgcc gcatcaacaa atagggaggt     360
   tcggactgta ttttggcttt tatcactcac aaacggtgtt ggtacaataa gttctgcact     420
   cgcagttgtg attgcattac caccaatcac atcagaactt atcttcttaa aagtaccatt     480
   accattacca tgttctgcat aaattgcgtt aggtccaata ctaccataag caaaaccacg     540
   taatgaaccg atgccacccg ctgtataagt ttgatagaac ggtaaacgct tgtttccaaa     600
   accatttgca tatcctgcag atgcttttgc agatacaacc cagaggtgat ctctgtctaa     660
   tgggtagaaa ccctgtacgt ctgcacttag tttgtagtat ttgttatcag aacctggaat     720
   agtaactcgt ccaccaagac ttgctttaac ccctttagtt gggaaatagc ctctattaag     780
   gctgttatag ttccaaccaa aagaaaaatc aaagtcattt gttttaatgc cattaccttt     840
   aaatttcatt gattgaatat ataaattacg gttatattct agagcaaagt tactaatttt     900
   attataggta tggcctaatc ctacataata ggagttattt tcatttacag ggaaacctaa     960
   agtaacatta cttccataag tcgtacgctt atagttagag g                        1001
<210> 70
   <211> 1001
   <212> DNA
   <213> Haemophilus influenzae
<400> 70
   ttagatttct cctaaatgag ttttttattt agttaagtat ggagaccaag ctggaaattt      60
   aacttgacca tcacttcctg gaaggctcgc cttaaagcga ccatctgcgg aaaccaattg     120
   tagcaccttt cctaagccct gtgtagaact ataaataatc ataattccat ttggagagag     180
   gcttgggctt tcgcctagaa aagatgtact aagtacctct gaaacgcccg ttgtgagatc     240
   ttgtttaact acattattgt taccattaat catcacaagt gtttttccat ctgcactaat     300
   ttgtgcgcta ccgcgaccac ccactgctgt tgcactacca ccgcttgcat ccattcgata     360
   aacttgtggc gaaccacttc tatcggatgt aaataaaatt gaatttccgt ctggcgacca     420
   cgctggttca gtattattac ccgcaccact cgtcaattga gtaggtgtac cgccatttgc     480
   tcccataacg taaatattca gaacaccatc acgagaagaa gcaaaagcta aacgagaacc     540
   atctggcgaa aaggctggtg cgccattatg cccttgaaaa gatgccacta ctttacgtgc     600
   gccagaattt aaatcctgta caacaagttg tgatttttta ttttcaaacg atacataagc     660
   caaacgctgg ccgtctggag accaagctgg agacataatt ggttgggcac tacgattgac     720
   gataaattga ttatagccat cataatctgc tacacgaact tcataaggtt gcgaaccgcc     780
   atttttttgc acaacataag cgatacgagt tctaaaggca ccacggatcg cagttaattt     840
   ttcaaaaact tcatcgctca cagtatgcgc gccatagcgt aaccatttat ttgttactgt     900
   atagctattt tgcattaata cagtccctgg cgtacctgat gcaccaaccg tatcaattaa     960
   ttgataagta atactataac cattacccga tggaaccact t                        1001
<210> 71
   <211> 1001
   <212> DNA
   <213> Haemophilus influenzae
<400> 71
   ggcgataacc gagtttttgg ggtatttagt gccaaagaag acccacaaaa cccaaaatta      60
   tccagagaaa ccttaattga tggcaagcta actactttta aaagaactga tgcaaaaacc     120
   aatacaacag ccgatacaac aaccaataaa acaaccaatg caataaccga tgaaaaaaac     180
   tttaagacgg aagatatact aagttttggt gaagctgatt atcttttaat tgacaatcag     240
   cctgttccgc ttttacctga aaaaaatact gatgatttca taagtagtag gcatcatact     300
   gtaggaaata aacgctataa agtggaagca tgttgcaaga atctaagcta tgtaaaattt     360
   ggtatgtatt atgaagaccc acttaaagaa gaagaaaaag aaaaagaaaa agaaaaagac     420
   caagaaaaaa aagaaaaaga aaaacaaacg acgacaacat ctatcgagac ttattatcaa     480
   ttcttattag gtcaccgtac tgccaaggcc gacatacctg caacgggaaa cgtgaaatat     540
   cgcggtaatt ggtttggtta tattggtgat gacacgacat cttactccac tactggagat     600
   aaaaatgctc tcgccgagtt tgatgtaaat tttgccgata aaaagctaac aggcgaatta     660
   aaacgacacg ataatggaaa taccgtattt aaaattactg cagaccttca aagtggtaag     720
   aatgacttca ctggtacagc aaccgcaaca aattttgtaa tagatggtaa caatagtcaa     780
   actggaaata cccaaattaa tattaaaact gaagtaaatg gggcatttta tggacctaag     840
   gctacagaat taggcggtta tttcacctat aacggaaatt ctacagctaa aaattcctca     900
   accgtacctt caccacccaa ttcaccaaat gcaagagctg cagttgtgtt tggagctaaa     960
   aaacaacaag tagaaacaac caagtaatgg aatactaaaa a                        1001
<210> 72
   <211> 1001
   <212> DNA
   <213> Haemophilus influenzae
<400> 72
   tagaattata ttcttataca aaattgataa ttgttcgcat tatcattttt tttttgtaat      60
   aatgtcaact tataattttt taagttcatg gataaaatat gaaaaatggc gtaaaacaac     120
   tttttctctt atcattaata ggcttatcat taacgaatgt agcttgggca gaagttgcac     180
   gtcctaaaaa tgatacattg acaaatacga ttcaaagtgc ggaattaaaa acctcctctt     240
   tttcctctat gcctaagaaa gaaataccaa ataggcatat tatttctctt tccaaaagcc     300
   aattagcgca ccatccaagg cttgttttgc gtgggttaat tcctgcttta tatcaaaata     360
   acactcaggc agttcaactg ttattaccac tatataaaca atttcctcaa caagataatt     420
   tcttactaac ttgggcaaag gctattgaag ctcgtgaaca aggtgattta actcaatcta     480
   ttgcttatta tcgtgaatta ttcgctcgag acgcatcttt actaccttta cgttattaat     540
   tagctcaagc tctatttttt aactatgaaa atgaagctgc caaaattcaa tttgaaaaat     600
   tacgtacaga ggtagatgat gaaaaatttt taggtgttat tgatcagtat cttttaacac     660
   taaatcagcg gaatcaatgg atatggcaag taggattaaa ttttttaaat gatgataatt     720
   tgaataacgc tccaaaaagt ggcacaaaaa ttggtagttg gaccgcttgg gaaaaagaaa     780
   gtgggcaggg ggtagggtat tctttatcag tagaaaaaaa atggccatgg gcagatcatt     840
   tttttagtaa aactatgttt aatgggaatg gaaaatatta ttgggataat aaaaaataca     900
   atgaggctac tgtgcgtata ggtggtggtt taggctatca aactgcctca gttgaagtct     960
   cgttgtttcc ttttcaagaa aaacgctggt atgcaggcgg t                        1001
<210> 73
   <211> 1001
   <212> DNA
   <213> Haemophilus influenzae
<400> 73
   taataaattg ctccataaag aggtttgtgc cttataaata aggcaataaa gattaatata      60
   aaccgtttat taaaatgcca aaggcttaat aaacagcaaa ctttgttttc ccaaaaaaag     120
   taaaaaactc ttccattata tatatatata tatataatta aagccctttt tgaaaaattt     180
   catatttttt tgaattaatt cgctgtaggt tgggtttttg cccacatgga gacatataaa     240
   aaagatttgt agggtgggcg taagcccacg cggaacatca tcaaacaact gtaatgttgt     300
   attaggcacg gtgggcttat gcctcgccta cggggaaatg aataaggata aatatgggct     360
   tagcccagtt tatggattta attatgttga aatggggaaa acaatgttta aaaaaacact     420
   tttatttttt accgcactat tttttgccgc actttgtgca ttttcagcca atgcagatgt     480
   gattatcact ggcaccagag tgatttatcc cgctgggcaa aaaaatgtta tcgtgaagtt     540
   agaaaacaat gatgattcgg cagcattggt gcaagcctgg attgataatg gcaatccaaa     600
   tgccgatcca aaatacacca aaaccccttt tgtgattacc ccgcctgttg ctcgagtgga     660
   agcgaaatca gggcaaagtt tgcggattac gttcacaggc agcgagcctt tacctgatga     720
   tcgcgaaagc ctcttttatt ttaatttgtt agatattccg ccgaaacctg atgcggcatt     780
   tctggcaaaa cacggcagct ttatgcaaat tgccattcgc tcacgtttga agttgtttta     840
   tcgccctgcg aaactctcga tggattctcg tgatgcaatg aaaaaagtag tgtttaaagc     900
   cacacctgaa ggggtgttgg tggataatca aaccccttat tatatgaact acattggttt     960
   gttacatcaa aataaacctg cgaaaaatgt caaaatggtt g                        1001
<210> 74
   <211> 1001
   <212> DNA
   <213> Haemophilus influenzae
<400> 74
   tagtagattt ccgcacgggc aaaaatacaa tggtgttatt taacctcact ttgccaaatg      60
   gcgagccagt gccaatggca tccaccgcac aagatagcga aggggcattt gtgggcgatg     120
   tggtgcaagg tggtgtgctt ttcgctaata aacttaccca gccaaaaggc gagttaatcg     180
   tcaaatgggg tgagcgagaa agcgaacaat gccgtttcca atatcaagtt gatttggata     240
   acgcacaaat acaaagtcac gatattcaat gcaaaaccgc aaaataaata attgaagagg     300
   atttatgcaa aaaacaccca aaaaattaac cgcgcttttc catcaaaaat ccactgctac     360
   ttgtagtgga gcaaattata gtggagcaaa ttatagtggc tcaaaatgct ttaggtttca     420
   tcgtctggct ctgcttgctt gcgtggctct gcttgattgc attgtggcac tgcctgctta     480
   tgcttacgat ggcagagtga cctttcaagg ggagatttta agtgatggca cttgtaaaat     540
   tgaaacagac agccaaaatc gcacggttac cctgccaaca gtgggaaaag ctaatttaag     600
   ccacgcaggg caaaccgccg cccctgtgcc tttttccatc acgttaaaag aatgcaatgc     660
   agatgatgct atgaaagcta atctgctatt taaaggggga gacaacacaa cagggcaatc     720
   ttatctttcc aataaggcag gcaacggcaa agccaccaac gtgggcattc aaattgtcaa     780
   agccgatggc ataggcacgc ctatcaaggt ggacggcacc gaagccaaca gcgaaaaagc     840
   ccccgacaca ggtaaagcgc aaaacggcac agttattcaa ccccgttttg gctactttgg     900
   ctcgttatta cgccacaggt gaagccaccg caggcgacgt tgaagccact gcaacttttg     960
   aagtgcagta taactaaaat atttattatc cagtgaaaaa a                        1001
<210> 75
   <211> 1001
   <212> DNA
   <213> Haemophilus influenzae
<400> 75
   ttatccgcta acatttcatc agtaattcca tgaactttaa tcgcatcagg atcancgggg      60
   cgatctggct taatataaat atgayaatta ttacctgtgt aacgacgatt tattaattca     120
   actgcaccaa tttcaataat gcagtgtcct tcataatgcg cgccaagctg attcatacct     180
   gtagtttcag tatctaatac aatttggcga ttgggattaa tcatttgttc aacctatctc     240
   tttccattaa aatacttgcc attctacaca acaacctttt tgttatgcck aaacagattg     300
   aaatttttac tgatggatct tgcttaggta atccaggggc gggcggaatt ggtgccgtat     360
   tgcgttataa acaacatgaa aaaacactct ccaaaggcta tttccaaacc accaataatc     420
   gaatggaatt acgcgctgtc attgaagcat taaatacatt aaaagaacct tgcttgatca     480
   cgctttatag tgatagccaa tatatgaaaa atggcataac caaatggatc tttaactgga     540
   aaaaaaataa ttggaaagca agttctggaa agcctgtaaa aaaccaagat ttatggatag     600
   ccttagatga atccatccaa cgtcataaaa ttaattggca atgggtaaaa ggccatgctg     660
   gacacagaga aaatgaaatt tgcgatgaat tagcaaaaaa aggggcagaa aatccgacat     720
   tggaagatat ggggtacata gaagaataat acaactgata taacgtcata tttttcgata     780
   cctaaaaata tttaatactt aaacctaaaa cagaataaaa aataatcaaa ttcatttaaa     840
   aaatgtgatc tcgatcagat ttcaagaaaa ttaaaatttt ggagtattga catcaaaaat     900
   tttttttgta aagatgcagc tcgtccgttt tggcgattgg acaattctat tggagaaaag      960
   ttcaatcata gatagtaaac aaccataagg aatacaaatt a                        1001
<210> 76
   <211> 924
   <212> DNA
   <213> Moraxella catarrhalis
<220>
   <221> CDS
   <222> (1)...(924)
<400> 76
   tca gtg ctt ggt ttt tta aga tat gta ccg ctg tca gtc ctg cat gga      48
   Ser Val Leu Gly Phe Leu Arg Tyr Val Pro Leu Ser Val Leu His Gly
   1               5                   10                  15
   ttg gcg gcg tgt gcg tct tat att tcc tat cat tgc agg ctt agt att      96
   Leu Ala Ala Cys Ala Ser Tyr Ile Ser Tyr His Cys Arg Leu Ser Ile
               20                  25                  30
   tat cgc agc atc caa gcc aat tta atc ttg gtt cac ccc aag atg cca     144
   Tyr Arg Ser Ile Gln Ala Asn Leu Ile Leu Val His Pro Lys Met Pro
           35                  40                  45
   gac gca cag cgg caa aaa ctc gcc aaa caa atc cta aaa aat cag ctc     192
   Asp Ala Gln Arg Gln Lys Leu Ala Lys Gln Ile Leu Lys Asn Gln Leu
       50                  55                  60
   atc agt gca gtc gac agt ctt aaa act tgg gca atg cca cca aaa tgg     240
   Ile Ser Ala Val Asp Ser Leu Lys Thr Trp Ala Met Pro Pro Lys Trp
   65                  70                  75                  80
   tct atc gca caa att aaa acg gtt cat cat gaa gat atc cta atc aaa     288
   Ser Ile Ala Gln Ile Lys Thr Val His His Glu Asp Ile Leu Ile Lys
                   85                  90                  95
   gca ctt gcc aat cca agt ggt atg ctt gcc att gtg cct cat atc ggc     336
   Ala Leu Ala Asn Pro Ser Gly Met Leu Ala Ile Val Pro His Ile Gly
               100                 105                 110
   act tgg gag atg atg aat gct tgg ctc aat acc ttt ggc tcc cct act     384
   Thr Trp Glu Met Met Asn Ala Trp Leu Asn Thr Phe Gly Ser Pro Thr
           115                 120                 125
   atc atg tat aag ccc atc aaa aat gcg gcg gta gat cgc ttt gtt tta     432
   Ile Met Tyr Lys Pro Ile Lys Asn Ala Ala Val Asp Arg Phe Val Leu
       130                 135                 140
   cag ggg cgt gaa aga cta aat gcc agc ctt gta ccc aca gat gct agt     480
   Gln Gly Arg Glu Arg Leu Asn Ala Ser Leu Val Pro Thr Asp Ala Ser
   145                 150                 155                 160
   ggt gtt aag gca att ttt aaa aca ctc aaa gca ggt gga ttt agt atc     528
   Gly Val Lys Ala Ile Phe Lys Thr Leu Lys Ala Gly Gly Phe Ser Ile
                   165                 170                 175
   ata ctg ccc gac cat gta cct gat cca tca ggt ggt gag att gct cct     576
   Ile Leu Pro Asp His Val Pro Asp Pro Ser Gly Gly Glu Ile Ala Pro
               180                 185                 190
   ttt ttt ggt att aaa acc cta acc agt acg ctg gcg tca aag ctt gct     624
   Phe Phe Gly Ile Lys Thr Leu Thr Ser Thr Leu Ala Ser Lys Leu Ala
           195                 200                 205
   gca aaa act ggt tgt gct ctt gtt ggc tta agc tgt att cgg cgt gaa     672
   Ala Lys Thr Gly Cys Ala Leu Val Gly Leu Ser Cys Ile Arg Arg Glu
       210                 215                 220
   gat ggc gat ggt ttt gaa att ttt tgt tat gaa tta aat gat gaa caa 720
   Asp Gly Asp Gly Phe Glu Ile Phe Cys Tyr Glu Leu Asn Asp Glu Gln
   225 230 235 240
   ctt tat tca aaa aat acc aaa att gca acc act gct tta aat ggt gcg 768
   Leu Tyr Ser Lys Asn Thr Lys Ile Ala Thr Thr Ala Leu Asn Gly Ala
   245 250 255
   atg gaa caa atg att tat cca cat ttt ttg cat tat atg tgg agc tat 816
   Met Glu Gln Met Ile Tyr Pro His Phe Leu His Tyr Met Trp Ser Tyr
   260 265 270
   cgt cgg ttc aag cat aca cca cta tta aat aat cct tat tta ctt aat 864
   Arg Arg Phe Lys His Thr Pro Leu Leu Asn Asn Pro Tyr Leu Leu Asn
   275 280 285
   gaa aat gag cta aaa aaa ata gcc ata aag ctt caa gcc atg tca aag 912
   Glu Asn Glu Leu Lys Lys Ile Ala Ile Lys Leu Gln Ala Met Ser Lys
   290 295 300
   gat agt tat gag 924
   Asp Ser Tyr Glu
   305
<210> 77
   <211> 894
   <212> DNA
   <213> Neisseria meningitidis
<220>
   <221> CDS
   <222> (1)...(894)
<400> 77
   atg ttt cgt tta caa ttc ggg ctg ttt ccc cct ttg cga acc gcc atg 48
   Met Phe Arg Leu Gln Phe Gly Leu Phe Pro Pro Leu Arg Thr Ala Met
   1 5 10 15
   cac atc ctg ttg acc gcc ctg ctc aaa tgc ctc tcc ctg ctg cca ctt 96
   His Ile Leu Leu Thr Ala Leu Leu Lys Cys Leu Ser Leu Leu Pro Leu
   20 25 30
   tcc tgt ctg cac acg ctg gga aac cgg ctc gga cat ctg gcg ttt tac 144
   Ser Cys Leu His Thr Leu Gly Asn Arg Leu Gly His Leu Ala Phe Tyr
   35 40 45
   ctt tta aag gaa gac cgc gcg cgc atc gtc gcc aat atg cgt cag gca 192
   Leu Leu Lys Glu Asp Arg Ala Arg Ile Val Ala Asn Met Arg Gln Ala
   50 55 60
   ggc atg aat ccc gac ccc aaa aca gtc aaa gcc gtt ttt gcg gaa acg 240
   Gly Met Asn Pro Asp Pro Lys Thr Val Lys Ala Val Phe Ala Glu Thr
   65 70 75 80
   gca aaa ggc ggt ttg gaa ctt gcc ccc gcg ttt ttc aga aaa ccg gaa 288
   Ala Lys Gly Gly Leu Glu Leu Ala Pro Ala Phe Phe Arg Lys Pro Glu
   85 90 95
   gac ata gaa aca atg ttc aaa gcg gta cac ggc tgg gaa cat gtg cag 336
   Asp Ile Glu Thr Met Phe Lys Ala Val His Gly Trp Glu His Val Gln
   100 105 110
   cag gct ttg gac aaa cac gaa ggg ctg cta ttc atc acg ccg cac atc 384
   Gln Ala Leu Asp Lys His Glu Gly Leu Leu Phe Ile Thr Pro His Ile
           115                 120                 125
   ggc agc tac gat ttg ggc gga cgc tac atc agc cag cag ctt ccg ttc      432
   Gly Ser Tyr Asp Leu Gly Gly Arg Tyr Ile Ser Gln Gln Leu Pro Phe
       130                 135                 140
   ccg ctg acc gcc atg tac aaa ccg ccg aaa atc aaa gcg ata gac aaa      480
   Pro Leu Thr Ala Met Tyr Lys Pro Pro Lys Ile Lys Ala Ile Asp Lys
   145                 150                 155                 160
   atc atg cag gcg ggc agg gtt cgc ggc aaa gga aaa acc gcg cct acc      528
   Ile Met Gln Ala Gly Arg Val Arg Gly Lys Gly Lys Thr Ala Pro Thr
                   165                 170                 175
   agc ata caa ggg gtc aaa caa atc atc aaa gcc ctg cgt tcg ggc gaa      576
   Ser Ile Gln Gly Val Lys Gln Ile Ile Lys Ala Leu Arg Ser Gly Glu
               180                 185                 190
   gca acc atc gtc ctg ccc gac cac gtc ccc tcc cct caa gaa ggc ggg      624
   Ala Thr Ile Val Leu Pro Asp His Val Pro Ser Pro Gln Glu Gly Gly
           195                 200                 205
   gaa ggc gta tgg gtg gat ttc ttc ggc aaa cct gcc tat acc atg acg      672
   Glu Gly Val Trp Val Asp Phe Phe Gly Lys Pro Ala Tyr Thr Met Thr
       210                 215                 220
   ctg gcg gca aaa ttg gca cac gtc aaa ggc gtg aaa acc ctg ttt ttc      720
   Leu Ala Ala Lys Leu Ala His Val Lys Gly Val Lys Thr Leu Phe Phe
   225                 230                 235                 240
   tgc tgc gaa cgc ctg cct ggc gga caa ggt ttc gat ttg cac atc cgc      768
   Cys Cys Glu Arg Leu Pro Gly Gly Gln Gly Phe Asp Leu His Ile Arg
                   245                 250                 255
   ccc gtc caa ggg gaa ttg aac ggc gac aaa gcc cat gat gcc gcc gtg      816
   Pro Val Gln Gly Glu Leu Asn Gly Asp Lys Ala His Asp Ala Ala Val
               260                 265                 270
   ttc aac cgc aat gcc gaa tat tgg ata cgc cgt ttt ccg acg cag tat      864
   Phe Asn Arg Asn Ala Glu Tyr Trp Ile Arg Arg Phe Pro Thr Gln Tyr
           275                 280                 285
   ctg ttt atg tac aac cgc tac aaa atg ccg                              894
   Leu Phe Met Tyr Asn Arg Tyr Lys Met Pro
       290                 295
<210> 78
   <211> 936
   <212> DNA
   <213> Haemophilus influenzae
<220>
   <221> CDS
   <222> (1)...(936)
<400> 78
   atg aaa aac gaa aaa ctc cct caa ttt caa ccg cac ttt tta gcc cca      48
   Met Lys Asn Glu Lys Leu Pro Gln Phe Gln Pro His Phe Leu Ala Pro
    1               5                  10                  15
   aaa tac tgg ctt ttt tgg cta ggc gtg gca att tgg cga agt att tta      96
   Lys Tyr Trp Leu Phe Trp Leu Gly Val Ala Ile Trp Arg Ser Ile Leu
               20                                      25                                      30
   tgt ctt ccc tat cct att ttg cgc cat att ggt cat ggt ttc ggt tgg       144
   Cys Leu Pro Tyr Pro Ile Leu Arg His Ile Gly His Gly Phe Gly Trp
           35                      40                      45
   ctg ttt tca cat tta aaa gtg ggt aaa cgt cga gct gcc att gca cgc       192
   Leu Phe Ser His Leu Lys Val Gly Lys Arg Arg Ala Ala Ile Ala Arg
       50                      55                      60
   cgt aat ctt gaa ctt tgt ttc cct gat atg cct gaa aac gaa cgt gag       240
   Arg Asn Leu Glu Leu Cys Phe Pro Asp Met Pro Glu Asn Glu Arg Glu
   65                      70                      75                      80
   acg att ttg caa gaa aat ctt cgt tca gta ggc atg gca att atc gaa       288
   Thr Ile Leu Gln Glu Asn Leu Arg Ser Val Gly Met Ala Ile Ile Glu
                   85                      90                      95
   act ggc atg gct tgg ttt tgg tcg gat tca cgt atc aaa aaa tgg tcg       336
   Thr Gly Met Ala Trp Phe Trp Ser Asp Ser Arg Ile Lys Lys Trp Ser
               100                     105                     110
   aaa gtt gaa ggc tta cat tat cta aaa gaa aat caa aaa gat gga att       384
   Lys Val Glu Gly Leu His Tyr Leu Lys Glu Asn Gln Lys Asp Gly Ile
           115                     120                     125
   gtt ctc gtc ggt gtt cat ttc tta acg cta gaa ctt ggc gca cgc atc       432
   Val Leu Val Gly Val His Phe Leu Thr Leu Glu Leu Gly Ala Arg Ile
       130                     135                     140
   att ggt tta cat cat cct ggc att ggt gtt tat cgt cca aat gat aat       480
   Ile Gly Leu His His Pro Gly Ile Gly Val Tyr Arg Pro Asn Asp Asn
   145                     150                     155                     160
   cct ttg ctt gat tgg cta caa aca caa ggc cgt tta cgc tcc aat aaa       528
   Pro Leu Leu Asp Trp Leu Gln Thr Gln Gly Arg Leu Arg Ser Asn Lys
                   165                     170                     175
   gat atg ctt gat cgt aaa gat tta cgc gga atg atc aaa gct tta cgc       576
   Asp Met Leu Asp Arg Lys Asp Leu Arg Gly Met Ile Lys Ala Leu Arg
               180                     185                     190
   cac gaa gaa acc att tgg tat gcg cct gat cac gat tac ggc aga aaa       624
   His Glu Glu Thr Ile Trp Tyr Ala Pro Asp His Asp Tyr Gly Arg Lys
           195                     200                     205
   aat gcc gtt ttt gtt cct ttt ttt gca gta cct gac act tgc act act       672
   Asn Ala Val Phe Val Pro Phe Phe Ala Val Pro Asp Thr Cys Thr Thr
       210                     215                     220
   act ggt agt tat tat tta ttg aaa tcc tcg caa aac agc aaa gtg att       720
   Thr Gly Ser Tyr Tyr Leu Leu Lys Ser Ser Gln Asn Ser Lys Val Ile
   225                     230                     235                     240
   cca ttt gcg cca tta cgc aat aaa gat ggt tca ggc tat acc gtg agt       768
   Pro Phe Ala Pro Leu Arg Asn Lys Asp Gly Ser Gly Tyr Thr Val Ser
                   245                     250                     255
   att tca gcg cct gtt gat ttt acg gat tta caa gat gaa acg gcg att       816
   Ile Ser Ala Pro Val Asp Phe Thr Asp Leu Gln Asp Glu Thr Ala Ile
               260                     265                     270
   gct gcg cga atg aat caa atc gta gaa aag gaa atc atg aag ggc ata       864
   Ala Ala Arg Met Asn Gln Ile Val Glu Lys Glu Ile Met Lys Gly Ile
               275                     280                     285
   tca caa tat atg tgg cta cat cgc cgt ttt aaa aca cgt cca gat gaa      912
   Ser Gln Tyr Met Trp Leu His Arg Arg Phe Lys Thr Arg Pro Asp Glu
       290                     295                     300
   aat acg cct agt tta tac gat taa                                      936
   Asn Thr Pro Ser Leu Tyr Asp  *
   305                 310
<210> 79
   <211> 957
   <212> DNA
   <213> Haemophilus influenzae
<220>
   <221> CDS
   <222> (1)...(957)
<400> 79
   atg tcg gat aat caa caa aat tta cgt ttg acg gcg aga gtg ggc tat       48
   Met Ser Asp Asn Gln Gln Asn Leu Arg Leu Thr Ala Arg Val Gly Tyr
    1               5                  10                  15
   gaa gcg cac ttt tca tgg tcg tat tta aag cct caa tat tgg ggg att       96
   Glu Ala His Phe Ser Trp Ser Tyr Leu Lys Pro Gln Tyr Trp Gly Ile
               20                  25                  30
   tgg ctt ggt att ttc ttt tta ttg ttg tta gca ttt gtg cct ttt cgt      144
   Trp Leu Gly Ile Phe Phe Leu Leu Leu Leu Ala Phe Val Pro Phe Arg
           35                  40                  45
   ctg cgc gat aaa ttg acg gga aaa tta ggt att tgg att ggg cat aaa      192
   Leu Arg Asp Lys Leu Thr Gly Lys Leu Gly Ile Trp Ile Gly His Lys
       50                  55                  60
   gca aag aaa cag cgt acg cgt gca caa act aac ttg caa tat tgt ttc      240
   Ala Lys Lys Gln Arg Thr Arg Ala Gln Thr Asn Leu Gln Tyr Cys Phe
   65                  70                  75                  80
   cct cat tgg act gaa caa caa cgt gag caa gtg att gat aaa atg ttt      288
   Pro His Trp Thr Glu Gln Gln Arg Glu Gln Val Ile Asp Lys Met Phe
                   85                  90                  95
   gcg gtt gtc gct cag gtt atg ttt ggt att ggt gag att gcc atc cgt      336
   Ala Val Val Ala Gln Val Met Phe Gly Ile Gly Glu Ile Ala Ile Arg
               100                 105                 110
   tca aag aaa cat ttg caa aaa cgc agc gaa ttt atc ggt ctt gaa cat      384
   Ser Lys Lys His Leu Gln Lys Arg Ser Glu Phe Ile Gly Leu Glu His
           115                 120                 125
   atc gaa cag gca aaa gct gaa gga aag aat att att ctt atg gtg cca      432
   Ile Glu Gln Ala Lys Ala Glu Gly Lys Asn Ile Ile Leu Met Val Pro
       130                 135                 140
   cat ggc tgg gcg att gat gcg tct ggc att att ttg cac act caa ggc      480
   His Gly Trp Ala Ile Asp Ala Ser Gly Ile Ile Leu His Thr Gln Gly
   145                 150                 155                 160
   atg cca atg act tct atg tat aat cca cac cgt aat cca ttg gtg gat      528
   Met Pro Met Thr Ser Met Tyr Asn Pro His Arg Asn Pro Leu Val Asp
                   165                 170                 175
   tgg ctt tgg acg att aca cgc caa cgt ttc ggc gga aaa atg cat gca      576
   Trp Leu Trp Thr Ile Thr Arg Gln Arg Phe Gly Gly Lys Met His Ala
               180                 185                 190
   cgc caa aat ggt att aaa cct ttt tta agt cat gtt cgt aaa ggc gaa      624
   Arg Gln Asn Gly Ile Lys Pro Phe Leu Ser His Val Arg Lys Gly Glu
           195                 200                 205
   atg ggt tat tac tta ccc gat gaa gat ttt ggg gcg gaa caa agc gta      672
   Met Gly Tyr Tyr Leu Pro Asp Glu Asp Phe Gly Ala Glu Gln Ser Val
       210                 215                 220
   ttt gtt gat ttc ttt ggg act tat aaa gcg aca tta cca ggg tta aat      720
   Phe Val Asp Phe Phe Gly Thr Tyr Lys Ala Thr Leu Pro Gly Leu Asn
   225                 230                 235                 240
   aaa atg gca aaa ctt tct aaa gcc gtt gtt att cca atg ttt cct cgt      768
   Lys Met Ala Lys Leu Ser Lys Ala Val Val Ile Pro Met Phe Pro Arg
                   245                 250                 255
   tat aac gct gaa acg ggc aaa tat gaa atg gaa att cat cct gca atg      816
   Tyr Asn Ala Glu Thr Gly Lys Tyr Glu Met Glu Ile His Pro Ala Met
               260                 265                 270
   aat tta agt gat gat cct gaa caa tca gcc cga gca atg aac gaa gaa      864
   Asn Leu Ser Asp Asp Pro Glu Gln Ser Ala Arg Ala Met Asn Glu Glu
           275                 280                 285
   ata gaa tct ttt gtt acg cca gcg cca gag caa tat gtt tgg att ttg      912
   Ile Glu Ser Phe Val Thr Pro Ala Pro Glu Gln Tyr Val Trp Ile Leu
       290                 295                 300
   caa tta ttg cgt aca agg aaa gat ggc gaa gat ctt tat gat taa          957
   Gln Leu Leu Arg Thr Arg Lys Asp Gly Glu Asp Leu Tyr Asp  *
   305                 310                 315
<210> 80
   <211> 1046
   <212> DNA
   <213> Moraxella catarrhalis
<220>
   <221> CDS
   <222> (1)...(1044)

<400> 80
   atg agt tgc cat cat cag cat aag cag aca ccc aaa cac gcc ata tcc       48
   Met Ser Cys His His Gln His Lys Gln Thr Pro Lys His Ala Ile Ser
   1               5                   10                  15
   att aag cat atg cca agc ttg aca gat act cat aaa caa agt agc caa       96
   Ile Lys His Met Pro Ser Leu Thr Asp Thr His Lys Gln Ser Ser Gln
               20                  25                  30
   gct gag cca aaa tcg ttt gaa tgg gcg ttt tta cat ccc aaa tat tgg      144
   Ala Glu Pro Lys Ser Phe Glu Trp Ala Phe Leu His Pro Lys Tyr Trp
           35                  40                  45
   gga gtt tgg ctg gct ttt gcg ttg att tta ccg ctg att ttt cta ccg      192
   Gly Val Trp Leu Ala Phe Ala Leu Ile Leu Pro Leu Ile Phe Leu Pro
       50                  55                  60
   ctg cgt tgg cag ttt tgg atc ggc aag cgt ctt ggc att ttg gta cat      240
   Leu Arg Trp Gln Phe Trp Ile Gly Lys Arg Leu Gly Ile Leu Val His
    65                  70                  75                  80
   tac tta gct aaa agc cga gtt caa gac act cta acc aac ctg cag ctt      288
   Tyr Leu Ala Lys Ser Arg Val Gln Asp Thr Leu Thr Asn Leu Gln Leu
                    85                  90                  95
   acc ttc cca aat caa cca aaa tca aaa cac aag gcc acc gca cgg caa      336
   Thr Phe Pro Asn Gln Pro Lys Ser Lys His Lys Ala Thr Ala Arg Gln
               100                 105                 110
   gta ttt att aat caa ggt att ggt att ttt gaa agt tta tgt gca tgg      384
   Val Phe Ile Asn Gln Gly Ile Gly Ile Phe Glu Ser Leu Cys Ala Trp
           115                 120                 125
   ttt cgc cct aat gtc ttt aaa cgc act ttt agc att tct ggt tta cag      432
   Phe Arg Pro Asn Val Phe Lys Arg Thr Phe Ser Ile Ser Gly Leu Gln
       130                 135                 140
   cat ttg att gat gcc caa aaa caa aat aaa gcg gtg att tta ctt ggt      480
   His Leu Ile Asp Ala Gln Lys Gln Asn Lys Ala Val Ile Leu Leu Gly
   145                 150                 155                 160
   gga cat cgc acg acg ctt gat ttg ggc ggt cgg tta tgt aca cag ttt      528
   Gly His Arg Thr Thr Leu Asp Leu Gly Gly Arg Leu Cys Thr Gln Phe
                   165                 170                 175
   ttt gcg gcg gac tgc gtg tat cgc cca caa aac aac cct ttg ctt gaa      576
   Phe Ala Ala Asp Cys Val Tyr Arg Pro Gln Asn Asn Pro Leu Leu Glu
               180                 185                 190
   tgg ttt atc tat aat gca cgc cgc tgt atc ttt gat gag caa atc tca      624
   Trp Phe Ile Tyr Asn Ala Arg Arg Cys Ile Phe Asp Glu Gln Ile Ser
           195                 200                 205
   aat cgt gat atg aaa aaa ctc atc act cgg ctc aaa caa ggt cgg ata      672
   Asn Arg Asp Met Lys Lys Leu Ile Thr Arg Leu Lys Gln Gly Arg Ile
       210                 215                 220
   att tgg tat tca cct gat caa gat ttt ggt ctt gag cat ggc gtg atg      720
   Ile Trp Tyr Ser Pro Asp Gln Asp Phe Gly Leu Glu His Gly Val Met
   225                 230                 235                 240
   gcg acc ttt ttt ggt gtg cct gca gca acg att acc gct cag cgt cgt      768
   Ala Thr Phe Phe Gly Val Pro Ala Ala Thr Ile Thr Ala Gln Arg Arg
                   245                 250                 255
   ctt att aag ctg ggt gat aaa gcc aat cct cct gtc atc atc atg atg      816
   Leu Ile Lys Leu Gly Asp Lys Ala Asn Pro Pro Val Ile Ile Met Met
               260                 265                 270
   gat atg ctc aga caa acg ccc gat tat atc gca aaa ggt cac cgt cca      864
   Asp Met Leu Arg Gln Thr Pro Asp Tyr Ile Ala Lys Gly His Arg Pro
           275                 280                 285
   cat tat cac atc agc cta agc gct gtg tta aaa aat tat ccc agc gat      912
   His Tyr His Ile Ser Leu Ser Ala Val Leu Lys Asn Tyr Pro Ser Asp
       290                 295                 300
   gac gaa acc gcc gat gct gaa cgc atc aat cga ctg att gag caa aat      960
   Asp Glu Thr Ala Asp Ala Glu Arg Ile Asn Arg Leu Ile Glu Gln Asn
   305                 310                 315                 320
   att caa aaa gat tta acc cag tgg atg tgg ttt cat cgc cgc ttt aaa      1008
   Ile Gln Lys Asp Leu Thr Gln Trp Met Trp Phe His Arg Arg Phe Lys
                   325                 330                 335
   act caa gcc gat gac acc aat tac tat caa cat taa tg                   1046
   Thr Gln Ala Asp Asp Thr Asn Tyr Tyr Gln His  *
               340                 345
<210> 81
   <211> 876
   <212> DNA
   <213> Neisseria meningitidis
<220>
   <221> CDS
   <222> (1)...(876)
<400> 81
   atg aaa ttt ata ttt ttt gta ctg tat gtt ttg cag ttt ctg ccg ttt        48
   Met Lys Phe Ile Phe Phe Val Leu Tyr Val Leu Gln Phe Leu Pro Phe
    1               5                  10                  15
   gcg ctg ctg cac aaa ctt gcc gac ctg acg ggt ttg ctc gcc tac ctt        96
   Ala Leu Leu His Lys Leu Ala Asp Leu Thr Gly Leu Leu Ala Tyr Leu
               20                  25                  30
   ttg gtc aaa ccc cgc cgc cgt atc ggc gaa atc aat ttg gca aaa tgc       144
   Leu Val Lys Pro Arg Arg Arg Ile Gly Glu Ile Asn Leu Ala Lys Cys
           35                  40                  45
   ttt ccc gag tgg gac gga aaa aag cgc gaa acc gta ttg aag cag cat       192
   Phe Pro Glu Trp Asp Gly Lys Lys Arg Glu Thr Val Leu Lys Gln His
       50                  55                  60
   ttc aaa cat atg gcg aaa ctg atg ctt gaa tac ggc tta tat tgg tac       240
   Phe Lys His Met Ala Lys Leu Met Leu Glu Tyr Gly Leu Tyr Trp Tyr
   65                  70                  75                  80
   gcg cct gcc ggg cgt ttg aaa tcg ctg gtg cgt tac cgc aat aag cat       288
   Ala Pro Ala Gly Arg Leu Lys Ser Leu Val Arg Tyr Arg Asn Lys His
                   85                  90                  95
   tat ttg gac gac gcg ctg gcg gcg ggg gaa aaa gtc atc att ctg tac       336
   Tyr Leu Asp Asp Ala Leu Ala Ala Gly Glu Lys Val Ile Ile Leu Tyr
               100                 105                 110
   ccg cac ttc acc gcg ttc gag atg gcg gtg tac gcg ctt aat cag gat       384
   Pro His Phe Thr Ala Phe Glu Met Ala Val Tyr Ala Leu Asn Gln Asp
           115                 120                 125
   gta ccg ctg atc agt atg tat tcc cac caa aaa aac aag ata ttg gac       432
   Val Pro Leu Ile Ser Met Tyr Ser His Gln Lys Asn Lys Ile Leu Asp
       130                 135                 140
   gca cag att ttg aaa ggc cgc aac cgc tac gac aat gtc ttc ctt atc       480
   Ala Gln Ile Leu Lys Gly Arg Asn Arg Tyr Asp Asn Val Phe Leu Ile
   145                 150                 155                 160
   ggg cgc acc gaa ggc gtg cgc gcc ctc gtc aaa cag ttc cgc aaa agc       528
   Gly Arg Thr Glu Gly Val Arg Ala Leu Val Lys Gln Phe Arg Lys Ser
                   165                 170                 175
   agc gcg ccg ttt ctg tat ctg ccc gat cag gat ttc gga cgc aac gat       576
   Ser Ala Pro Phe Leu Tyr Leu Pro Asp Gln Asp Phe Gly Arg Asn Asp
               180                 185                 190
   tcg gtt ttt gtg gat ttt ttc ggt att cag acg gca acg att acc ggc      624
   Ser Val Phe Val Asp Phe Phe Gly Ile Gln Thr Ala Thr Ile Thr Gly
           195                 200                 205
   ttg agc cgc att gcc gcg ctt gca aat gca aaa gtg ata ccc gcc atc      672
   Leu Ser Arg Ile Ala Ala Leu Ala Asn Ala Lys Val Ile Pro Ala Ile
       210                 215                 220
   ccc gtc cgc gag gcg gac aat acg gtt aca ttg cat ttc tac ccg gct      720
   Pro Val Arg Glu Ala Asp Asn Thr Val Thr Leu His Phe Tyr Pro Ala
   225                 230                 235                 240
   tgg gaa tcc ttt ccg agt gaa gat gcg cag gcc gac gcg cag cgc atg      768
   Trp Glu Ser Phe Pro Ser Glu Asp Ala Gln Ala Asp Ala Gln Arg Met
                   245                 250                 255
   aac cgt ttt atc gag gaa ccg tgc gcg aac atc ccg agc agt att ttt      816
   Asn Arg Phe Ile Glu Glu Pro Cys Ala Asn Ile Pro Ser Ser Ile Phe
               260                 265                 270
   ggc tgc aca agc gtt tca aaa ccc gtc cgg aag gca gcc ccg att ttt      864
   Gly Cys Thr Ser Val Ser Lys Pro Val Arg Lys Ala Ala Pro Ile Phe
           275                 280                 285
   act gat acg taa                                                      876
   Thr Asp Thr  *
       290
<210> 82
   <211> 38
   <212> DNA
   <213> Artificial Sequence
<220>
   <223> PorA5' Fwd primer
<400> 82
   cccaagcttg ccgtctgaat acatcccgtc attcctca 38
<210> 83
   <211> 34
   <212> DNA
   <213> Artificial Sequence
<220>
   <223> PorA5' Rev primer
<400> 83
   cgatgctcgc gactccagag acctcgtgcg ggcc 34
<210> 84
   <211> 38
   <212> DNA
   <213> Artificial Sequence
<220>
   <223> PorA3' Fwd primer
<400> 84
   ggaagatctg attaaatagg cgaaaatacc agctacga 38
<210> 85
   <211> 37
   <212> DNA
   <213> Artificial Sequence
<220>
   <223> PorA3'Rev primer
<400> 85
gccgaattct tcagacggcg cagcaggaat ttatcgg 37
<210> 86
   <211> 41
   <212> DNA
   <213> Artificial sequence
<220>
   <223> PoLa Rev1 primer
<400> 86
   gaattgttat ccgctcacaa ttccgggcaa acacccgata c 41
<210> 87
   <211> 70
   <212> DNA
   <213> Artificial Sequence
<220>
   <223> PoLa Rev2 primer
<400> 87
   gaattccata tgatcggctt ccttttgtaa atttgataaa aacctaaaaa catcgaattg 60
   ttatccgctc 70
<210> 88
   <211> 30
   <212> DNA
   <213> Artificial Sequence
<220>
   <223> PorAlaco Fwd primer
<400> 88
   aagctctgca ggaggtctgc gcttgaattg 30
<210> 89
   <211> 28
   <212> DNA
   <213> Artificial sequence
<220>
   <223> PorAlaco Rev primer
<400> 89
   cttaaggcat atgggcttcc ttttgtaa 28
<210> 90
   <211> 22
   <212> DNA
   <213> Artificial Sequence
<220>
   <223> PPA1 primer
<400> 90
   gcggccgttg ccgatgtcag cc 22
<210> 91
   <211> 24
   <212> DNA
   <213> Artificial sequence
<220>
   <223> PPA2 primer
<400> 91
   ggcatagctg atgcgtggaa ctgc 24
<210> 92
   <211> 33
   <212> DNA
   <213> Artificial sequence
<220>
   <223> N-full-01 primer
<400> 92
   gggaattcca tatgaaaaaa gcacttgcca cac 33
<210> 93
   <211> 31
   <212> DNA
   <213> Artificial sequence
<220>
   <223> Nde-NspA-3 primer
<400> 93
   ggaattccat atgtcagaat ttgacgcgca c 31
<210> 94
   <211> 30
   <212> DNA
   <213> Artificial sequence
<220>
   <223> PNS1 primer
<400> 94
   ccgcgaattc ggaaccgaac acgccgttcg 30
<210> 95
   <211> 27
   <212> DNA
   <213> Artificial sequence
<220>
   <223> PNS1 primer
<400> 95
   cgtctagacg tagcggtatc cggctgc 27
<210> 96
   <211> 38
   <212> DNA
   <213> Artificial sequence
<220>
   <223> PromD15-51X primer
<400> 96
   gggcgaattc gcggccgccg tcaacggcac acccgttg 38
<210> 97
   <211> 28
   <212> DNA
   <213> Artificial Sequence
<220>
   <223> ProD15-52 primer
<400> 97
   gctctagagc ggaatgcggt ttcagacg 28
<210> 98
   <211> 47
   <212> DNA
   <213> Artificial Sequence
<220>
   <223> PNS4 primer
<400> 98
   agctttattt aaatccttaa ttaacgcgtc cggaaaatat gcttatc 47
<210> 99
   <211> 33
   <212> DNA
   <213> Artificial Sequence
<220>
   <223> PNS5 primer
<400> 99
   agctttgttt aaaccctgtt ccgctgcttc ggc 33
<210> 100
   <211> 43
   <212> DNA
   <213> Artificial Sequence
<220>
   <223> D15-S4 primer
<400> 100
   gtccgcattt aaatccttaa ttaagcagcc ggacagggcg tgg 43
<210> 101
   <211> 33
   <212> DNA
   <213> Artificial Sequence
<220>
   <223> D15-S5 primer
<400> 101
   agctttgttt aaaggatcag ggtgtggtcg ggc 33
<210> 102
   <211> 28
   <212> DNA
   <213> Artificial Sequence
<220>
   <223> DT88 primer
<400> 102
   gaagagaagg tggaaatggc gttttggc 28
<210> 103
   <211> 27
   <212> DNA
   <213> Artificial sequence
<220>
   <223> DT89 primer
<400> 103
   ccaaaacgcc atttccacct tctcttc 27
<210> 104
   <211> 25
   <212> DNA
   <213> Artificial Sequence
<220>
   <223> PorA3 primer
<400> 104
   ccaaatcctc gctcccctta aagcc 25
<210> 105
   <211> 24
   <212> DNA
   <213> Artificial Sequence
<220>
   <223> pl-2 primer
<400> 105
   cgctgatttt cgtcctgatg cggc 24
<210> 106
   <211> 25
   <212> DNA
   <213> Artificial Sequence
<220>
   <223> p1-1 primer
<400> 106
   ggtcaattgc gcctggatgt tcctg 25
<210> 107
   <211> 26
   <212> DNA
   <213> Artificial Sequence
<220>
   <223> porB1 primer
<400> 107
   ggtagcggtt gtaacttcag taactt 26
<210> 108
   <211> 25
   <212> DNA
   <213> Artificial Sequence
<220>
   <223> porB2 primer
<400> 108
   gtcttcttgg cctttgaagc cgatt 25
<210> 109
   <211> 25
   <212> DNA
   <213> Artificial sequence
<220>
   <223> porB3 primer
<400> 109
   ggagtcagta ccggcgatag atgct 25
<210> 110
   <211> 37
   <212> DNA
   <213> Artificial sequence
<220>
   <223> ProD15-51X primer
<400> 110
   gggcgaattc gcggccgccg tcaacggcac accgttg 37
<210> 111
   <211> 43
   <212> DNA
   <213> Artificial sequence
<220>
   <223> TnRD15-KpnI/xbaI + US primer
<400> 111
   cgccggtacc tctagagccg tctgaaccac tcgtggacaa ccc 43
<210> 112
   <211> 29
   <212> DNA
   <213> Artificial sequence
<220>
   <223> TnR03Cam(KpnI) primer
<400> 112
   cgccggtacc gccgctaact ataacggtc 29
<210> 113
   <211> 31
   <212> DNA
   <213> Artificial Sequence
<220>
   <223> PorA-01 primer
<400> 113
   cgccggtacc gaggtctgcg cttgaattgt g 31
<210> 114
   <211> 33
   <212> DNA
   <213> Artificial Sequence
<220>
   <223> PorA02 primer
<400> 114
   cgccggtacc tctagacatc gggcaaacac ccg 33
<210> 115
   <211> 20
   <212> DNA
   <213> Artificial Sequence
<220>
   <223> Cam-05 primer
<400> 115
   gtactgcgat gagtggcagg 20
<210> 116
   <211> 31
   <212> DNA
   <213> Artificial sequence
<220>
   <223> Hsf 01-Nde primer
<400> 116
   ggaattccat atgatgaaca aaatataccg c 31
<210> 117
   <211> 31
   <212> DNA
   <213> Artificial Sequence
<220>
   <223> Hsf 02-Nhe primer
<400> 117
   gtagctagct agcttaccac tgataaccga c 31
<210> 118
   <211> 36
   <212> DNA
   <213> Artificial sequence
<220>
   <223> GFP-mut-Asn primer
<400> 118
   aactgcagaa ttaatatgaa aggagaagaa cttttc 36
<210> 119
   <211> 33
   <212> DNA
   <213> Artificial Sequence
<220>
   <223> GFP-Spe primer
<400> 119
   gacatactag tttatttgta gagctcatcc atg 33
<210> 120
   <211> 30
   <212> DNA
   <213> Artificial Sequence
<220>
   <223> RP1 (sacII) primer
<400> 120
   tccccgcggg ccgtctgaat acatcccgtc 30
<210> 121
   <211> 51
   <212> DNA
   <213> Artificial Sequence
<220>
   <223> RP2 primer
<400> 121
   catatgggct tccttttgta aatttgaggg caaacacccg atacgtcttc a 51
<210> 122
   <211> 48
   <212> DNA
   <213> Artificial Sequence
<220>
   <223> RP3 primer
<400> 122
   agacgtatcg ggtgtttgcc ctcaaattta caaaaggaag cccatatg 48
<210> 123
   <211> 33
   <212> DNA
   <213> Artificial Sequence
<220>
   <223> RP4 (ApaI) primer
<400> 123
   gggtattccg ggcccttcag acggcgcagc agg 33
<210> 124
   <211> 28
   <212> DNA
   <213> Artificial Sequence
<220>
   <223> PNS1' primer
<400> 124
   ccgcgaattc gacgaagccg ccctcgac 28
<210> 125
   <211> 37
   <212> DNA
   <213> Artificial Sequence
<220>
   <223> BAD01-2 primer
<400> 125
   ggcgcccggg ctcgagctta tcgatggaaa acgcagc 37
<210> 126
   <211> 47
   <212> DNA
   <213> Artificial Sequence
<220>
   <223> BAD02-2 primer
<400> 126
   ggcgcccggg ctcgagttca gacggcgcgc ttatatagtg gattaac 47
<210> 127
   <211> 39
   <212> DNA
   <213> Artificial Sequence
<220>
   <223> BAD 15-2 primer
<400> 127
   ggcgcccggg ctcgagtcta gacatcgggc aaacacccg 39
<210> 128
   <211> 39
   <212> DNA
   <213> Artificial Sequence
<220>
   <223> BAD 03-2 primer
<400> 128
   ggcgcccggg ctcgagcact agtattaccc tgttatccc 39
<210> 129
   <211> 19
   <212> DNA
   <213> Artificial Sequence
<220>
   <223> BAD 25 primer
<400> 129
   gagcgaagcc gtcgaacgc 19
<210> 130
   <211> 20
   <212> DNA
   <213> Artificial Sequence
<220>
   <223> BAD08 primer
<400> 130
   cttaagcgtc ggacatttcc 20
<210> 131
   <211> 31
   <212> DNA
   <213> Artificial Sequence
<220>
   <223> PLA1 Amo5 primer
<400> 131
   gccgtctgaa tttaaaattg cgcgtttaca g 31
<210> 132
   <211> 38
   <212> DNA
   <213> Artificial Sequence
<220>
   <223> PLA1 Amo3 primer
<400> 132
   gtagtctaga ttcagacggc gcaatttggt ttccgcac 38
<210> 133
   <211> 27
   <212> DNA
   <213> Artificial Sequence
<220>
   <223> CIRC1-Bgl primer
<400> 133
   cctagatctc tccgcccccc attgtcg 27
<210> 134
   <211> 46
   <212> DNA
   <213> Artificial Sequence
<220>
   <223> CIRC1-XH-RBS/2 primer
<400> 134
   ccgctcgagt acaaaaggaa gccgatatga atatacggaa tatgcg 46
<210> 135
   <211> 24
   <212> DNA
   <213> Artificial sequence
<220>
   <223> CIRC2-XHO/2 primer
<400> 135
   ccgctcgaga tgaatatacg gaat 24
<210> 136
   <211> 38
   <212> DNA
   <213> Artificial Sequence
<220>
   <223> BAD20 primer
<400> 136
   tcccccggga gatctcacta gtattaccct gttatccc 38
<210> 137
   <211> 32
   <212> DNA
   <213> Artificial Sequence
<220>
   <223> CM-PORA-3 primer <400> 137
   ccgctcgaga taaaaaccta aaaacatcgg gc 32
<210> 138 <211> 28

   <212> DNA
   <213> Artificial Sequence
<220>
   <223> CM-PORA-D15/3 primer
<400> 138
   cggctcgagt gtcagttcct tgtggtgc 28
<210> 139
   <211> 45
   <212> DNA
   <213> Artificial sequence
<220>
   <223> BAD16 primer
<400> 139
   ggcctagcta gccgtctgaa gcgattagag tttcaaaatt tattc 45
<210> 140
   <211> 42
   <212> DNA
   <213> Artificial Sequence
<220>
   <223> BAD17 primer
<400> 140
   ggccaagctt cagacggcgt tcgaccgagt ttgagccttt gc 42
<210> 141
   <211> 39
   <212> DNA
   <213> Artificial Sequence
<220>
   <223> BAD18 primer
<400> 141
   tcccccggga agatctggac gaaaaatctc aagaaaccg 39
<210> 142
   <211> 64
   <212> DNA
   <213> Artificial Sequence
<220>
   <223> BAD19 primer
<400> 142
   ggaagatctc cgctcgagca aatttacaaa aggaagccga tatgcaacag caacatttgt 60
   tccg 64
<210> 143
   <211> 36
   <212> DNA
   <213> Artificial Sequence
<220>
   <223> BAD21 primer
<400> 143
   ggaagatctc cgctcgagac atcgggcaaa cacccg 36
<210> 144
   <211> 36
   <212> DNA
   <213> Artificial sequence
<220>
   <223> PQ-rec5-Nhe primer
<400> 144
   ctagctagcg ccgtctgaac gacgcgaagc caaagc 36
<210> 145
   <211> 37
   <212> DNA
   <213> Artificial Sequence
<220>
   <223> PQ-rec3-Hin primer
<400> 145
   gccaagcttt tcagacggca cggtatcgtc cgattcg 37
<210> 146
   <211> 30
   <212> DNA
   <213> Artificial Sequence
<220>
   <223> CIRC1-PQ-Bgl primer
<400> 146
   ggaagatcta atggagtaat cctcttctta 30
<210> 147
   <211> 50
   <212> DNA
   <213> Artificial Sequence
<220>
   <223> CIRC1-PQ-XHO primer
<400> 147
   ccgctcgagt acaaaaggaa gccgatatga ttaccaaact gacaaaaatc 50
<210> 148
   <211> 33
   <212> DNA
   <213> Artificial sequence
<220>
   <223> CIRC2-PQ-X primer
<400> 148
   ccgctcgaga tgaataccaa actgacaaaa atc 33
<210> 149
   <211> 40
   <212> DNA
   <213> Artificial Sequence
<220>
   <223> CM-PORA-3 primer
<400> 149
   ccgctcgaga taaaaaccta aaaacatcgg gcaaacaccc 40
<210> 150
   <211> 28
   <212> DNA
   <213> Artificial sequence
<220>
   <223> CM-PORA-D153 primer
<400> 150
   gggctcgagt gtcagttcct tgtggtgc 28
<210> 151
   <211> 32
   <212> DNA
   <213> Artificial Sequence
<220>
   <223> CIRC-Kan-NCO primer
<400> 151
   catgccatgg ttagaaaaac tcatcgagca tc 32
<210> 152
   <211> 31
   <212> DNA
   <213> Artificial Sequence
<220>
   <223> CIRC-Kan-Xba primer
<400> 152
   ctagtctaga tcagaattgg ttaattggtt g 31
<210> 153
   <211> 43
   <212> DNA
   <213> Artificial Sequence
<220>
   <223> SAC/NCO/NEW5 primer
<400> 153
   catgccatgg gaggatgaac gatgaacatc aaaaagtttg caa 43
<210> 154
   <211> 33
   <212> DNA
   <213> Artificial Sequence
<220>
   <223> SAC/NCO/NEW3 primer
<400> 154 33
   gatcccatgg ttatttgtta actgttaatt gtc 33
<210> 155
   <211> 72
   <212> DNA
   <213> Artificial Sequence
<220>
   <223> Kan-PorA-5 primer
<400> 155
   gccgtctgaa cccgtcattc ccgcgcaggc gggaatccag tccgttcagt ttcgggaaag 60
   ccacgttgtg tc 72
<210> 156
   <211> 69
   <212> DNA
   <213> Artificial Sequence
<220>
   <223> Kan-PorA-3 primer
<400> 156
   ttcagacggc gcagcaggaa tttatcggaa ataactgaaa ccgaacagac taggctgagg 60
   tctgcctcg 69